# EUROPEAN PATENT APPLICATION

(11) **EP 4 293 035 A1**
(43) Date of publication of application: **20.12.2023**
(21) Application number: 22752816.3
(22) Date of filing: 10.02.2022
(51) Int. Cl.: C07H 21/04, C07D 213/71, C07D 235/28, C07D 249/04, C07D 257/04, C07D 263/58, C07D 277/78, C07D 513/04, C12N 15/11

(54) **OLIGONUCLEOTIDE PRODUCTION METHOD**

(30) Priority: 12.02.2021 JP 2021021175
(71) Applicant: AJINOMOTO CO., INC., Chuo-ku Tokyo 104-8315 (JP)
(72) Inventor: HAYAKAWA, Nobuhiko, Kawasaki-shi, Kanagawa 210-8681 (JP); ICHIMARU, Taisuke, Kawasaki-shi, Kanagawa 210-8681 (JP); INOUE, Satoshi, Kawasaki-shi, Kanagawa 210-8681 (JP); TAKAHASHI, Daisuke, Kawasaki-shi, Kanagawa 210-8681 (JP)
(74) Representative: Mewburn Ellis LLP
(86) International application number: PCT/JP2022/005362
(87) International publication number: WO 2022/172994

(57) **Abstract**

The present invention provides a method for producing an oligonucleotide having a phosphate ester bond by oxidizing an oligonucleotide precursor having a phosphite ester bond or a phosphonate ester bond with an oxidant, wherein the oxidant is a compound represented by the formula (I) excluding 2,2-dipyridyl disulfide: 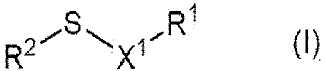 wherein the symbols are as defined in the specification.

## Description

### [Technical Field]

The present invention relates to a method for producing oligonucleotide.

### [Background Art]

As a production method of oligonucleotides, phosphoramidite method and H-phosphonate method are widely used. In the phosphoramidite method including oxidation, for example, as shown in the following formula, phosphoramidite (compound (1)) and nucleoside (compound (2)) are condensed to synthesize an oligonucleotide precursor having a phosphite ester bond (compound (3)), and then the oligonucleotide precursor is oxidized to obtain an oligonucleotide (compound (4)). In the present specification, the "compound represented by the formula (1)" is sometimes abbreviated as "compound (1)". Compounds represented by other formulas, groups represented by other formulas, and the like are also sometimes abbreviated in the same way. 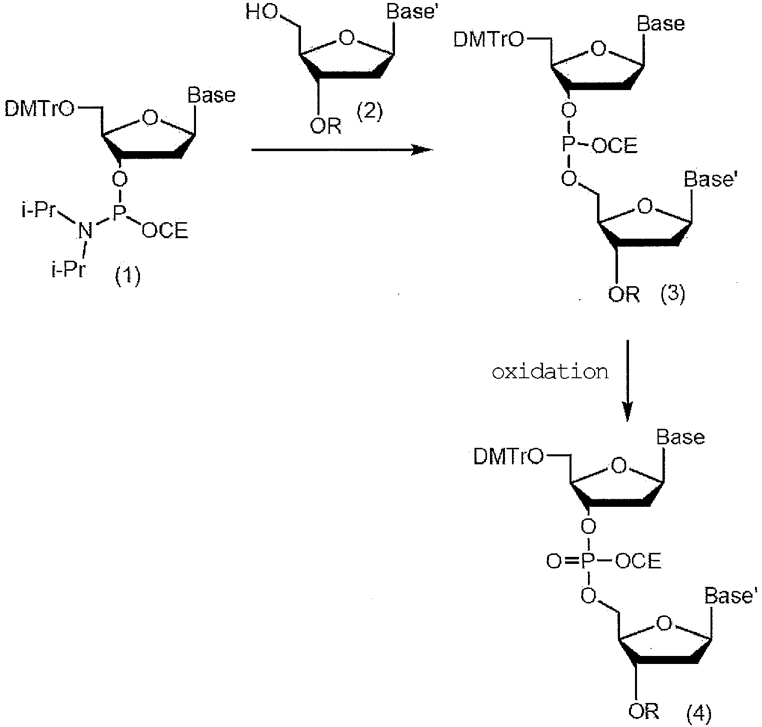 wherein DMTr is a 4,4'-dimethoxytrityl group, i-Pr is an isopropyl group, CE is a 2-cyanoethyl group, Base and Base' are optionally protected nucleic acid bases, and R is a protecting group or a solid support.

In the phosphoramidite method, nucleotide or oligonucleotide (e.g., oligonucleotide with phosphorothioate bond) may be condensed instead of nucleoside. In addition, in the phosphoramidite method, sulfurization of the oligonucleotide precursor may be performed instead of the oxidation of the oligonucleotide precursor.

In the H-phosphonate method including oxidation, for example, as shown in the following formula, H-phosphonate (compound (5)) and pivaloyl chloride (PivCl) are reacted to synthesize an activated H-phosphonate (compound (6)), and it is condensed with nucleoside (compound (7)) to synthesize an oligonucleotide precursor having an H-phosphonate ester bond (compound (8)), and then the oligonucleotide precursor is oxidized to obtain an oligonucleotide (compound (9)). 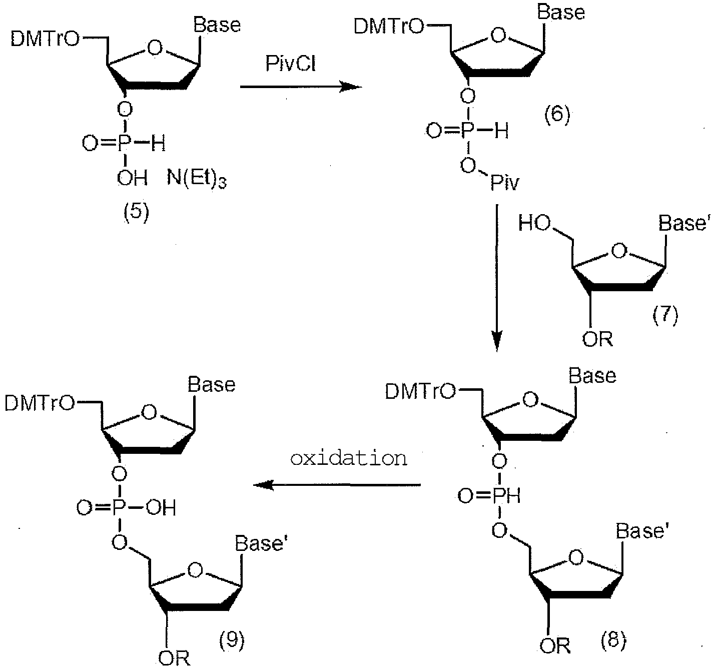 wherein DMTr is 4,4'-dimethoxytrityl, N(Et)₃ is triethylamine, Base and Base' are particularly protected nucleic acid bases, Piv is a pivaloyl group, and R is a protecting group or a solid support.

In the H-phosphonate method, nucleotide or oligonucleotide (e.g., oligonucleotide having a thiophosphate ester bond) may be condensed instead of nucleoside. In the H-phosphonate method, an oligonucleotide precursor may be sulfurized instead of the oxidation of an oligonucleotide precursor.

In addition to the aforementioned representative phosphoramidite method and H-phosphonate method as described above, various methods for producing oligonucleotides have been proposed. For example, Non Patent Literature 1 discloses that, as shown in the following formula, H-phosphonate (compound 1) and nucleoside (compound 2) are condensed and oxidized in the presence of a large quantity of triphenylphosphine and a large quantity of 2,2'-dipyridyl disulfide to produce a dinucleotide (compound 4). 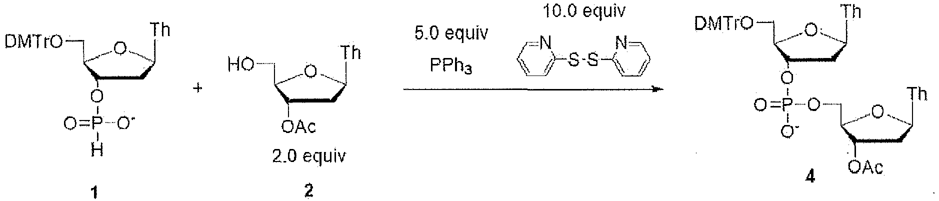 wherein DMTr is 4,4'-dimethoxytrityl, Th is a thymine base, PPhs is triphenylphosphine, and Ac is an acetyl group.

While Non Patent Literature 2 and Patent Literature 1 do not disclose a production method of oligonucleotide, they disclose, as shown in the following formula, reduction of a benzoisothiazolone derivative (compound 1) with triethyl phosphite (P(OEt)₃) (in other words, oxidation of triethyl phosphite (P(OEt)₃) with a benzoisothiazolone derivative (compound 1) to form triethyl phosphate (O=P(OEt)₃)).

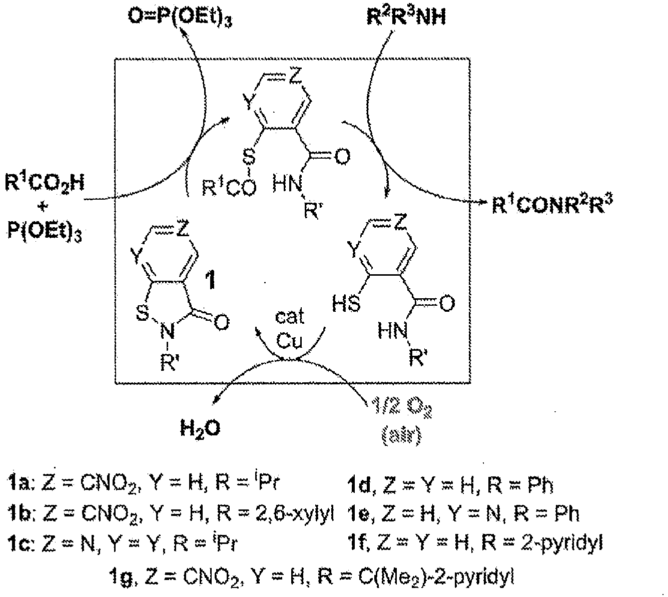

### [Citation List]

### [Patent Literature]

[PTL 1]
WO 2017/070157

### [Non Patent Literature]

[NPL 1]
   ARKIVOC 2009 (iii) 264-273
[NPL 2]
   J. Am. Chem. Soc. 2016, 138, 6715-6718

### [Summary of Invention]

### [Technical Problem]

In the phosphoramidite method, iodine is widely used as an oxidant used for oxidation of an oligonucleotide precursor having a phosphite ester bond (e.g., a compound represented by the aforementioned formula (3)). Also in the H-phosphonate method, iodine is widely used as an oxidant used for oxidation of an oligonucleotide precursor having a phosphonate ester bond (e.g., a compound represented by the aforementioned formula (8)).

Thus, the present inventors have found that use of iodine as an oxidant for oligonucleotide precursors having a phosphite ester bond results in the formation of a byproduct due to the decomposition of the phosphite ester binding site of the precursor by the action with the oxidant (hereinafter sometimes to be referred to as "defective byproduct"). In addition, they have found that oxidation of an oligonucleotide precursor having a thiophosphate ester bond in addition to a phosphite ester bond or a phosphonate ester bond with iodine results in the formation of a byproduct due to the conversion of the aforementioned thiophosphate ester bond to a phosphate ester bond (hereinafter sometimes to be referred to as "desulfurized byproduct").

The present invention has been made by noting the above-mentioned situation and aims to provide a method for producing an oligonucleotide capable of suppressing the aforementioned defective byproduct or desulfurized byproduct as compared with the use of iodine.

### [Solution to Problem]

The present inventors have conducted intensive studies and found that the production of the aforementioned byproduct can be suppressed by oxidizing an oligonucleotide precursor having a phosphite ester bond or a phosphonate ester bond by using as an oxidant a compound represented by the formula (I): 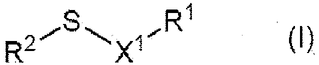 wherein
X¹ is a single bond, a sulfur atom, an oxygen atom, -S(=O)₂- or -N(-R³)-,
when X¹ is a single bond, then R¹ is a halogen atom, and R² is an optionally substituted aryl group or an optionally substituted heteroaryl group,
when X¹ is a sulfur atom or -S(=O)₂-, then R¹ is an optionally substituted alkyl group, an optionally substituted aryl group, or an optionally substituted heteroaryl group, and R² is an optionally substituted aryl group or an optionally substituted heteroaryl group,
when X¹ is an oxygen atom, then R¹ and R² form, together with a sulfur atom and an oxygen atom bonded thereto, an optionally substituted heterocycle, and
when X¹ is -N(-R³)-, then R¹ is an optionally substituted alkyl group, an optionally substituted aryl group, or an optionally substituted heteroaryl group, and R² and R³ form, together with a sulfur atom and a nitrogen atom bonded thereto, an optionally substituted heterocycle, or R¹ and R³ form, together with a nitrogen atom bonded thereto, an optionally substituted heterocycle, and R² is an optionally substituted aryl group or an optionally substituted heteroaryl group
   provided that 2,2-dipyridyl disulfide is excluded. Based on this finding, the present invention provides the following.

[1] A method for producing an oligonucleotide having a phosphate ester bond by oxidizing an oligonucleotide precursor having a phosphite ester bond or a phosphonate ester bond with an oxidant, wherein
   the aforementioned oxidant is a compound represented by the formula (I): 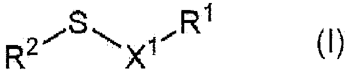 wherein
   X¹ is a single bond, a sulfur atom, an oxygen atom, -S(=O)₂- or -N(-R³) -,
   when X¹ is a single bond, then R¹ is a halogen atom, and R² is an optionally substituted aryl group or an optionally substituted heteroaryl group,
   when X¹ is a sulfur atom or -S(=O)₂-, then R¹ is an optionally substituted alkyl group, an optionally substituted aryl group, or an optionally substituted heteroaryl group, and R² is an optionally substituted aryl group or an optionally substituted heteroaryl group,
   when X¹ is an oxygen atom, then R¹ and R² form, together with a sulfur atom and an oxygen atom bonded thereto, an optionally substituted heterocycle, and
   when X¹ is -N(-R³)-, then R¹ is an optionally substituted alkyl group, an optionally substituted aryl group, or an optionally substituted heteroaryl group, and R² and R³ form, together with a sulfur atom and a nitrogen atom bonded thereto, an optionally substituted heterocycle, or R¹ and R³ form, together with a nitrogen atom bonded thereto, an optionally substituted heterocycle, and R² is an optionally substituted aryl group or an optionally substituted heteroaryl group provided that 2,2-dipyridyl disulfide is excluded.
[2] The method of the aforementioned [1], wherein the aforementioned compound represented by the formula (I) comprises a compound represented by the formula (Ia): 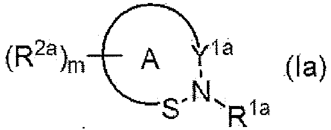 wherein
   R^{1a} is an optionally substituted alkyl group, an optionally substituted aryl group, or an optionally substituted heteroaryl group,
   ring A is a 5- or 6-membered unsaturated heterocycle,
   Y^{1a} is -S(=0)-, -S(=O)₂-, -C(=O)-, or -C (=S) -,
   m is an integer of 0 to 3,
   R^{2a} in the number of m are each independently an optionally substituted alkyl group, an optionally substituted alkoxy group, or an electron-withdrawing group, and
   when m is an integer of not less than 2, the adjacent two R^{2a} optionally form, together with ring A, an optionally substituted fused ring, and/or a compound represented by the formula (Ib): 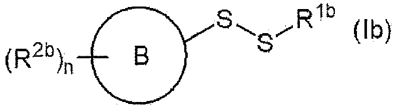 wherein
      R^{1b} is an optionally substituted alkyl group, an optionally substituted aryl group, or an optionally substituted heteroaryl group,
      ring B is a 6-membered aromatic hydrocarbon ring or a 5- or 6-membered aromatic heterocycle,
      n is an integer of 0 to 5,
      R^{2b} in the number of n are each independently an optionally substituted alkyl group, an optionally substituted alkoxy group, or an electron-withdrawing group, and
      when n is an integer of not less than 2, the adjacent two R^{2b} optionally form, together with ring B, an optionally substituted bicyclic fused aromatic heterocycle
         provided that 2,2-dipyridyl disulfide is excluded.
[3] The method of the aforementioned [2], wherein the aforementioned compound represented by the formula (Ia) is a compound represented by the formula (Ic): 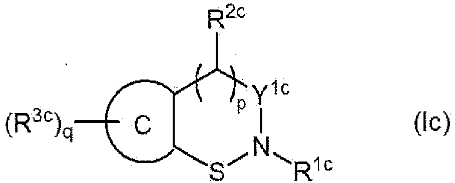 wherein
   R^{1c} is an optionally substituted alkyl group, an optionally substituted aryl group, or an optionally substituted heteroaryl group,
   Y^{1c} is -S(=O)-, -S(=O)₂-, -C(=O)-, or -C(=S)-,
   p is 0 or 1,
   R^{2c} is a hydrogen atom, an optionally substituted alkyl group, an optionally substituted alkoxy group, or an electron-withdrawing group,
   ring C is a 6-membered aromatic hydrocarbon ring, a 6-membered nitrogen-containing aromatic heterocycle, or a 10-membered bicyclic fused aromatic heterocycle containing a nitrogen atom,
   q is an integer of 0 to 4, and
   R^{3c} in the number of q are each independently an optionally substituted alkyl group, an optionally substituted alkoxy group, or an electron-withdrawing group.
[4] The method of the aforementioned [3], wherein the aforementioned compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, 2-[1-methyl-1-(2-pyridyl)ethyl]-5-nitro-1,2-benzothiazol-3(2H)-one, 2-phenylisothiazolo[4,5-c]pyridin-3(2H)-one, 2-phenylisothiazolo[5,4-b]quinolin-3(2H)-one, 2-phenyl-5-trifluoromethylisothiazolo[5,4-b]pyridin-3(2H)-one, 2-phenyl-2H-1,2-benzothiazin-3(4H)-one, 2-(2,6-dimethylphenyl)isothiazolo[5,4-b]pyridin-3(2H)-one, 2-(4-methyl-2-pyridinyl)isothiazolo[5,4-b]pyridin-3(2H)-one, 2-(4-methoxyphenyl)isothiazolo[5,4-b]pyridin-3(2H)-one, and 2-(4-fluorophenyl)isothiazolo[5,4-b]pyridin-3(2H)-one.
[5] The method of the aforementioned [3], wherein the aforementioned ring C is a 6-membered aromatic hydrocarbon ring or a 6-membered nitrogen-containing aromatic heterocycle.
[6] The method of the aforementioned [5], wherein the aforementioned compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, and 2-[1-methyl-1-(2-pyridyl)ethyl]-5-nitro-1,2-benzothiazol-3(2H)-one.
[7] The method of any one of the aforementioned [2] to [6], wherein, in the compound represented by the aforementioned formula (Ib), a group represented by the formula (r1): 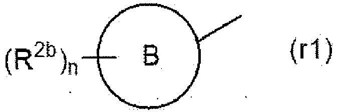 is
   (1) a 2-benzothiazolyl group optionally substituted by 1 to 4 substituents selected from the group consisting of an optionally substituted alkyl group, an optionally substituted alkoxy group, and an electron-withdrawing group,
   (2) a 2-benzoimidazolyl group optionally substituted by 1 to 5 substituents selected from the group consisting of an optionally substituted alkyl group, an optionally substituted alkoxy group, and an electron-withdrawing group,
   (3) a 2-benzoxazolyl group optionally substituted by 1 to 4 substituents selected from the group consisting of an optionally substituted alkyl group, an optionally substituted alkoxy group, and an electron-withdrawing group,
   (4) a 5-(1,2,3-triazolyl) group optionally substituted by 1 or 2 substituents selected from the group consisting of an optionally substituted alkyl group, an optionally substituted alkoxy group, and an electron-withdrawing group,
   (5) a 1H-tetrazol-5-yl group optionally substituted by one substituent selected from the group consisting of an optionally substituted alkyl group and an optionally substituted aryl group,
   (6) a (pyridine-N-oxide)-2-yl group optionally substituted by 1 to 4 substituents selected from the group consisting of an optionally substituted alkyl group, an optionally substituted alkoxy group, and an electron-withdrawing group,
   (7) a 4-pyridyl group optionally substituted by 1 to 4 substituents selected from the group consisting of an optionally substituted alkyl group, an optionally substituted alkoxy group, and an electron-withdrawing group,
   (8) a 2-pyridyl group optionally substituted by 1 to 4 substituents selected from the group consisting of an optionally substituted alkyl group, an optionally substituted alkoxy group, and an electron-withdrawing group,
   (9) a phenyl group optionally substituted by 1 to 5 substituents selected from the group consisting of an optionally substituted alkyl group, an optionally substituted alkoxy group, and an electron-withdrawing group, or
   (10) a 2-imidazolyl group optionally substituted by 1 to 3 substituents selected from the group consisting of an optionally substituted alkyl group, an optionally substituted alkoxy group, and an electron-withdrawing group.
[8] The method of the aforementioned [7], wherein the aforementioned compound represented by the formula (Ib) is at least one selected from the group consisting of 2,2'-dibenzothiazolyl disulfide, 2-(2-benzothiazolyldithio)propanoic acid, 3-(2-benzothiazolyldithio)propanoic acid, 2,2'-dibenzoimidazolyl disulfide, 2,2'-dibenzoxazolyl disulfide, 5,5'-di(1,2,3-triazolyl) disulfide, 5,5'-dithiobis(1-phenyl-1H-tetrazole), 2,2'-dithiobis(pyridine-N-oxide), 4,4'-dipyridyl disulfide, 3-(2-pyridyldithio)propanoic acid, 2,2'-dithiobis(1H-imidazole), and 2,2'-dithiobis(1-methyl-1H-imidazole).
[9] The method of any one of the aforementioned [1] to [8], wherein the aforementioned oxidation is performed in the presence of water.
[10] The method of any one of the aforementioned [1] to [9], wherein the oligonucleotide precursor having a phosphite ester bond or a phosphonate ester bond is
   (1) an oligonucleotide precursor having a phosphite ester bond which is obtained by condensation of a nucleoside, nucleotide, or oligonucleotide with a phosphoramidited nucleoside, nucleotide, or oligonucleotide, or
   (2) an oligonucleotide precursor having a phosphite ester bond which is obtained by condensation of an oligonucleotide with a phosphoramidite.
[11] The method of any one of the aforementioned [1] to [10], wherein the oligonucleotide precursor having a phosphite ester bond or a phosphonate ester bond is an oligonucleotide precursor having a thiophosphate ester bond in addition to the phosphite ester bond or the phosphonate ester bond.
[12] A method for producing an oligonucleotide by an one-pot synthesis, wherein the aforementioned one-pot synthesis comprises
   step (1) in which nucleoside, nucleotide, or oligonucleotide (a), each having a hydrophobic protecting group, and nucleoside, nucleotide, or oligonucleotide (b), each of which is phosphoramidited and in which a hydroxy group is protected by a temporary protecting group removable under acidic conditions are condensed in a solution containing a non-polar solvent to form oligonucleotide precursor (c) having a phosphite ester bond and the hydrophobic protecting group, in which the hydroxy group is protected by the temporary protecting group removable under acidic conditions,
   step (2) in which quencher (i) for the aforementioned phosphoramidited, nucleoside, nucleotide, or oligonucleotide (b) is added to the solution after step (1) to quench the aforementioned phosphoramidited nucleoside, nucleotide, or oligonucleotide (b)
   step (3) in which an oxidant is added to the solution after step (2) to oxidize the aforementioned oligonucleotide precursor (c) to form oligonucleotide (d) having a phosphate ester bond and the hydrophobic protecting group, in which the hydroxy group is protected by the temporary protecting group removable under acidic conditions,
   step (4) in which quencher (ii) for the oxidant is added to the solution after step (3) to quench the oxidant,
   step (5) in which an acid is added to the solution after step (4) to remove the temporary protecting group removable under acidic conditions from the aforementioned oligonucleotide (d) to form oligonucleotide (e) having an unprotected hydroxy group and the hydrophobic protecting group, and
   step (7) in which a polar solvent is added to the solution containing the aforementioned oligonucleotide (e) to precipitate the aforementioned oligonucleotide (e), and
   the aforementioned oxidant is a compound represented by the formula (I): 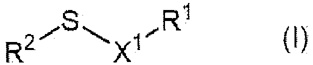 wherein
      X¹ is a single bond, a sulfur atom, an oxygen atom, -S(=O)₂-, or -N(-R³)-,
      when X¹ is a single bond, then R¹ is a halogen atom, and R² is an optionally substituted aryl group or an optionally substituted heteroaryl group,
      when X¹ is a sulfur atom or -S(=O)₂-, then R¹ is an optionally substituted alkyl group, an optionally substituted aryl group, or an optionally substituted heteroaryl group, and R² is an optionally substituted aryl group or an optionally substituted heteroaryl group,
      when X¹ is an oxygen atom, then R¹ and R² form, together with a sulfur atom and an oxygen atom bonded thereto, an optionally substituted heterocycle, and
      when X¹ is -N(-R³)-, then R¹ is an optionally substituted alkyl group, an optionally substituted aryl group, or an optionally substituted heteroaryl group, and R² and R³ form, together with a sulfur atom and a nitrogen atom bonded thereto, an optionally substituted heterocycle, or R¹ and R³ form, together with a nitrogen atom bonded thereto, an optionally substituted heterocycle, and R² is an optionally substituted aryl group or an optionally substituted heteroaryl group
         provided that 2,2-dipyridyl disulfide is excluded.
[13] The method of the aforementioned [12], wherein the compound represented by the aforementioned formula (I) comprises a compound represented by the formula (Ia): 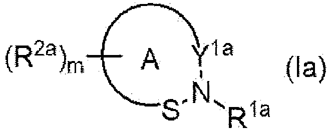 wherein
   R^{1a} is an optionally substituted alkyl group, an optionally substituted aryl group, or an optionally substituted heteroaryl group,
   ring A is a 5- or 6-membered unsaturated heterocycle,
   Y^{1a} is -S(=0)-, -S(=O)₂-, -C(=O)-, or -C(=S)-,
   m is an integer of 0 to 3,
   R^{2a} in the number of m are each independently an optionally substituted alkyl group, an optionally substituted alkoxy group, or an electron-withdrawing group, and
   when m is an integer of not less than 2, the adjacent two R^{2a} optionally form, together with ring A, an optionally substituted fused ring, and/or
      a compound represented by the formula (Ib): 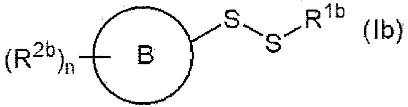 wherein
      R^{1b} is an optionally substituted alkyl group, an optionally substituted aryl group, or an optionally substituted heteroaryl group,
      ring B is a 6-membered aromatic hydrocarbon ring or a 5- or 6-membered aromatic heterocycle,
      n is an integer of 0 to 5,
      R^{2b} in the number of n are each independently an optionally substituted alkyl group, an optionally substituted alkoxy group, or an electron-withdrawing group, and
      when n is an integer of not less than 2, the adjacent two R^{2b} optionally form, together with ring B, an optionally substituted bicyclic fused aromatic heterocycle
         provided that 2,2-dipyridyl disulfide is excluded.
[14] The method of the aforementioned [13], wherein the compound represented by the aforementioned formula (Ia) is a compound represented by the formula (Ic): 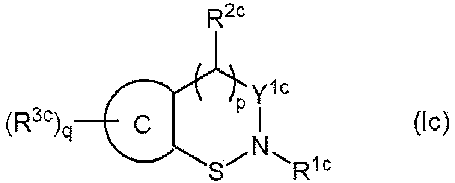 wherein
   R^{1c} is an optionally substituted alkyl group, an optionally substituted aryl group, or an optionally substituted heteroaryl group,
   Y^{1c} is -S(=0)-, -S(=O)₂-, -C(=O)- or -C(=S)-,
   p is 0 or 1,
   R^{2c} is a hydrogen atom, an optionally substituted alkyl group, an optionally substituted alkoxy group, or an electron-withdrawing group,
   ring C is a 6-membered aromatic hydrocarbon ring, a 6-membered nitrogen-containing aromatic heterocycle, or a 10-membered bicyclic fused aromatic heterocycle containing a nitrogen atom,
   q is an integer of 0 to 4, and
   R^{3c} in the number of q are each independently an optionally substituted alkyl group, an optionally substituted alkoxy group, or an electron-withdrawing group.
[15] The method of the aforementioned [14], wherein the compound represented by the aforementioned formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, 2-[1-methyl-1-(2-pyridyl)ethyl]-5-nitro-1,2-benzothiazol-3(2H)-one, 2-phenylisothiazolo[4,5-c]pyridin-3(2H)-one, 2-phenylisothiazolo[5,4-b]quinolin-3(2H)-one, 2-phenyl-5-trifluoromethylisothiazolo[5,4-b]pyridin-3(2H)-one, 2-phenyl-2H-1,2-benzothiazin-3(4H)-one, 2-(2,6-dimethylphenyl)isothiazolo[5,4-b]pyridin-3(2H)-one, 2-(4-methyl-2-pyridinyl)isothiazolo[5,4-b]pyridin-3(2H)-one, 2-(4-methoxyphenyl)isothiazolo[5,4-b]pyridin-3(2H)-one, and 2-(4-fluorophenyl)isothiazolo[5,4-b]pyridin-3(2H)-one.
[16] The method of the aforementioned [14], wherein the aforementioned ring C is a 6-membered aromatic hydrocarbon ring or a 6-membered nitrogen-containing aromatic heterocycle.
[17] The method of the aforementioned [16], wherein the aforementioned compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, and 2-[1-methyl-1-(2-pyridyl)ethyl]-5-nitro-1,2-benzothiazol-3(2H)-one.
[18] The method of any one of the aforementioned [13] to [17], wherein, in the compound represented by the aforementioned formula (Ib), a group represented by the formula (r1): 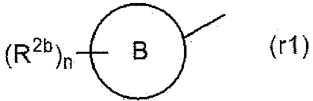 is
   (1) a 2-benzothiazolyl group optionally substituted by 1 to 4 substituents selected from the group consisting of an optionally substituted alkyl group, an optionally substituted alkoxy group, and an electron-withdrawing group,
   (2) a 2-benzoimidazolyl group optionally substituted by 1 to 5 substituents selected from the group consisting of an optionally substituted alkyl group, an optionally substituted alkoxy group, and an electron-withdrawing group,
   (3) a 2-benzoxazolyl group optionally substituted by 1 to 4 substituents selected from the group consisting of an optionally substituted alkyl group, an optionally substituted alkoxy group, and an electron-withdrawing group,
   (4) a 5-(1,2,3-triazolyl) group optionally substituted by 1 or 2 substituents selected from the group consisting of an optionally substituted alkyl group, an optionally substituted alkoxy group, and an electron-withdrawing group,
   (5) a 1H-tetrazol-5-yl group optionally substituted by one substituent selected from the group consisting of an optionally substituted alkyl group and an optionally substituted aryl group,
   (6) a (pyridine-N-oxide)-2-yl group optionally substituted by 1 to 4 substituents selected from the group consisting of an optionally substituted alkyl group, an optionally substituted alkoxy group, and an electron-withdrawing group,
   (7) a 4-pyridyl group optionally substituted by 1 to 4 substituents selected from the group consisting of an optionally substituted alkyl group, an optionally substituted alkoxy group, and an electron-withdrawing group,
   (8) a 2-pyridyl group optionally substituted by 1 to 4 substituents selected from the group consisting of an optionally substituted alkyl group, an optionally substituted alkoxy group, and an electron-withdrawing group,
   (9) a phenyl group optionally substituted by 1 to 5 substituents selected from the group consisting of an optionally substituted alkyl group, an optionally substituted alkoxy group, and an electron-withdrawing group, or
   (10) a 2-imidazolyl group optionally substituted by 1 to 3 substituents selected from the group consisting of an optionally substituted alkyl group, an optionally substituted alkoxy group, and an electron-withdrawing group.
[19] The method of the aforementioned [18], wherein the aforementioned compound represented by the formula (Ib) is at least one selected from the group consisting of 2,2'-dibenzothiazolyl disulfide, 2-(2-benzothiazolyldithio)propanoic acid, 3-(2-benzothiazolyldithio)propanoic acid, 2,2'-dibenzoimidazolyl disulfide, 2,2'-dibenzoxazolyl disulfide, 5,5'-di(1,2,3-triazolyl) disulfide, 5,5'-dithiobis(1-phenyl-1H-tetrazole), 2,2'-dithiobis(pyridine-N-oxide), 4,4'-dipyridyl disulfide, 3-(2-pyridyldithio)propanoic acid, 2,2'-dithiobis(1H-imidazole), and 2,2'-dithiobis(1-methyl-1H-imidazole).
[20] The method of any one of the aforementioned [12] to [19], wherein the aforementioned quencher (i) is water.
[21] The method of any one of the aforementioned [12] to [20], wherein the aforementioned quencher (ii) is an organic phosphorus compound.
[22] The method of the aforementioned [21], wherein the organic phosphorus compound is at least one selected from the group consisting of a phosphine, a phosphorous acid triester, a phosphinite ester, a phosphonous acid diester, and a phosphinate ester.
[23] The method of the aforementioned [21], wherein the organic phosphorus compound is a phosphine.
[24] The method of any one of the aforementioned [12] to [23], wherein the aforementioned one-pot synthesis further comprises adding an aromatic amine to the solution after step (3).
[25] The method of any one of the aforementioned [12] to [24], wherein the aforementioned one-pot synthesis further comprises step (6) of adding, after step (5) and before step (7), a base to the solution after step (5).
[26] The method of any one of the aforementioned [12] to [25], wherein the aforementioned oligonucleotide precursor (c) is an oligonucleotide precursor having a thiophosphate ester bond in addition to the phosphite ester bond.
[27] A compound represented by the formula (In): 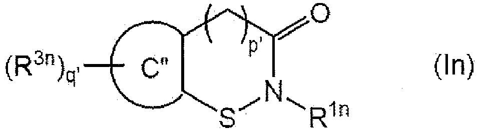 wherein
   (1) R¹ⁿ is an optionally substituted phenyl group, p' is 0, ring C" is a 10-membered bicyclic fused aromatic heterocycle containing a nitrogen atom, and q' is 0, or
   (2) R¹ⁿ is an optionally substituted phenyl group, p' is 0, ring C" is a 6-membered nitrogen-containing aromatic heterocycle, q' is 1, and R³ⁿ is a C₁₋₆ perfluoroalkyl group, or
   (3) R¹ⁿ is an optionally substituted phenyl group, p' is 1, ring C" is a 6-membered nitrogen-containing aromatic heterocycle, and q' is 0, or
   (4) R¹ⁿ is a pyridyl group substituted by one C₁₋₆ alkyl group, p' is 0, ring C" is a 6-membered nitrogen-containing aromatic heterocycle, and q' is 0.
[28] The compound of the aforementioned [27] wherein the compound represented by the aforementioned formula (In) is 2-phenylisothiazolo[5,4-b]quinolin-3(2H)-one, 2-phenyl-5-trifluoromethylisothiazolo[5,4-b]pyridin-3(2H)-one, 2-phenyl-2H-1,2-benzothiazin-3(4H)-one, or 2-(4-methyl-2-pyridinyl)isothiazolo[5,4-b]pyridin-3(2H)-one.

### [Advantageous Effects of Invention]

According to the present invention, oligonucleotide can be produced while suppressing the aforementioned defective byproduct or desulfurized byproduct as compared with when iodine is used.

### [Description of Embodiments]

### 1. Terms

The terms used in the present specification are described. Unless particularly described, the terms described in the present specification have the same meaning as those generally understood by those of ordinary skill in the art the present invention belongs to.

### 1-1. Terms relating to groups

In the present specification, the "C_{a-b}" means a carbon number of not less than a and not more than b (a, b are integers).

In the present specification, the "halogen atom" means a fluorine atom, a chlorine atom, a bromine atom or iodine atom.

In the present specification, examples of the "hydrocarbon group" include an aliphatic hydrocarbon group, an aromaticaliphatic hydrocarbon group, a monocyclic saturated hydrocarbon group, an aromatic hydrocarbon group, and the like, and specific examples thereof include monovalent groups and divalent groups such as alkyl group, alkenyl group, alkynyl group, cycloalkyl group, aryl group, aralkyl group, alkylene group, and the like.

In the present specification, the "alkyl (group)" may be any of a linear and a branched chain. As the "alkyl (group)", an alkyl group having one or more carbon number can be mentioned. When the carbon number is not particularly limited, it is preferably a C₁₋₁₀ alkyl group, more preferably a C₁₋₆ alkyl group, further preferably a C₁₋₅ alkyl group. Specific examples thereof include methyl, ethyl, propyl, isopropyl, butyl, isobutyl, secbutyl, tert-butyl, pentyl, hexyl, and the like.

In the present specification, the "alkenyl (group)" may be any of a linear and a branched chain. Examples of the "alkenyl (group)" include a C₂₋₆ alkenyl group and the like Specific examples thereof include vinyl, 1-propenyl, allyl, isopropenyl, butenyl, isobutenyl, and the like.

In the present specification, the "alkynyl (group)" may be any of a linear and a branched chain. Examples of the "alkynyl (group)" include C₂₋₆ alkynyl group and the like. Specific examples thereof include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and the like.

In the present specification, the "cycloalkyl (group)" means a cyclic alkyl group, and examples thereof include C₃₋₈ cycloalkyl group, preferably C₃₋₈ cycloalkyl group. Specific examples thereof include cyclopropyl, cyclobutyl, cyclopentyl, cyclohexyl, cycloheptyl, cyclooctyl, and the like.

In the present specification, the "aryl (group)" means a monocyclic aromatic or polycyclic (fused) aromatic hydrocarbon group, and examples thereof include C₆₋₁₄ aryl groups such as phenyl, 1-naphthyl, 2-naphthyl, biphenylyl, 2-anthryl, and the like. Among them, a C₆₋₁₀ aryl group is more preferred, and phenyl is particularly preferred.

In the present specification, the "heteroaryl (group)" means a monocyclic or polycyclic (fused) aromatic heterocyclic group containing, besides carbon atom, a hetero atom selected from a nitrogen atom, a sulfur atom, and an oxygen atom as a ring-constituting atom. Examples of the heteroaryl group include 5- or 6-membered monocyclic heteroaryl group and 8- to 14-membered fused polycyclic heteroaryl group.

In the present specification, examples of the "5- or 6-membered monocyclic heteroaryl (group)" include thienyl, furyl, pyrrolyl, imidazolyl, pyrazolyl, thiazolyl, isothiazolyl, oxazolyl, isoxazolyl, pyridyl, (pyridine-N-oxide)-yl, pyrazinyl, pyrimidinyl, pyridazinyl, 1,2,4-oxa diazolyl, 1,3,4-oxa diazolyl, 1,2,4-thiadiazolyl, 1,3,4-thiadiazolyl, triazolyl, tetrazolyl, triazinyl, and the like. In the present invention, the "heteroaryl group" encompasses a (pyridine-N-oxide)-yl group represented by the following formula (in the following formula, * is a binding position).

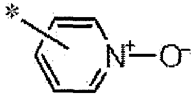

In the present specification, examples of the "5- or 6-membered monocyclic nitrogen-containing heteroaryl (group)" include the aforementioned "5- or 6-membered monocyclic heteroaryl (group)" containing at least one nitrogen atom as a ring-constituting atom.

In the present specification, examples of the "8- to 14-membered fused polycyclic heteroaryl (group)" include benzothiophenyl, benzofuranyl, benzoimidazolyl, benzoxazolyl, benzoisoxazolyl, benzothiazolyl, benzoisothiazolyl, benzotriazolyl, imidazopyridinyl, thienopyridinyl, furopyri dinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acrydinyl, phenazinyl, phenothiazinyl, phenoxazinyl, and the like.

In the present specification, examples of the "8- to 14-membered fused polycyclic nitrogen-containing heteroaryl (group)" include the aforementioned "8- to 14-membered fused polycyclic heteroaryl (group)" containing at least one nitrogen atom as a ring-constituting atom.

In the present specification, as the "aralkyl (group)", a C₇₋₂₀ aralkyl group can be mentioned, and a C₇₋₁₆ aralkyl group (C₆₋₁₀ aryl-C₁₋₆ alkyl group) is preferred. Specific examples thereof include benzyl, 1-phenylethyl, 2-phenylethyl, 1-phenylpropyl, naphthylmethyl, 1-naphthylethyl, 1-naphthylpropyl, and the like.

In the present specification, the "alkylene (group)" may be any of a linear and a branched chain. As the "alkylene (group)", an alkylene group having a carbon number of one or more can be mentioned. When the range of carbon number is not particularly limited, it is preferably a C₁₋₁₀ alkylene group, more preferably a C₁₋₆ alkylene group. Specific examples thereof include methylene, ethylene, propylene, butylene, pentylene, and hexylene.

In the present specification, the "alkoxy (group)" may be any of a linear and a branched chain. As the "alkoxy (group)", an alkoxy group having one or more carbon atoms can be mentioned. When the carbon number is not particularly limited, it is preferably a C₁₋₁₀ alkoxy group, more preferably a C₁₋₆ alkoxy group. Specific examples thereof include methoxy, ethoxy, propoxy, isopropoxy, butoxy, isobutoxy, sec-butoxy, tert-butoxy, pentyloxy, hexyloxy, and the like.

In the present specification, the "acyl (group)" may be any of a linear and a branched chain. Examples of the "acyl (group)" include C₁₋₆ alkanoyl group, C₇₋₁₃ aroyl group, and the like. Specific examples of the "acyl (group)" include formyl, acetyl, n-propionyl, isopropionyl, n-butyryl, isobutyryl, pivaloyl, valeryl, hexanoyl, benzoyl, naphthoyl, levulinyl, and the like. The "acyl (group)" is optionally substituted.

In the present specification, the "6-membered aromatic hydrocarbon ring" means a benzene ring. When "6-membered aromatic hydrocarbon ring" forms a part of the fused ring, the "6-membered aromatic hydrocarbon ring" means a benzene ring that forms a part of the fused ring.

In the present specification, examples of the "5- or 6-membered aromatic heterocycle" include thiophene ring, furan ring, 1H-pyrrole ring, imidazole ring, pyrazole ring, thiazole ring, isothiazole ring, oxazole ring, isoxazole ring, 1,2,4-oxadiazole ring, 1,3,4-oxadiazole ring, 1,2,4-thiadiazole ring, 1,3,4-thiadiazole ring, triazole ring, tetrazole ring, pyridine ring, pyridine-N-oxide ring, pyrazine ring, pyrimidine ring, pyridazine ring, triazine ring, and the like. In the present invention, the "aromatic heterocycle" encompasses a pyridine-N-oxide ring.

In the present specification, examples of the "4 to 8-membered nitrogen-containing heterocycle" include azetidine ring, pyrrolidine ring, pyrroline ring, 2H-pyrrole ring, piperidine ring, piperazine ring, azacycloheptane ring, azacyclooctane ring, 1H-pyrrole ring, imidazole ring, pyrazole ring, thiazole ring, isothiazole ring, oxazole ring, isoxazole ring, 1,2,4-oxadiazole ring, 1,3,4-oxadiazole ring, 1,2,4-thiadiazole ring, 1,3,4-thiadiazole ring, triazole ring, tetrazole ring, pyridine ring, pyridine-N-oxide ring, pyrazine ring, pyrimidine ring, pyridazine ring, triazine ring, and the like.

In the present specification, examples of the "5- or 6-membered nitrogen-containing heterocycle" include the aforementioned "4 to 8-membered nitrogen-containing heterocycle" having 5 or 6 ring-constituting atoms.

In the present specification, examples of the "6-membered nitrogen-containing aromatic heterocycle" include pyridine ring, pyridine-N-oxide ring, pyrazine ring, pyrimidine ring, pyridazine ring, triazine ring, and the like.

In the present specification, examples of the "bicyclic nitrogen-containing fused heterocycle" include pyrrolidine ring, decahydroisoquinoline ring, decahydroquinoline ring, benzothiophene ring, benzofuran ring, isobenzofuran ring, benzoimidazole ring, benzoxazole ring, benzoisoxazole ring, benzothiazole ring, benzoisothiazole ring, benzotriazole ring, indole ring, isoindole ring, 1H-indazole ring, purine ring, isoquinoline ring, 5,6,7,8-tetrahydroisoquinoline ring, quinoline ring, 5,6,7,8-tetrahydroquinoline ring, phthalazine ring, pteridine ring, naphthyridine ring, quinoxaline ring, quinazoline ring, cinnoline ring, and the like.

In the present specification, examples of the "bicyclic fused aromatic heterocycle" include benzothiophene ring, benzofuran ring, isobenzofuran ring, benzoimidazole ring, benzoxazole ring, benzoisoxazole ring, benzothiazole ring, benzoisothiazole ring, benzotriazole ring, indole ring, isoindole ring, 1H-indazole ring, purine ring, isoquinoline ring, 5,6,7,8-tetrahydroisoquinoline ring, quinoline ring, 5,6,7,8-tetrahydroquinoline ring, phthalazine ring, pteridine ring, naphthyridine ring, quinoxaline ring, quinazoline ring, cinnoline ring, and the like. In the present specification, the "bicyclic fused aromatic heterocycle" also includes a bicyclic fused ring (e.g., 5,6,7,8-tetrahydroisoquinoline ring) in which one ring is an aromatic heterocycle and the other ring is a nonaromatic heterocycle or nonaromatic hydrocarbon ring.

In the present specification, examples of the "10-membered bicyclic fused aromatic heterocycle containing a nitrogen atom" include 5,6,7,8-tetrahydroisoquinoline ring, quinoline ring, and the like.

In the present specification, examples of the "electron-withdrawing group" include halogen atom, perchloroalkyl group (e.g., trichloromethyl group), perfluoroalkyl group (e.g., trifluoromethyl group), nitro group, cyano group, formyl group, alkylcarbonyl group (-CO-R, R: alkyl group), perchloroalkylcarbonyl group (-CO-R, R: perchloroalkyl group), perfluoroalkylcarbonyl group (-CO-R, R: perfluoroalkyl group), carboxy group, alkoxycarbonyl group (-CO-OR, R: alkyl group), alkylsulfonyl group (-S(=O)₂-R, R: alkyl group), sulfo group, and the like.

In the present specification, the "perchloroalkyl group" means an alkyl group in which all hydrogen atoms are substituted by a chlorine atom.

In the present specification, the "perfluoroalkyl group" means an alkyl group in which all hydrogen atoms are substituted by a fluorine atom.

In the present specification, examples of the "linker" include -O-, -C(=O)-, -C(=O)O-, -OC(=O)-, -NR'-, -C(=O)NR'-, - NR'C(=O)-, -S-, -SO-, -SO₂-, -Si(R') (R")O-, -Si(R') (R")- (R' and R" are each independently a hydrogen atom or a C₁₋₂₂ hydrocarbon group), and the like. The linker is preferably -O-, -C(=O)-, - C(=O)O-, -OC(=O)-, -C(=O)NH-, -NHC(=O)-, -S-, -SO-, -SO₂-, - Si(R') (R")O-, or -Si(R') (R")- (R' and R" are each independently a hydrogen atom or a C₁₋₂₂ hydrocarbon group) .

In the present specification, examples of the "substituent" include, in addition to halogen atom, alkyl group, aralkyl group, alkoxy group, acyl group, alkenyl group, alkynyl group, cycloalkyl group, and aryl group as mentioned above, hydroxy group, nitro group, cyano group, guanidyl group, carboxy group, alkoxycarbonyl group (the alkoxy moiety is the same as that in the aforementioned alkoxy group), sulfo group, phospho group, alkylsulfanyl group (the alkyl moiety is the same as that in the aforementioned alkyl group), alkylsulfinyl group (the alkyl moiety is the same as that in the aforementioned alkyl group), alkylsulfonyl group (the alkyl moiety is the same as that in the aforementioned alkyl group), amino group, monoalkylamino group (the alkyl moiety is the same as that in the aforementioned alkyl group), dialkylamino group (the alkyl moiety is the same as that in the aforementioned alkyl group), oxo group, and the like.

In the present specification, the "hydroxy-protecting group" is not particularly limited and known protecting groups can be used. Examples of the protecting group include methyl group, benzyl group, p-methoxybenzyl group, tert-butyl group, methoxymethyl group, 2-methoxyethyl group, 2-tetrahydropyranyl group, 2-ethoxyethyl group, 2-cyanoethyl group, (2-cyanoethoxy)methyl group, 1-(2-cyanoethoxy)ethyl group, bis(2-acetoxyethoxy)methyl (ACE) group, (2-nitrobenzyl)oxymethyl (NBOM) group, (2-(trimethylsilyl)ethoxy)methyl (SEM) group, 1-(2-cyanoethoxy)ethyl group, 2-((4-methylphenyl)sulfonyl) ethoxymethyl (TEM) group, tert-butyldithio methyl (DTM) group, ((2-(methylthio)phenyl)thio)methyl (MPTM) group, (N-dichloroacetyl-N-methyl)aminobenzyloxymethyl (DCMABOM) group, (2-acetylethyl)carbonyloxymethyl group (also called "acetal levnylyl ester (ALE) group" in the pertinent field), phenylcarbamoyl group, 1,1-dioxothiomorpholine-4-thiocarbamoyl group, acetyl group, pivaloyl group, benzoyl group, trimethylsilyl group, triethylsilyl group, triisopropylsilyl group, tert-butyldimethylsilyl group, [(triisopropylsilyl)oxy]methyl (Tom) group, 1-(4-chlorophenyl)-4-ethoxypiperidin-4-yl (Cpep) group, and the like.

In the present specification, examples of the "temporary hydroxy-protecting group removable under acidic conditions" include bis(C₁₋₆ alkoxy)trityl groups such as trityl group, 9-phenyl-9-xanthenyl group, 9-phenyl-9-thioxanthenyl group, 1,1-bis(4-methoxyphenyl)-1-phenylmethyl group (sometimes referred to as "4,4'-dimethoxytrityl group" in the present specification), and the like, mono (C₁₋₁₈ alkoxy) trityl groups such as 1-(4-methoxyphenyl)-1,1-diphenylmethyl group (sometimes referred to as "4-monomethoxytrityl group" in the present specification) and the like, and the like. From the aspects of easy deprotection and the like, the aforementioned temporary protecting group is preferably a 4,4'-dimethoxytrityl group or a 4-monomethoxytrityl group, more preferably a 4,4'-dimethoxytrityl group.

In the present specification, the "amino-protecting group" is not particularly limited and known protecting groups can be used. Examples of the protecting group include pivaloyl group, pivaloyloxymethyl group, acetyl group, trifluoroacetyl group, phenoxyacetyl group, 4-isopropylphenoxyacetyl group, 4-tert-butylphenoxyacetyl group, benzoyl group, isobutyryl group, (2-hexyl)decanoyl group, dimethylformamidinyl group, 1-(dimethylamino)ethylidene group, and 9-fluorenylmethyloxycarbonyl group. Among these, acetyl group, phenoxyacetyl group, 4-isopropylphenoxyacetyl group, benzoyl group, isobutyryl group, (2-hexyl)decanoyl group, dimethylformamidinyl group, and a group represented by =NC(R¹¹)-N(R¹²)(R¹³) wherein R¹¹ is a methyl group, R¹² and R¹³ are each independently a C₁₋₆ alkyl group, or R¹¹ and R¹² are optionally joined to form, together with a carbon atom and a nitrogen atom bonded thereto, a 5- or 6-membered nitrogen-containing heterocycle), and the like can be mentioned. Examples of the aforementioned group represented by =NC(R¹¹)-N(R¹²)(R¹³) include 1-(dimethylamino)ethylidene group.

In the present specification, the "phosphate-protecting group" is not particularly limited, and known protecting groups can be used. Examples of the phosphate-protecting group include the following:
2-cyanoethyl group represented by the following formula (Pg-1), 2-[2-(4,4'-dimethoxytrityloxy)ethylsulfonyl]ethyl group represented by the following formula (Pg-2),
[3-(4,4'-dimethoxytrityloxy)-2,2-di(ethoxycarbonyl)]propyl group represented by the following formula (Pg-3),
[3-(4,4'-dimethoxytrityloxy)-2,2-di(N-methylcarbamoyl)]propyl group represented by the following formula (Pg-4).

The phosphate-protecting group is preferably a 2-cyanoethyl group. 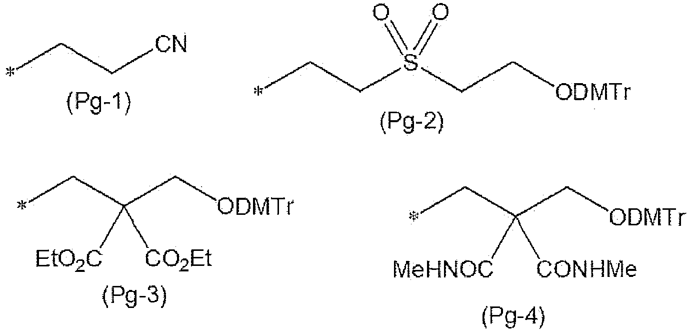 wherein Me is a methyl group, Et is an ethyl group, DMTr is a 4,4'-dimethoxytrityl group, and * is a binding position to the phosphate group.

As the "hydrophobic protecting group" in the present specification, for example, the protecting groups described as "anchor" in WO 2017/104836 can be used.

The hydrophobic protecting group is preferably a group represented by the following formula (Pg-5):

**L-Y-Z (Pg-5)

wherein
** is a binding position to the protected group;
L is a single bond, or a group represented by the formula (Pg-i-1) or (Pg-i-2): 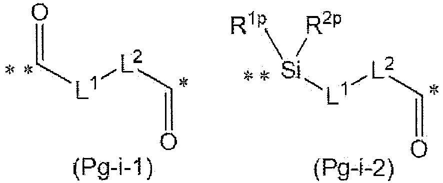
wherein
* is a binding position to Y;
** is as defined above;
R^{1p} and R^{2p} are each independently a C₁₋₂₂ hydrocarbon group;
L¹ is an optionally substituted divalent C₁₋₂₂ hydrocarbon group wherein -CH₂- in the aforementioned divalent C₁₋₂₂ hydrocarbon group may be replaced with a linker;
L² is a single bond or a group represented by ***C(=O)N(R^{3p})-R^{4p}-N (R^{5p}) **** wherein *** is a binding position to L¹, **** is a binding position to C=O, R^{4p} is a C₁₋₂₂ alkylene group, R^{3p} and R^{5p} are each independently a hydrogen atom or a C₁₋₂₂ alkyl group, or R^{3p} and R^{5p} are optionally joined to form a ring;
Y is a single bond, an oxygen atom or NR (R is a hydrogen atom, an alkyl group or an aralkyl group); and
Z is a group represented by any of the formula (Pg-ii-1) to the formula (Pg-ii-5):

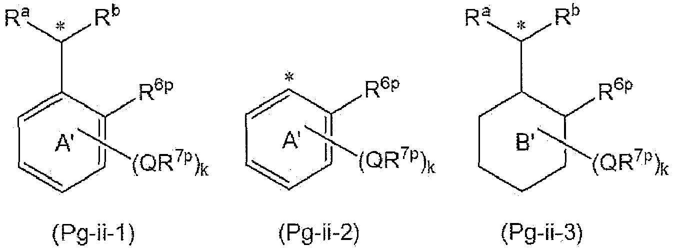

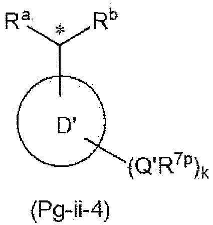

   *-(CH₂)ᵤ-R^{10p} (Pg-ii-5)

   wherein
   * indicates a binding position;
   ring A' is a benzene ring;
   ring B' is a cyclohexane ring;
   ring D' is a naphthalene ring or a bicyclic fused aromatic heterocycle;
   R^{6p} is a hydrogen atom, or when R^{b} is a group represented by the following formula (Pg-iii), R^{6p} of ring A' or ring B' optionally shows, together with R^{8p}, a single bond or -O- to form, together with ring A' or ring B' and ring C', a fused ring;
   k is an integer of 1 to 4;
   Q in the number of k are each independently -O-, -C(=O)-, - C(=O)O-, -OC(=O)-, -NR'-, -C(=O)NR'- or-NR'C(=O)- (wherein R's are each independently a hydrogen atom or a C₁₋₆ alkyl group) (Q in the number of k are preferably each independently -O-, -C(=O)-, - C(=O)O-, -OC(=O)-, -C(=O)NH-, or -NHC(=O)-);
   Q' in the number of k are each independently a single bond, -O-, -C(=O)-, -C(=O)O-, -OC(=O)-, -NR'-, -C(=O)NR'-, or-NR'C(=O)-(wherein R's are each independently a hydrogen atom or a C₁₋₆ alkyl group
   R^{7p} in the number of k are each independently a hydrocarbon group wherein a linear aliphatic hydrocarbon group having a carbon number of not less than 10 is bound via a single bond or a linker;
   ring A' and ring B', each independently, optionally have, in addition to QR^{7p} in the number of k, a substituent selected from the group consisting of a halogen atom, a C₁₋₆ alkyl group optionally substituted by a halogen atom, and a C₁₋₆ alkoxy group optionally substituted by a halogen atom;
   R^{a} is a hydrogen atom; and
   R^{b} is a hydrogen atom, or a group represented by the formula (Pg-iii): 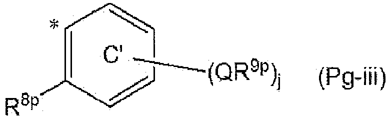 wherein
      * indicates a binding position;
      ring C' is a benzene ring:
      j is an integer of 0 to 4;
      Q in the number of j are each independently as defined above;
      R^{9p} in the number of j are each independently a hydrocarbon group wherein a linear aliphatic hydrocarbon group having a carbon number of not less than 10 is bound via a single bond or a linker;
      R^{8p} is a hydrogen atom, or optionally shows, together with R^{6p} of ring A' or ring B', a single bond or -O- to form, together with ring A' or ring B' and ring C', a fused ring;
      ring C' optionally has, in addition to QR^{9p} in the number of j, a substituent selected from the group consisting of a halogen atom, a C₁₋₆ alkyl group optionally substituted by a halogen atom, and a C₁₋₆ alkoxy group optionally substituted by a halogen atom, or
      R^{a} and R^{b} are joined to form an oxo group;
      u is 1 or 2; and
      R^{10p} is a C₂₋₂₁ perfluoroalkyl group.]]

When ring A' , ring C' , R^{6p}, and R^{8p} form a fused ring, ring A' and ring C' are each a benzene ring in the aforementioned fused ring. When ring B', ring C' , R^{6p}, and R^{8p} form a fused ring, ring C' is a benzene ring in the aforementioned fused ring, and ring B' is a cyclohexane ring in the aforementioned fused ring.

The aforementioned linear aliphatic hydrocarbon group having a carbon number of not less than 10 is preferably selected from a linear C₁₀₋₄₀ alkyl group and a linear C₁₀₋₄₀ alkenyl group, more preferably a linear C₁₀₋₄₀ alkyl group, further preferably a linear C₁₀₋₃₀ alkyl group, particularly preferably a linear C₁₂₋₂₈ alkyl group, most preferably a linear C₁₄₋₂₆ alkyl group.

The aforementioned linker is preferably -O-, -C(=O)-, - C(=O)O-, -OC(=O)-, -NR'-, -C(=O)NR'-, or -NR'C(=O)- (R' is a hydrogen atom or a C₁₋₂₂ hydrocarbon group), more preferably -O-, - C(=O)-, -C(=O)O-, -OC(=O)-, -C(=O)NH-, or -NHC(=O)-, further preferably -O-.

The aforementioned "hydrocarbon group wherein a linear aliphatic hydrocarbon group having a carbon number of not less than 10 is bound via a single bond or a linker" is preferably a linear C₁₀₋₄₀ alkyl group, a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

Q is preferably -O-, -C(=O)NH- or -NHC(=O)-, more preferably -O-.

Q' is preferably a single bond, -O-, -C(=O)NH- or -NHC(=O)-, more preferably a single bond or -O-.

In the formula (Pg-5), a preferable embodiment of L represented by the formula (Pg-i-1) is a group wherein
L¹ is a divalent C₁₋₂₂ hydrocarbon group or CH₂-O-1,4-phenylene-O-CH₂; and
L² is a single bond or a group represented by ***C(=O)N(R^{3p})-R^{4p}-N (R^{5p}) **** wherein *** is a binding position to L¹, **** is a binding position to C=O, R^{4p} is a C₁₋₆ alkylene group, R^{3p} and R^{5p} are each independently a hydrogen atom or an optionally substituted C₁₋₆ alkyl group, or R^{3p} and R^{5p} are optionally joined to form an optionally substituted C₁₋₆ alkylene group.

Another preferable embodiment of L represented by the formula (Pg-i-1) is a group wherein
L¹ is a divalent C₁₋₂₂ hydrocarbon group; and
L² is a single bond.

Another preferable embodiment of L represented by the formula (Pg-i-1) is a group wherein
L¹ is an ethylene group; and
L² is a group represented by ***C(=O)N(R^{3p})-R^{4p}-N(R^{5p})**** wherein *** is a binding position to L¹, **** is a binding position to C=O, R^{4p} is a C₁₋₂₂ alkylene group, R^{3p} and R^{5p} are each independently a hydrogen atom or a C₁₋₂₂ alkyl group, or R^{3p} and R^{5p} are optionally joined to form a ring.

Another preferable embodiment of L represented by the formula (Pg-i-1) is a group wherein
L¹ is an ethylene group; and
L² is a group represented by ***C(=O)N(R^{3p})-R^{4p}-N(R^{5p})**** wherein *** is a binding position to L¹, **** is a binding position to C=O, and N(R^{3p})-R^{4p}-N(R^{5p}) moiety forms a 1,4-piperazinediyl group.

Another preferable embodiment of L represented by the formula (Pg-i-1) is a group wherein
L¹ is an ethylene group; and
L² is a group represented by ***C (=O) N (R^{3p}) -R^{4p}-N (R^{5p}) **** wherein *** is a binding position to L¹, **** is a binding position to C=O, R^{4p} is a pentylene group or a hexylene group, and R^{3p} and R^{5p} are each independently a hydrogen atom or a methyl group.

Another preferable embodiment of L represented by the formula (Pg-i-1) is a group wherein
L¹ is a phenylene group optionally having substituent(s); and
L² is a group represented by ***C(=O)N(R^{3p})-R^{4p}-N(R^{5p})**** wherein *** is a binding position to L¹, **** is a binding position to C=O, and N(R^{3p})-R^{4p}-N(R^{5p}) moiety forms a 1,4-piperazinediyl group.

L represented by the formula (Pg-i-1) is particularly preferably a succinyl group.

In the formula (Pg-5), L represented by the formula (Pg-i-2) is explained below.

L¹ in the formula (Pg-i-2) is preferably a divalent C₆₋₁₀ aromatic hydrocarbon group, more preferably a phenylene group.

L² in the formula (Pg-i-2) is preferably a single bond.

A preferable combination of L¹ and L² in the formula (Pg-i-2) is a combination of a divalent C₆₋₁₀ aromatic hydrocarbon group for L¹ and a single bond for L². A more preferable combination of L¹ and L² in the formula (Pg-i-2) is a combination of a phenylene group for L¹ and a single bond for L².

R^{1p} and R^{2p} in the formula (Pg-i-2) are each independently preferably a C₁₋₂₂ alkyl group, more preferably a C₁₋₁₀ alkyl group.

A preferable embodiment of L represented by the formula (Pgi-2) is a group wherein
R^{1p} and R^{2p} are each independently a C₁₋₂₂ alkyl group;
L¹ is a divalent C₆₋₁₀ aromatic hydrocarbon group; and
L² is a single bond.

Another preferable embodiment of L represented by the formula (Pg-i-2) is a group wherein
R^{1p} and R^{2p} is are each independently a C₁₋₁₀ alkyl group;
L¹ is a phenylene group; and
L² is a single bond.

When Y in the formula (Pg-5) is NR, the aforementioned R is preferably a hydrogen atom, a C₁₋₆ alkyl group or a C₇₋₁₆ aralkyl group, more preferably a hydrogen atom, methyl, ethyl or benzyl, further preferably a hydrogen atom. Y is preferably a single bond, an oxygen atom or NR, more preferably a single bond or an oxygen atom.

In the formula (Pg-ii-1), R^{6p} is preferably a hydrogen atom. In the formula (Pg-ii-1), R^{a} and R^{b} are each preferably a hydrogen atom, or are joined to form an oxo group.

A preferable embodiment of Z represented by the formula (Pg-ii-1) is a group wherein
R^{a} and R^{b} are hydrogen atoms;
R^{6p} is a hydrogen atom;
k is an integer of 1 to 3;
Q in the number of k are -O-; and
R^{7p} in the number of k are each independently a linear C₁₀₋₄₀ alkyl group.

Another preferable embodiment of Z represented by the formula (Pg-ii-1) is a group wherein
R^{a} and R^{b} are hydrogen atoms;
R^{6p} is a hydrogen atom;
k is an integer of 1 to 3;
Q in the number of k are -O-; and
R^{7p} in the number of k are each independently a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

Another preferable embodiment of Z represented by the formula (Pg-ii-1) is a group wherein
R^{a} is a hydrogen atom;
R^{6p} is a hydrogen atom;
k is an integer of 1 to 3;
Q in the number of k are -O-; and
R^{7p} in the number of k are each independently a linear C₁₀₋₄₀ alkyl group; and
R^{b} is a group represented by the formula (Pg-iii) wherein * indicates a binding position, j is an integer of 0 to 3, Q in the number of j are -O-, R^{9p} in the number of j are each independently a linear C₁₀₋₄₀ alkyl group, and R^{8p} is a hydrogen atom.

Another preferable embodiment of Z represented by the formula (Pg-ii-1) is a group wherein
R^{a} is a hydrogen atom;
k is an integer of 1 to 3;
Q in the number of k are -O-; and
R^{7p} in the number of k are each independently a linear C₁₀₋₄₀ alkyl group; and
R^{b} is a group represented by the formula (Pg-iii) wherein * indicates a binding position, j is an integer of 0 to 3, Q in the number of j are -O-, R^{9p} in the number of j are each independently a linear C₁₀₋₄₀ alkyl group, and R^{8p} optionally shows, together with R^{6p}, a single bond or -O- to form that forms, together with ring A' and ring C', a fused ring.

Another preferable embodiment of Z represented by the formula (Pg-ii-1) is a group wherein
R^{a} and R^{b} are joined to form an oxo group;
R^{5p} is a hydrogen atom;
k is an integer of 1 to 3;
Q in the number of k are -O-; and
R^{7p} in the number of k are each independently a linear C₁₀₋₄₀ alkyl group.

Another preferable embodiment of Z represented by the formula (Pg-ii-1) is a group wherein
R^{a} and R^{b} are joined to form an oxo group;
R^{6p} is a hydrogen atom;
k is an integer of 1 to 3;
Q in the number of k are -O-; and
R^{7p} in the number of k are each independently a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

A preferred embodiment of Z represented by the formula (Pg-ii-2) is a group wherein
R^{6p} is a hydrogen atom;
k is an integer of 1 to 3;
Q in the number of k are -O-; and
R^{7p} in the number of k are each independently a linear C₁₀₋₄₀ alkyl group, a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

In the formula (Pg-ii-3), R^{6p} is preferably a hydrogen atom. In the formula (Pg-ii-3), R^{a} and R^{b} are preferably hydrogen atoms, or are joined to form an oxo group.

A preferable embodiment of Z represented by the formula (Pg-ii-3) is a group wherein
R^{a} and R^{b} are hydrogen atoms;
R^{6p} is a hydrogen atom;
k is an integer of 1 to 3;
Q in the number of k are -O-; and
R^{7p} in the number of k are each independently a linear C₁₀₋₄₀ alkyl group.

Another preferable embodiment of Z represented by the formula (Pg-ii-3) is a group wherein
R^{a} and R^{b} are hydrogen atoms;
R^{6p} is a hydrogen atom;
k is an integer of 1 to 3;
Q in the number of k are -O-; and
R^{7p} in the number of k are each independently a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

Another preferable embodiment of Z represented by the formula (Pg-ii-3) is a group wherein
R^{a} is a hydrogen atom;
R^{6p} is a hydrogen atom;
k is an integer of 1 to 3;
Q in the number of k are -O-;
R^{7p} in the number of k are each independently a linear C₁₀₋₄₀ alkyl group; and
R^{b} is a group represented by the formula (Pg-iii) wherein * indicates a binding position, j is an integer of 0 to 3, Q in the number of j are -O-, R^{9p} in the number of j is are each independently a C₁₀₋₄₀ alkyl group, and R^{8p} is a hydrogen atom.

Another preferable embodiment of Z represented by the formula (Pg-ii-3) is a group wherein
R^{a} is a hydrogen atom;
k is an integer of 1 to 3;
Q in the number of k are -O-;
R^{7p} in the number of k are each independently a linear C₁₀₋₄₀ alkyl group; and
R^{b} is a group represented by the formula (Pg-iii) wherein * indicates a binding position, j is an integer of 0 to 3, Q in the number of j are -O-, R^{9p} in the number of j is are each independently a C₁₀₋₄₀ alkyl group, and R^{8p} shows, together with R^{6p}, a single bond or -O- to form, together with ring B' and ring C', a fused ring.

Another preferable embodiment of Z represented by the formula (Pg-ii-3) is a group wherein
R^{a} and R^{b} are joined to form an oxo group;
R^{6p} is a hydrogen atom;
k is an integer of 1 to 3;
Q in the number of k are -O-; and
R^{7p} in the number of k are each independently a linear C₁₀₋₄₀ alkyl group.

Another preferable embodiment of Z represented by the formula (Pg-ii-3) is a group wherein
R^{a} and R^{b} are joined to form an oxo group;
R^{6p} is a hydrogen atom;
k is an integer of 1 to 3;
Q in the number of k are -O-;
R^{7p} in the number of k are each independently a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

A preferable embodiment of Z represented by the formula (Pg-ii-4) is a group wherein
ring D' is a naphthalene ring;
R^{a} and R^{b} are hydrogen atoms;
k is an integer of 1 to 3;
Q' in the number of k are -O-; and
R^{7p} in the number of k are each independently a linear C₁₀₋₄₀ alkyl group, a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-.

Another preferable embodiment of Z represented by the formula (Pg-ii-4) is a group wherein
ring D' is an indole ring;
R^{a} and R^{b} are hydrogen atoms;
k is 13;
Q' is a single bond;
R^{7p} in the number of k are each independently a linear C₁₀₋₄₀ alkyl group, a benzyl group to which 1 to 3 linear C₁₀₋₄₀ alkyl groups are bound via -O-, or a cyclohexylmethyl group to which 1 to 3 linear C₁₀₋₄₀ alkyl groups are bound via -O-; and
R^{7p} binds to a nitrogen atom of the indole ring.

A preferable embodiment of Z represented by the formula (Pg-ii-5) is a group wherein
u is 1 or 2; and
_{R}10^{p} is a C₄₋₁₀ perfluoroalkyl group.

Z is preferably a group represented by the formula (Pg-ii-1), the formula (Pg-ii-2), or the formula (Pg-ii-3).

Protecting group (Pg-5) is preferably a group wherein
L is a succinyl group, or a group represented by the formula (Pg-i-2) (in the formula (Pg-i-2), R^{1p} and R^{2p} are each independently a C₁₋₁₀ alkyl group, L¹ is a divalent phenylene group, L² is a single bond), and
Y-Z is a 3,4,5-tris(octadecyloxy)benzyloxy group, a 3,5-bis(docosyloxy)benzyloxy group, a 3,5-bis[3',4',5'-tris(octadecyloxy)benzyloxy]benzyloxy group, a 3,4,5-tris[3',4',5'-tris(octadecyloxy)benzyloxy]benzyloxy group, a 3,4,5-tris(octadecyloxy)benzylamino group, a 2,4-bis(docosyloxy)benzylamino group, 3,5-bis(docosyloxy)benzylamino group, a bis(4-docosyloxyphenyl)methylamino group, a 4-methoxy-2-[3',4',5'-tris(octadecyloxy)benzyloxy]benzylamino group, a 4-methoxy-2-[3',4',5'-tris(octadecyloxy)cyclohexylmethyloxy]benzylamino group, a 2,4-bis(dodecyloxy)benzylamino group, a phenyl(2,3,4-tris(octadecyloxy)phenyl)methylamino group, a bis[4-(12-docosyloxydodecyloxy)phenyl]methylamino group, a 3,5-bis[3',4',5'-tris(octadecyloxy)benzyloxy]benzylamino group, a 3,4,5-tris[3',4',5'-tris(octadecyloxy)benzyloxy]benzylamino group, a 3,4,5-tris(octadecyloxy)cyclohexylmethyloxy group, a 3,5-bis(docosyloxy)cyclohexylmethyloxy group, a 3,5-bis[3',4',5'-tris(octadecyloxy)cyclohexylmethyloxy]cyclohexylmethyloxy group, a 3,4,5-tris[3',4',5'-tris(octadecyloxy)cyclohexylmethyloxy]cyclohexylmethyloxy group, a 3,4,5-tris(octadecyloxy)cyclohexylmethylamino group, a 2,4-bis(docosyloxy)cyclohexylmethylamino group, a 3,5-bis(docosyloxy)cyclohexylmethylamino group, a bis(4-docosyloxycyclohexyl)methylamino group, a 4-methoxy-2-[3',4',5'-tris(octadecyloxy)cyclohexylmethyloxy]cyclohexylmethylamino group, a 4-methoxy-2-[3',4',5'-tris(octadecyloxy)cyclohexylmethyloxy]cyclohexylmethylamino group, a 2,4-bis(dodecyloxy)cyclohexylmethylamino group, phenyl(2,3,4-tris(octadecyloxy)cyclohexyl)methylamino group, a bis[4-(12-docosyloxydodecyloxy)cyclohexyl]methylamino group, a 3,5-bis[3' ,4' ,5'-tris(octadecyloxy)cyclohexylmethyloxy]cyclohexylmethylamino group, or a 3,4,5-tris[3',4',5'-tris(octadecyloxy)cyclohexylmethyloxy]cyclohexylmethylamino group, or
   a group wherein
L-Y is a single bond or a succinyl-1,4-piperazinediyl group, and
Z is a 3,4,5-tris(octadecyloxy)benzoyl group, a 3,5-bis(docosyloxy)benzoyl group, a 3,5-bis[3',4',5'-tris(octadecyloxy)benzyloxy]benzoyl group or a 3,4,5-tris[3',4',5'-tris(octadecyloxy)benzyloxy]benzoyl group.

Protecting group (Pg-5) is more preferably a group wherein
L is a succinyl group, and
Y-Z is a 3,4,5-tris(octadecyloxy)benzyloxy group, a 3,5-bis(docosyloxy)benzyloxy group, a 3,5-bis[3',4',5'-tris(octadecyloxy)benzyloxy]benzyloxy group, a 3,4,5-tris[3',4',5'-tris(octadecyloxy)benzyloxy]benzyloxy group, a 3,4,5-tris(octadecyloxy)benzylamino group, a 2,4-bis(docosyloxy)benzylamino group, a 3,5-bis(docosyloxy)benzylamino group, a bis(4-docosyloxyphenyl)methylamino group, a 4-methoxy-2-[3',4',5'-tris(octadecyloxy)benzyloxy]benzylamino group, a 4-methoxy-2-[3' ,4' ,5'-tris(octadecyloxy)cyclohexylmethyloxy]benzylamino group, a 2,4-bis(dodecyloxy)benzylamino group, a phenyl(2,3,4-tris(octadecyloxy)phenyl)methylamino group, a bis[4-(12-docosyloxydodecyloxy)phenyl]methylamino group, a 3,5-bis[3',4',5'-tris(octadecyloxy)benzyloxy]benzylamino group, a 3,4,5-tris[3',4',5'-tris(octadecyloxy)benzyloxy]benzylamino group, a 3,4,5-tris(octadecyloxy)cyclohexylmethyloxy group, a 3,5-bis(docosyloxy)cyclohexylmethyloxy group, a 3,5-bis[3',4',5'-tris(octadecyloxy)cyclohexylmethyloxy]cyclohexylmethyloxy group, a 3,4,5-tris[3',4',5'-tris(octadecyloxy)cyclohexylmethyloxy]cyclohexylmethyloxy group, a 3,4,5-tris(octadecyloxy)cyclohexylmethylamino group, a 2,4-bis(docosyloxy)cyclohexylmethylamino group, a 3,5-bis(docosyloxy)cyclohexylmethylamino group, a bis(4-docosyloxycyclohexyl)methylamino group, a 4-methoxy-2-[3',4',5'-tris(octadecyloxy)cyclohexylmethyloxy]cyclohexylmethylamino group, a 4-methoxy-2-[3',4',5'-tris(octadecyloxy)cyclohexylmethyloxy]cyclohexylmethylamino group, a 2,4-bis(dodecyloxy)cyclohexylmethylamino group, phenyl(2,3,4-tris(octadecyloxy)cyclohexyl)methylamino group, a bis[4-(12-docosyloxydodecyloxy)cyclohexyl]methylamino group, a 3,5-bis[3',4',5'-tris(octadecyloxy)cyclohexylmethyloxy]cyclohexylmethylamino group, or a 3,4,5-tris[3',4',5'-tris(octadecyloxy)cyclohexylmethyloxy]cyclohexylmethylamino group, or
   a group wherein
L-Y is a single bond or a succinyl-1,4-piperazinediyl group, and
Z is a 3,4,5-tris(octadecyloxy)benzoyl group, a 3,5-bis(docosyloxy)benzoyl group, a 3,5-bis[3',4',5'-tris(octadecyloxy)benzyloxy]benzoyl group, or a 3,4,5-tris[3',4',5'-tris(octadecyloxy)benzyloxy]benzoyl group.

Protecting group (Pg-5) is further preferably a group wherein
L is a succinyl group, and
Y-Z is a 3,4,5-tris(octadecyloxy)benzyloxy group, a 3,4,5-tris(octadecyloxy)cyclohexylmethyloxy group, a 3,5-bis(docosyloxy)cyclohexylmethyloxy group, a 3,5-bis[3',4',5'-tris(octadecyloxy)cyclohexylmethyloxy]cyclohexylmethyloxy group, a 3,4,5-tris[3',4',5'-tris(octadecyloxy)cyclohexylmethyloxy]cyclohexylmethyloxy group, a 3,4,5-tris(octadecyloxy)cyclohexylmethylamino group, a 2,4-bis(docosyloxy)cyclohexylmethylamino group, a 3,5-bis(docosyloxy)cyclohexylmethylamino group, a 4-methoxy-2-[3',4',5'-tris(octadecyloxy)cyclohexylmethyloxy]cyclohexylmethylamino group, a 4-methoxy-2-[3',4',5'-tris(octadecyloxy)cyclohexylmethyloxy]cyclohexylmethylamino group, a 2,4-bis(dodecyloxy)cyclohexylmethylamino group, a 3,5-bis[3',4',5'-tris(octadecyloxy)cyclohexylmethyloxy]cyclohexylmethylamino group, or a 3,4,5-tris[3',4',5'-tris(octadecyloxy)cyclohexylmethyloxy]cyclohexylmethylamino group, or
   a group wherein
L-Y is a single bond or a succinyl-1,4-piperazinediyl group, and
Z is a 3,4,5-tris(octadecyloxy)benzoyl group.

Protecting group (Pg-5) is particularly preferably a group wherein
L is a succinyl group, and
Y-Z is a 3,4,5-tris(octadecyloxy)benzyloxy group, a 3,4,5-tris(octadecyloxy)cyclohexylmethyloxy group or a phenyl(2,3,4-tris(octadecyloxy)phenyl)methylamino group, or
   a group wherein
L-Y is a succinyl-1,4-piperazinediyl group, and
Z is a 3,4,5-tris(octadecyloxy)benzoyl group.

Protecting group (Pg-5) is most preferably a group wherein
L is a succinyl group, and
Y-Z is a 3,4,5-tris(octadecyloxy)benzyloxy group or a 3,4,5-tris(octadecyloxy)cyclohexylmethyloxy group, or
   a group wherein
L-Y is a succinyl-1,4-piperazinediyl group, and
Z is a 3,4,5-tris(octadecyloxy)benzoyl group.

The protecting group (Pg-5) and a compound used to form the protecting group can be formed or produced by known methods (e.g., the method described in WO 2017/104836, WO 2019/131719, WO 2020/235658, WO 2021/039935, or WO 2021/198883) or a method analogous thereto.

### 1-2. Terms relating to oligonucleotide synthesis

In the present specification, the "solid support" is not particularly limited as long as it is used for the solid phase synthesis in oligonucleotide synthesis in the pertinent field, and examples thereof include glass bead, resin bead, and the like. The supports and resins used as solid supports are known in the pertinent technical field, and may be any support or resin suitable for use in solid phase syntheses. Examples of the aforementioned support or resin include polystyrene support (polystyrene support may be further functionalized by, for example, p-methylbenzyl-hydrylamine), diatomaceous earth-encapsulated polydimethylacrylamide (pepsin K), silica, fine pore glass, amphiphilic polystyrene-polyethylene glycol (PEG) resin, PEG-polyamide, PEG-polyester resin, Wang-PEG resin, Rink-amide PEG resin, and the like. The aforementioned supports and resins may be modified. Examples of the modified support or resin include long chain alkylamino Controlled Pore Glass (lcaa CPG) and the like.

In the present specification, the "nucleoside" to be the constitutional unit of oligonucleotide means a compound in which a nucleic acid base is bound to the 1-position of sugar (e.g., 2-deoxyribose, ribose, 2-deoxyribose or ribose in which the 2-position carbon atom and the 4-position carbon atom are bound by a divalent organic group, 2-deoxyribose or ribose in which the 3-position carbon atom and the 5-position carbon atom are bound by a divalent organic group, and 2-deoxyribose or ribose in which the 3-position carbon atom and the 4-position carbon atom are bound by a divalent organic group, and the like) by N-glycosidation.

In the present specification, the "nucleic acid base" is not particularly limited as long as it is used for the synthesis of nucleic acids. Examples thereof include pyrimidine bases such as cytosyl group (cytosine base), uracil group (uracil base), thyminyl group (thymine base), and the like, purine bases such as adenyl group (adenine base), guanyl group (guanine base), and the like. The "optionally protected nucleic acid base" means, for example, that the amino group, carbonyl group, and the like in the nucleic acid base may be protected. The optionally protected nucleic acid base is preferably a nucleic acid base in which the amino group may be protected by the aforementioned amino-protecting group, more preferably a nucleic acid base having a nucleic acid base without an amino group or a nucleic acid base having an amino group protected by the aforementioned amino-protecting group.

The carbonyl group can be protected, for example, by reacting carbonyl group with phenol, 2,5-dichlorophenol, 3-chlorophenol, 3,5-dichlorophenol, 2-formylphenol, 2-naphthol, 4-methoxyphenol, 4-chlorophenol, 2-nitrophenol, 4-nitrophenol, 4-acetylaminophenol, pentafluorophenol, 4-pivaloyloxybenzyl alcohol, 4-nitrophenethyl alcohol, 2-(methylsulfonyl)ethanol, 2-(phenylsulfonyl)ethanol, 2-cyanoethanol, 2-(trimethylsilyl)ethanol, dimethylcarbamoyl chloride, diethylcarbamoyl chloride, ethylphenylcarbamoyl chloride, 1-pyrrolidinecarbonyl chloride, 4-morpholinecarbonyl chloride, diphenylcarbamoyl chloride, and the like.

In the present specification, a modified nucleic acid base (e.g., 8-bromoadenyl group, 8-bromoguanyl group, 5-bromocytosyl group, 5-iodocytosyl group, 5-bromouracil group, 5-iodouracil group, 5-fluorouracil group, 5-methylcytosyl group, 8-oxoguanyl group, hypoxanthinyl group, etc.), which is substituted by a substituent (e.g., halogen atom, alkyl group, aralkyl group, alkoxy group, acyl group, alkoxyalkyl group, hydroxy group, amino group, monoalkylamino, dialkylamino, carboxy, cyano, nitro, etc.), is also encompassed in the "nucleic acid base".

The "sugar" in the present specification also encompasses an amino sugar in which the hydroxy group is replaced by an amino group, and a ribose in which the hydroxy group at the 2-position is replaced by a halogen atom.

Examples of the amino sugar include 2-deoxyribose in which the hydroxy group at the 3-position is replaced by an amino group, ribose in which the hydroxy group at the 3-position is replaced by an amino group, and ribose in which the hydroxy group at the 3-position is replaced by an amino group and the hydroxy group at the 2-position is replaced by a halogen, as shown below (in the following formulas, X^{s} is a halogen atom).

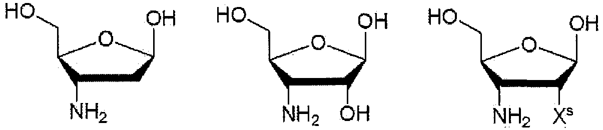

Examples of the 2-deoxyribose or ribose wherein 2-position carbon atom and 4-position carbon atom are bound by a divalent organic group, the 2-deoxyribose or ribose wherein 3-position carbon atom and 5-position carbon atom are bound by a divalent organic group, or the 2-deoxyribose or ribose wherein 3-position carbon atom and 4-position carbon atom are bound by a divalent organic group include the following compounds. 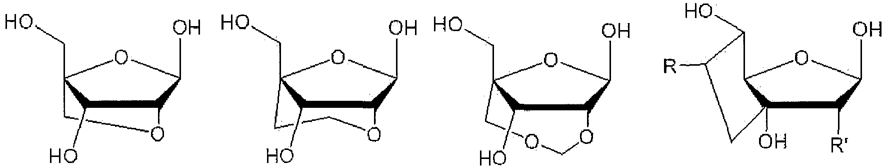 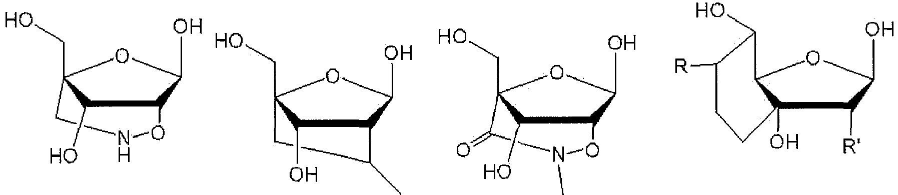 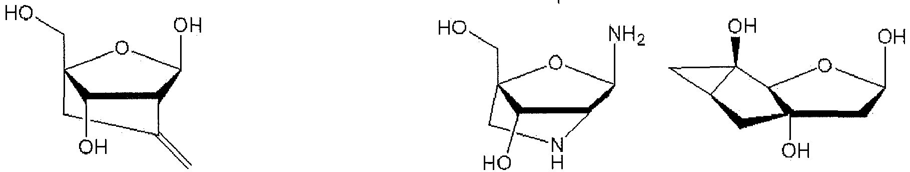 wherein R is a hydrogen atom, an optionally substituted hydrocarbon group, an optionally substituted hydroxy group, or an optionally substituted amino group, and R' is a hydrogen atom or a hydroxy group.

In the present specification, the "phosphate group" encompasses not only -O-P(=O)(OH)₂ but also a group in which an oxygen atom is replaced by a sulfur atom or NH (e.g., -OP(=S)(OH)₂, -NH-P(=O)(OH)₂, -NH-P(=S)(OH)₂). In addition, a group (e.g., protected phosphate group) in which a hydroxy group (-OH) in the phosphate group is replaced by -ORP wherein RP is an organic group (e.g., phosphate-protecting group) is also encompassed in the "phosphate group".

In the present specification, the "phosphite ester bond" is a bond represented by the following formula (P1-1) or a bond represented by the following formula (P1-2) in which an oxygen atom in the aforementioned bond is replaced by NH (in the following formulas, * is a binding position).

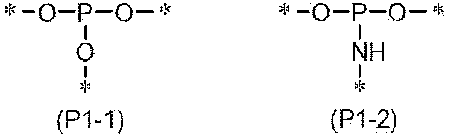

In the present specification, the "phosphonate ester bond" is a bond represented by the following formula (P2-1) or a bond represented by the following formula (P2-2) in which an oxygen atom in the aforementioned bond is replaced by NH (in the following formulas, * is a binding position).

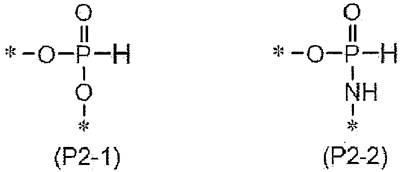

In the present specification, the "phosphate ester bond" is a bond represented by the following formula (P3-1) or a bond represented by the following formula (P3-2) in which an oxygen atom in the aforementioned bond is replaced by NH (in the following formulas, * is a binding position).

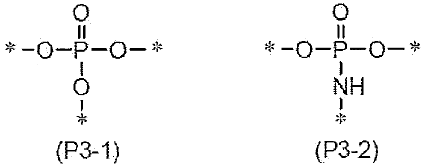

In the present specification, the "thiophosphate ester bond" is a bond represented by the following formula (P4-1) or a bond represented by the following formula (P4-2) in which an oxygen atom in the aforementioned bond is replaced by NH (in the following formulas, * is a binding position).

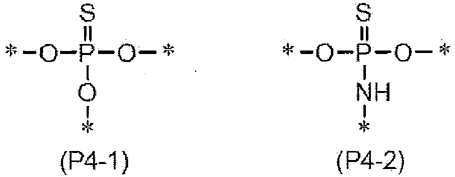

In the present specification, the "nucleotide" means a compound in which a phosphate group binds to nucleoside. The phosphate group is as described above.

In the present specification, the "oligonucleotide" means a compound in which one or more nucleotides bind to nucleoside. The "oligonucleotide" encompasses not only an oligonucleotide having a phosphate ester bond which is represented by the aforementioned formula (P3-1), but also an oligonucleotide having a phosphate ester bond which is represented by the aforementioned formula (P3-2), an oligonucleoside having a thiophosphate ester bond which is represented by the aforementioned formula (P4-1), and an oligonucleoside having a thiophosphate ester bond which is represented by the aforementioned formula (P4-2). The number of nucleosides in the oligonucleotide in the present invention is not particularly limited, and is preferably 2 to 50, more preferably 2 to 30.

In the present specification, the "phosphoramidite" means a phosphorous acid diester monoamide (P(OR)₂(NR₂) wherein 4 R's are each independently an optionally substituted alkyl group, and the aforementioned NR₂ may form a cyclic amino group by the binding of two R's to each other).

In the present specification, the "phosphoramidited nucleoside" means a compound obtained by introducing a group represented by -Xⁿ-P(OR)(NR₂) (wherein Xⁿ is an oxygen atom or NH, 3 R's are each independently an optionally substituted alkyl group, and the aforementioned NR₂ may form a cyclic amino group by the binding of two R's to each other) into nucleoside.

In the present specification, the "phosphoramidited nucleotide" means a compound having a group represented by -Xⁿ-P(OR) (NR₂) and a phosphate group, which is obtained by introducing a group represented by -Xⁿ-P(OR) (NR₂) (wherein Xⁿ is an oxygen atom or NH, 3 R's are each independently an optionally substituted alkyl group, and the aforementioned NR₂ may form a cyclic amino group by the binding of two R's to each other) into nucleotide. The aforementioned phosphate group may be substituted.

In the present specification, the "phosphoramidited oligonucleotide" means a compound obtained by introducing a group represented by -Xⁿ-P(OR) (NR₂) (wherein Xⁿ is an oxygen atom or NH, 3 R's are each independently an optionally substituted alkyl group, and the aforementioned NR₂ may form a cyclic amino group by the binding of two R's to each other) into oligonucleotide.

In the present specification, the "oligonucleotide precursor having a phosphite ester bond" means a precursor in which the phosphate ester bond in the oligonucleotide is replaced with a phosphite ester bond. An oligonucleotide having a phosphate ester bond can be produced by oxidizing this precursor.

The oligonucleotide precursor having a phosphite ester bond is preferably
(1) an oligonucleotide precursor having a phosphite ester bond which is obtained by condensation of a nucleoside, nucleotide, or oligonucleotide with a phosphoramidited nucleoside, nucleotide, or oligonucleotide, or
(2) an oligonucleotide precursor having a phosphite ester bond which is obtained by condensation of an oligonucleotide with a phosphoramidite.

The oligonucleotide precursor having a phosphite ester bond is preferably an oligonucleotide precursor having a phosphite ester bond which is obtained by condensation of a nucleoside, nucleotide, or oligonucleotide with a phosphoramidited nucleoside, nucleotide, or oligonucleotide.

The oligonucleotide precursor having a phosphite ester bond is further preferably an oligonucleotide precursor having a phosphite ester bond, which is obtained by condensation of nucleoside, nucleotide, or oligonucleotide (a), each having a hydrophobic protecting group, and nucleoside, nucleotide, or oligonucleotide (b), each of which is phosphoramidited and in which a hydroxy group is protected by a temporary protecting group removable under acidic conditions.

The aforementioned condensation may be any of condensation in which the oligonucleotide chain is extended in the direction from the 3'-end to the 5' -end (hereinafter referred to as "3'-5' condensation") and condensation in which the oligonucleotide chain is extended in the direction from the 5'-end to the 3' -end (hereinafter referred to as "5'-3' condensation"). In the following, the aforementioned nucleoside, nucleotide, or oligonucleotide (a) and the aforementioned phosphoramidited nucleoside, nucleotide, or oligonucleotide (b) that are preferred for each of the 3'-5' condensation and the 5'-3' condensation are described in order.

In the 3'-5' condensation, examples of the aforementioned nucleoside, nucleotide, or oligonucleotide (a) include a compound represented by the formula (a-I): 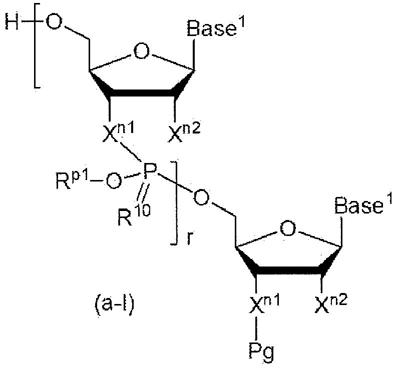 wherein
r is an integer of not less than 0;
Base¹ in the number of r+1 are each independently an optionally protected nucleic acid base;
Xⁿ¹ in the number of r+1 are each independently an oxygen atom or NH;
Xⁿ² in the number of r+1 are each independently a hydrogen atom, a halogen atom, an optionally protected hydroxy group, or a divalent organic group bound to 2-position carbon atom and 4-position carbon atom;
R¹⁰ in the number of r are each independently an oxygen atom or a sulfur atom;
R^{p1} in the number of r are each independently is a phosphate-protecting group;
Pg is **L-Y-Z (wherein ** is a binding position to Xⁿ¹), **L-Y-Sp (wherein ** is a binding position to Xⁿ¹, and Sp is a solid support) or a solid support; and
the definition and description of L, Y, and Z are the same as those for L, Y and Z in the aforementioned protecting group (Pg-5) (i.e., nucleoside or oligonucleotide).

When r is 0, compound (a-I) is a nucleoside, and when r is one or more, compound (a-I) is an oligonucleotide. r is preferably not more than 49, more preferably not more than 29, further preferably not more than 19, particularly preferably not more than 4, and most preferably not more than 2.

In the nucleic acid base Base¹, the amino group is preferably protected by a protecting group. Examples of the amino-protecting group include the aforementioned amino-protecting groups, and the amino-protecting group in Base¹ is preferably an acetyl group, a phenoxyacetyl group, a 4-isopropylphenoxyacetyl group, a benzoyl group, an isobutyryl group, a (2-hexyl)decanoyl group, a dimethylformamidinyl group, or a group represented by =NC(R¹¹)-N(R¹²)(R¹³) wherein R¹¹ is a methyl group, R¹² and R¹³ are each independently a C₁₋₆ alkyl group, or R¹¹ and R¹² are optionally joined to form, together with a carbon atom and a nitrogen atom bonded thereto, a 5- or 6-membered nitrogen-containing heterocycle). When compound (a-I) has plural amino groups, only one kind of the amino-protecting group may be used or two or more kinds thereof may be used..

As the halogen atom for Xⁿ², a fluorine atom and a chlorine atom are preferred, and a fluorine atom is more preferred.

Examples of the optionally protected hydroxy-protecting group for Xⁿ² include the aforementioned hydroxy-protecting group, preferably methyl group, 2-methoxyethyl group, triethylsilyl group, triisopropylsilyl group, tert-butyldimethylsilyl group, (2-cyanoethoxy)methyl group, or 1-(2-cyanoethoxy)ethyl group.

The "divalent organic group bound to 2-position carbon atom and 4-position carbon atom" for Xⁿ² is not particularly limited as long as it is bound to 2-position carbon atom and 4-position carbon atom of nucleoside. Examples of the divalent organic group include an optionally substituted C₂₋₇ alkylene group, and a divalent organic group constituted of an optionally substituted C₁₋₇ alkylene group and a divalent linker selected from -O-, -NR³³-(R³³ is a hydrogen atom or a C₁₋₆ alkyl group), -S-, -CO-, -COO-, - OCONR³⁴- (R³⁴ is a hydrogen atom or a C₁₋₆ alkyl group), and -CONR³⁵-(R³⁵ is a hydrogen atom or a C₁₋₆ alkyl group), and the like. Examples of the substituent that the C₁₋₇ alkylene group and C₂₋₇ alkylene group optionally have include a methylidene group (CH₂=).

As the "divalent organic group bound to 2-position carbon atom and 4-position carbon atom", an optionally substituted C₂₋₇ alkylene group, -ORⁱ- (Rⁱ is a C₁₋₆ alkylene group bound to 4-position carbon atom), -O-NR³³-R^{j}- (R^{j} is a C₁₋₆ alkylene group bound to 4-position carbon atom, R³³ is as defined above), and -O-R^{k}-O-R^{l}-(R^{k} is a C₁₋₆ alkylene group, R^{l} is a C₁₋₆ alkylene group bound to and crosslinked with 4-position carbon atom) are preferred, and -ORⁱ-(Rⁱ is as defined above), -O-NR³³-R^{j}- (R^{j} and R³³ are as defined above), and -O-R^{k}-O-R^{l}- (R^{k} and R^{l} are as defined above) are more preferred. C₁₋₆ alkylene groups for Rⁱ, R^{j}, R^{k} and R^{l} are preferably each independently a methylene group or an ethylene group.

As the "divalent organic group bound to 2-position carbon atom and 4-position carbon atom", -O-CH₂-, -O-CH₂-CH₂-, -O-NR³³-CH₂-(R³³ is as defined above), and -O-CH₂-O-CH₂- are more preferred, and -O-CH₂-, -O-CH₂-CH₂-, -O-NH-CH₂-, -O-N(CH₃)-CH₂-, -O-CH₂-O-CH₂- (in each of which the left side binds to 2-position carbon atom and the right side binds to 4-position carbon atom) are further preferred.

Xⁿ² in the number of r+1 are each independently preferably a hydrogen atom, a halogen atom or an optionally protected hydroxy group, more preferably a hydrogen atom or an optionally protected hydroxy group.

Examples of the R^{p1} in the number of r include the aforementioned phosphate-protecting group, preferably 2-cyanoethyl group.

When Pg in the aforementioned formula (a-I) is **L-Y-Sp (wherein ** is a binding position to Xⁿ¹, and Sp is a solid support) or a solid support, compound (a-I) is a nucleoside or oligonucleotide carried on a solid support. Pg is preferably **L-Y-Z (wherein ** is a binding position to Xⁿ¹) or a solid support, more preferably **L-Y-Z. The definition and description of L, Y, and Z are the same as those for L, Y and Z in the aforementioned protecting group (Pg-5).

In the 3'-5'condensation, examples of the aforementioned phosphoramidited nucleoside, nucleotide, or oligonucleotide (b) include a compound represented by the formula (b-I): 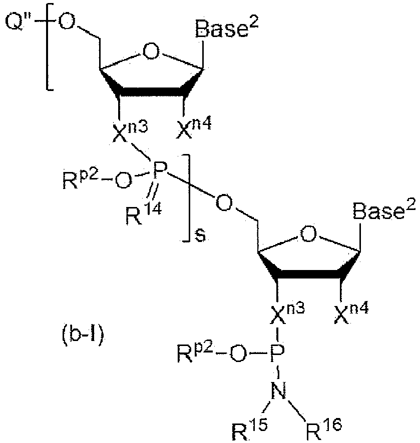 wherein
s is an integer of not less than 0;
Base² in the number of s+1 are each independently an optionally protected nucleic acid base;
Xⁿ³ in the number of s+1 are each independently an oxygen atom or NH;
Xⁿ⁴ in the number of s+1 are each independently a hydrogen atom, a halogen atom, an optionally protected hydroxy group, or a divalent organic group bound to 2-position carbon atom and 4-position carbon atom;
Q" is a temporary hydroxy-protecting group removable under acidic conditions;
R¹⁴ in the number of s are each independently an oxygen atom or a sulfur atom;
R^{p2} in the number of s+1 are each independently a phosphate-protecting group; and
R¹⁵ and R¹⁶ are each independently an alkyl group, or a 5- or 6-membered saturated cyclic amino group formed together with the adjacent nitrogen atom, and the saturated cyclic amino group, optionally has, as a ring-constituting atom, one oxygen atom or sulfur atom besides nitrogen atom (i.e., nucleoside or oligonucleotide).

When s is 0, compound (b-I) is a phosphoramidited nucleoside, and when s is one or more, compound (b-I) is a phosphoramidited oligonucleotide. In compound (b-I), s is preferably not more than 49, more preferably not more than 29, further preferably not more than 19, particularly preferably not more than 4, and most preferably not more than 2.

In the nucleic acid base Base², the amino group is preferably protected by a protecting group. As the protecting group, the aforementioned -L-Y-Z group can be used in addition to the aforementioned amino-protecting group. The protecting group is preferably the aforementioned amino-protecting group, more preferably an acetyl group, a phenoxyacetyl group, a 4-isopropylphenoxyacetyl group, a benzoyl group, an isobutyryl group, a (2-hexyl)decanoyl group, a dimethylformamidinyl group, or a group represented by =NC(R¹¹)-N(R¹²)(R¹³) wherein R¹¹ is a methyl group, R¹² and R¹³ are each independently a C₁₋₆ alkyl group, or R¹¹ and R¹² are optionally joined to form, together with a carbon atom and a nitrogen atom bonded thereto, a 5-membered or 6-membered nitrogen-containing heterocycle. When compound (b-I) has plural amino groups, the amino-protecting group may be only one kind or two or more kinds.

The explanation on Xⁿ⁴ in the aforementioned formula (b-I) is the same as that on Xⁿ² in the aforementioned formula (a-I) . Xⁿ⁴ in the number of s+1 are each independently preferably a hydrogen atom, a halogen atom, or an optionally protected hydroxy group, more preferably a hydrogen atom or an optionally protected hydroxy group.

The explanation on R^{p2} in the aforementioned formula (b-I) is the same as that on R^{p1} in the aforementioned formula (a-I).

Examples of the R^{p2} in the number of s+1 include the aforementioned phosphate-protecting groups, preferably 2-cyanoethyl group.

R¹⁵ and R¹⁶ in the aforementioned formula (b-I) are each independently preferably a C₁₋₁₀ alkyl group or a 5- or 6-membered saturated cyclic amino group formed together with the adjacent nitrogen atom, more preferably a C₁₋₁₀ alkyl group, further preferably a C₁₋₆ alkyl group.

Compound (a-I) and compound (b-I) can be produced by known methods (e.g., the method described in WO 2017/104836) or a method analogous thereto.

The oligonucleotide precursor having a phosphite ester bond which is obtained by condensation of compound (a-I) and compound (b-I) is a compound represented by the following formula (c-I) (definition and explanation of symbols in the following formula (c-I) are as described above).

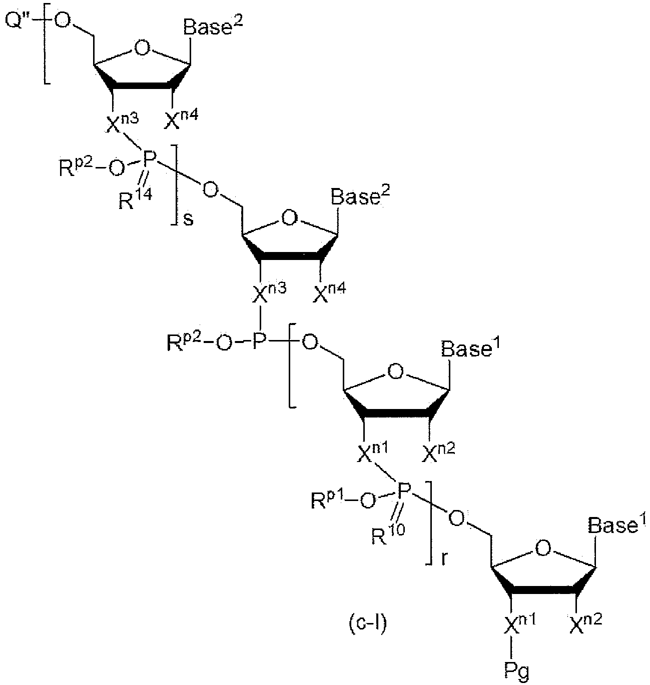

When Pg in the aforementioned formula (c-I) is **L-Y-Sp (wherein ** is a binding position to Xⁿ¹, and Sp is a solid support) or a solid support, compound (c-I) is an oligonucleotide precursor carried on a solid support. Pg is preferably **L-Y-Z (wherein ** is a binding position to Xⁿ¹) or a solid support, more preferably **L-Y-Z.

In the aforementioned formula (c-I), at least one of R¹⁴ in the number of s and R¹⁰ in the number of r is preferably a sulfur atom.

In the 5'-3'condensation, examples of the aforementioned nucleoside, nucleotide, or oligonucleotide (a) include a compound represented by the formula (a-I'): 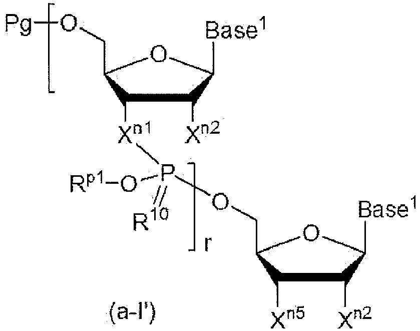 wherein
r is an integer of not less than 0;
Base¹ in the number of r+1 are each independently an optionally protected nucleic acid base;
Xⁿ¹ in the number of r+1 are each independently an oxygen atom or NH;
Xⁿ² in the number of r are each independently a hydrogen atom, a halogen atom, an optionally protected hydroxy group, or a divalent organic group bound to 2-position carbon atom and 4-position carbon atom;
Xⁿ⁵ is a hydroxy group or an amino group;
R¹⁰ in the number of r are each independently an oxygen atom or a sulfur atom;
R^{p1} in the number of r are each independently is a phosphate-protecting group;
Pg is **L-Y-Z (wherein ** is a binding position to oxygen atom), **L-Y-Sp (wherein ** is a binding position to Xⁿ¹, and Sp is a solid support) or a solid support; and
the definition and description of L, Y, and Z are the same as those for L, Y and Z in the aforementioned protecting group (Pg-5) (i.e., nucleoside or oligonucleotide).

Xⁿ⁵ in the formula (a-I') is preferably a hydroxy group. The explanation on the symbols other than Xⁿ⁵ is the same as that on the symbols in the aforementioned formula (a-I).

When Pg in the aforementioned formula (a-I') is **L-Y-Sp (wherein ** is a binding position to Xⁿ¹, and Sp is a solid support) or a solid support, compound (a-I') is a nucleoside or oligonucleotide carried on a solid support. Pg is preferably **L-Y-Z (wherein ** is a binding position to oxygen atom) or a solid support, more preferably **L-Y-Z.

In the 5'-3'condensation, examples of the aforementioned phosphoramidited nucleoside, nucleotide, or oligonucleotide (b) include a compound represented by the formula (b-I'): 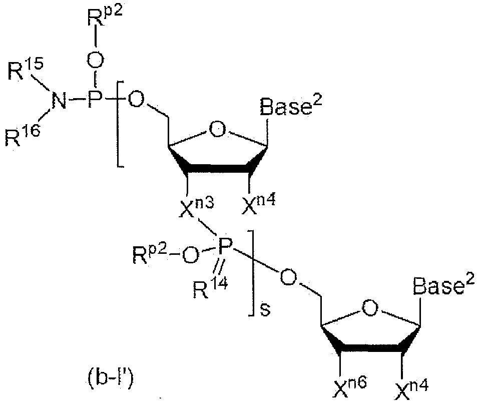 wherein
s is an integer of not less than 0;
Base² in the number of s+1 are each independently an optionally protected nucleic acid base;
Xⁿ³ in the number of s are each independently an oxygen atom or NH;
Xⁿ⁴ in the number of s+1 are each independently a hydrogen atom, a halogen atom, an optionally protected hydroxy group, or a divalent organic group bound to 2-position carbon atom and 4-position carbon atom;
Xⁿ⁶ is a hydroxy group protected by a temporary protecting group removable under acidic conditions, or an amino group protected by a temporary protecting group removable under acidic conditions;
R¹⁴ in the number of s are each independently an oxygen atom or a sulfur atom;
RP2 in the number of s+1 are each independently a phosphate-protecting group; and
R¹⁵ and R¹⁶ are each independently an alkyl group, or a 5- or 6-membered saturated cyclic amino group formed together with the adjacent nitrogen atom, and the saturated cyclic amino group, optionally has, as a ring-constituting atom, one oxygen atom or sulfur atom besides nitrogen atom.

Xⁿ⁶ is preferably a hydroxy group protected by a temporary protecting group removable under acidic conditions. The explanation on the symbols other than Xⁿ⁶ is the same as that on the symbols in the aforementioned formula (b-I).

Compound (a-I') and compound (b-I') can be produced by known methods (e.g., the method described in WO 2017/104836) or a method analogous thereto.

The oligonucleotide precursor having a phosphite ester bond which is obtained by condensation of compound (a-I') and compound (b-I') is a compound represented by the following formula (c-I'): 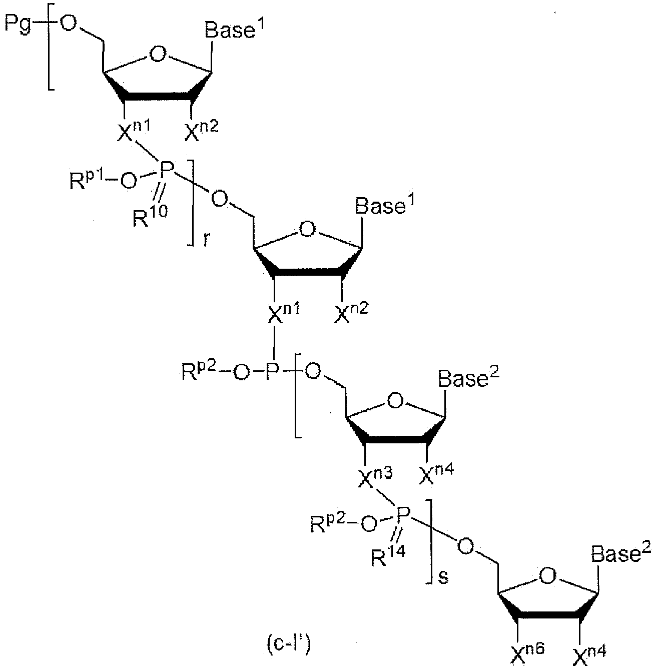 wherein
r is an integer of not less than 0;
Base¹ in the number of r+1 are each independently an optionally protected nucleic acid base;
Xⁿ¹ in the number of r+1 are each independently an oxygen atom or NH;
Xⁿ² in the number of r+1 are each independently a hydrogen atom, a halogen atom, an optionally protected hydroxy group, or a divalent organic group bound to 2-position carbon atom and 4-position carbon atom;
R¹⁰ in the number of r are each independently an oxygen atom or a sulfur atom;
R^{p1} in the number of r are each independently is a phosphate-protecting group;
s is an integer of not less than 0;
Base² in the number of s+1 are each independently an optionally protected nucleic acid base;
Xⁿ³ in the number of s are each independently an oxygen atom or NH;
Xⁿ⁴ in the number of s+1 are each independently a hydrogen atom, a halogen atom, an optionally protected hydroxy group, or a divalent organic group bound to 2-position carbon atom and 4-position carbon atom;
Xⁿ⁶ is a hydroxy group protected by a temporary protecting group removable under acidic conditions, or an amino group protected by a temporary protecting group removable under acidic conditions;
R¹⁴ in the number of s are each independently an oxygen atom or a sulfur atom;
R^{p2} in the number of s+1 are each independently a phosphate-protecting group;
Pg is **L-Y-Z (wherein ** is a binding position to oxygen atom), **L-Y-Sp (wherein ** is a binding position to Xⁿ¹, and Sp is a solid support) or a solid support; and
the definition and description of L, Y, and Z are the same as those for L, Y and Z in the aforementioned protecting group (Pg-5) (wherein the symbols are as described above).

When Pg in the aforementioned formula (c-I') is **L-Y-Sp (wherein ** is a binding position to Xⁿ¹, and Sp is a solid support) or a solid support, compound (c-I') is an oligonucleotide precursor carried on a solid support. Pg is preferably **L-Y-Z (wherein ** is a binding position to oxygen atom) or a solid support, more preferably **L-Y-Z.

In the aforementioned formula (c-I'), at least one of R¹⁰ in the number of r and R¹⁴ in the number of s is preferably a sulfur atom.

The "oligonucleotide precursor having a phosphite ester bond which is obtained by condensation of an oligonucleotide and a phosphoramidite" is described now. Examples of the aforementioned precursor include a compound represented by the formula (γ-I) or the formula (γ-I'): 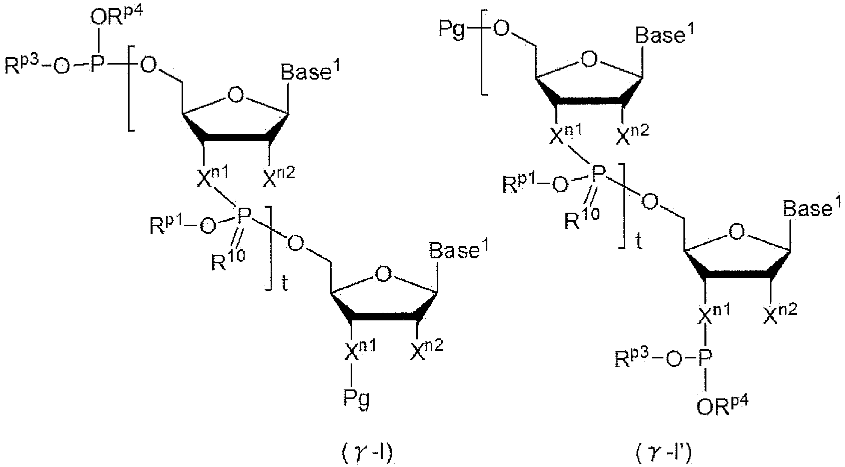 wherein
t is an integer of not less than 1,
Base¹ in the number of t+1 are each independently an optionally protected nucleic acid base;
Xⁿ¹ in the number of t+1 are each independently an oxygen atom or NH;
Xⁿ² in the number of t+1 are each independently a hydrogen atom, a halogen atom, an optionally protected hydroxy group, or a divalent organic group bound to 2-position carbon atom and 4-position carbon atom;
R¹⁰ in the number of t are each independently an oxygen atom or a sulfur atom;
R^{p1} in the number of t and R^{p3} are each independently is a phosphate-protecting group;
R^{p4} is an alkyl group having a protected amino group, or a phosphate-protecting group;
Pg is **L-Y-Z (wherein ** is a binding position to Xⁿ¹ or a binding position to oxygen atom), **L-Y-Sp (wherein ** is a binding position to Xⁿ¹, and Sp is a solid support) or a solid support; and
the definition and description of L, Y, and Z are the same as those for L, Y and Z in the aforementioned protecting group (Pg-5).

When Pg in the aforementioned formula (γ-I) or the formula (γ-I') is **L-Y-Sp (wherein ** is a binding position to Xⁿ¹, and Sp is a solid support) or a solid support, compound (γ-I) or compound (γ-I') is an oligonucleotide precursor carried on a solid support. Pg is preferably **L-Y-Z (wherein ** is a binding position to an oxygen atom) or a solid support, more preferably **L-Y-Z.

The symbols in the aforementioned formula (γ-I) and the formula (y-I') are explained. t is preferably not more than 49, more preferably not more than 29, further preferably not more than 19, particularly preferably not more than 4, and most preferably not more than 2. Examples of the "phosphate-protecting group" for RP3 or RP4 include the aforementioned phosphate-protecting groups. R^{p3} is preferably a 2-cyanoethyl group. Examples of the "protected amino group" of the "alkyl group having a protected amino group" for R^{p4} include the aforementioned amino groups protected by an amino-protecting group. The "alkyl group having a protected amino group" for R^{p4} is preferably a C₁₋₆ alkyl group having a protected amino group, more preferably a 6-(trifluoroacetylamino)hexyl group. R^{p4} is preferably a 2-cyanoethyl group or a 6-(trifluoroacetylamino)hexyl group. Other symbols are as described above.

In the present specification, the "oligonucleotide precursor having a phosphonate ester bond" means a precursor in which a phosphate ester bond in oligonucleotide is replaced with a phosphonate ester bond. By oxidizing the precursor, an oligonucleotide having a phosphate ester bond can be produced.

The oligonucleotide precursor having a phosphonate ester bond is preferably a oligonucleotide precursor having a phosphonate ester bond which is obtained by condensation of nucleoside, nucleotide, or oligonucleotide (a) having a hydrophobic protecting group and H-phosphonate (B) with a hydroxy group protected by a temporary protecting group removable under acidic conditions. The aforementioned H-phosphonate (B) may also be used in the form of a salt, for example, triethylamine salt or the like.

The aforementioned condensation may be either 3'-5'condensation or 5'-3'condensation. The aforementioned nucleoside, nucleotide, or oligonucleotide (a) and the aforementioned H-phosphonate (B) that are preferred in each of the 3'-5'condensation and 5'-3'condensation are described in order.

In the 3'-5'condensation, the aforementioned nucleoside, nucleotide, or oligonucleotide (a) is preferably the aforementioned compound (a-I).

In the 3'-5'condensation, examples of the aforementioned H-phosphonate (B) include a compound represented by the following formula (B-I) (definition and explanation of the symbols in the following formula (B-I) are the same as those of the symbols in the aforementioned formula (b-I)).

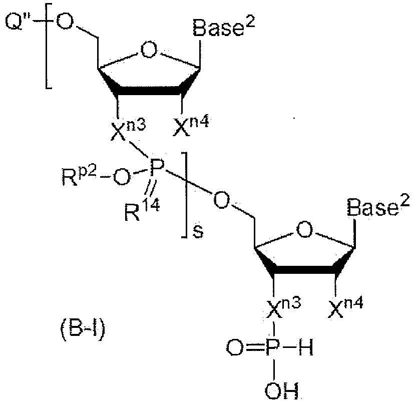

Compound (B-I) can be produced by known methods (e.g., the method described in Acta Biochimica Polonica. 1998, 45, 907-915.) or a method analogous thereto.

The oligonucleotide precursor having a phosphonate ester bond which is obtained by condensation of compound (a-I) and compound (B-I) is a compound represented by the following formula (C-I) (definition and explanation of symbols in the following formula (C-I) are the same as those of the symbols in the aforementioned formula (c-I)).

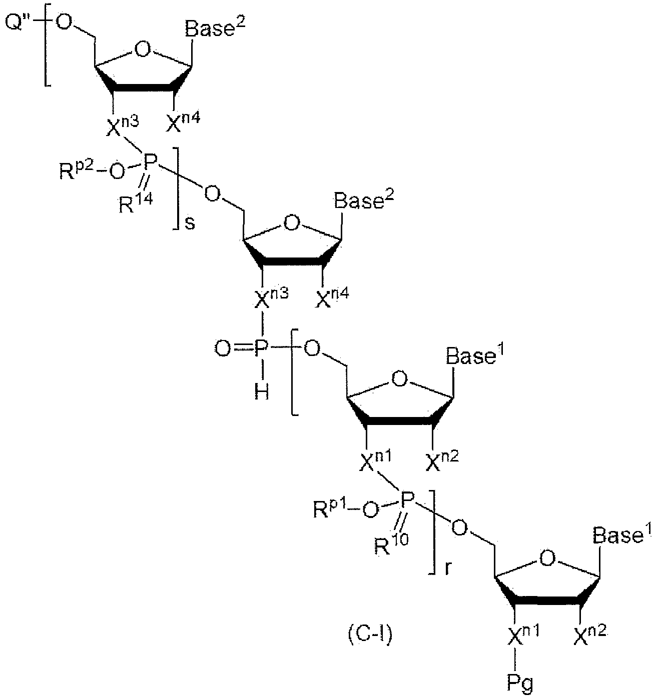

When Pg in the aforementioned formula (C-I) is **L-Y-Sp (wherein ** is a binding position to Xⁿ¹, and Sp is a solid support) or a solid support, compound (C-I) is an oligonucleotide precursor carried on a solid support. Pg is preferably **L-Y-Z (wherein ** is a binding position to Xⁿ¹) or a solid support, more preferably **L-Y-Z.

In the aforementioned formula (C-I), at least one of R¹⁴ in the number of s and R¹⁰ in the number of r is preferably a sulfur atom.

In the 5'-3'condensation, the aforementioned nucleoside, nucleotide, or oligonucleotide (a) is preferably the aforementioned compound (a-I').

In the 5'-3'condensation, examples of H-phosphonate (B) include a compound represented by the following formula (B-I') (definition and explanation of symbols in the following formula (B-I') are the same as those of the symbols in the aforementioned formula (b-I').

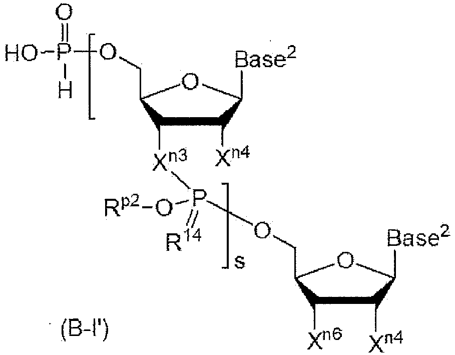

Compound (B-I') can be produced by known methods (e.g., the method described in Phosphorus Chemistry II. Topics in Current Chemistry, vol 361. Springer, Cham. Recent Advances in H-Phosphonate Chemistry) or a method analogous thereto.

The oligonucleotide precursor having a phosphonate ester bond which is obtained by condensation of compound (a-I') and compound (B-I') is a compound represented by the following formula (C-I') (definition and explanation of symbols in the following formula (C-I') are the same as those of the symbols in the aforementioned formula (c-I'):

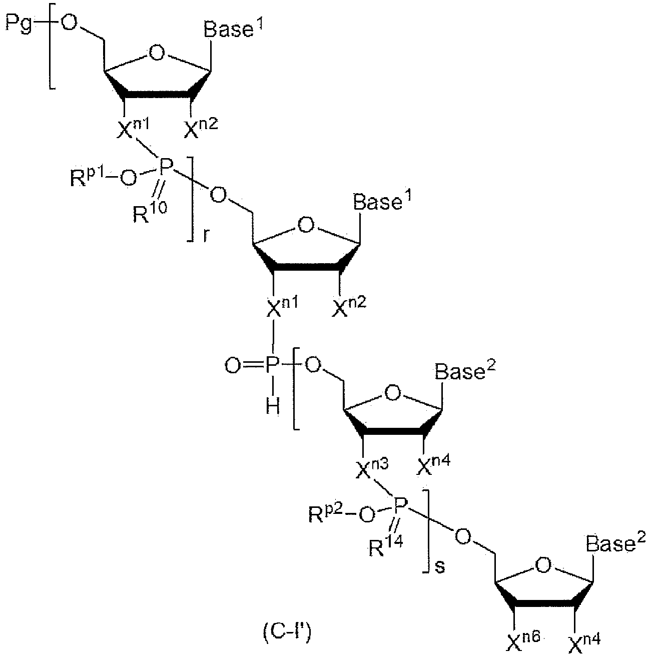

When Pg in the aforementioned formula (C-I') is **L-Y-Sp (wherein ** is a binding position to Xⁿ¹, and Sp is a solid support) or a solid support, compound (C-I) is an oligonucleotide precursor carried on a solid support. Pg is preferably **L-Y-Z (wherein ** is a binding position to oxygen atom) or a solid support, more preferably **L-Y-Z.

- In the aforementioned formula (C-I'), at least one of R₁₀ in the number of r and R¹⁴ in the number of s is preferably a sulfur atom.

In the present specification, the "one-pot synthesis" means a synthesis consisting of multiple steps, including a step of synthesizing an intermediate and a step of synthesizing the desired final product, in which the aforementioned intermediate is not isolated. The aforementioned "step of synthesizing an intermediate" may be one or two or more.

### 2. Production method of the present invention

The production method of the present invention is described below. The production method of the present invention is characterized in that an oligonucleotide having a phosphate ester bond is produced by oxidation of an oligonucleotide precursor having a phosphite ester bond or a phosphonate ester bond by using a compound represented by the following formula (I): 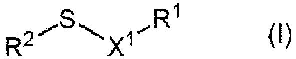 as an oxidant, provided that 2,2-dipyridyl disulfide is excluded. Only one kind of compound (I) may be used, or two or more kinds thereof may be used in combination.

### 2-1. Oxidant

Compound (I) excluding 2,2-dipyridyl disulfide which is an oxidant to be used in the production method of the present invention is described below.

In the aforementioned formula (I), X¹ is a single bond, a sulfur atom, an oxygen atom, -S(=O)₂-, or -N(-R³)-.

When X¹ is a single bond, then R¹ in the aforementioned formula (I) is a halogen atom, and R² in the aforementioned formula (I) is an optionally substituted aryl group or an optionally substituted heteroaryl group.

When X¹ is a sulfur atom or -S(=O)₂-, then R¹ in the aforementioned formula (I) is an optionally substituted alkyl group, an optionally substituted aryl group, or an optionally substituted heteroaryl group, and R² in the aforementioned formula (I) is an optionally substituted aryl group or an optionally substituted heteroaryl group.

When X¹ is an oxygen atom, then R¹ and R² in the aforementioned formula (I) form, together with a sulfur atom and an oxygen atom bonded thereto, an optionally substituted heterocycle.

When X¹ is -N(-R³)-, then
(1) R¹ in the aforementioned formula (I) is an optionally substituted alkyl group, an optionally substituted aryl group, or an optionally substituted heteroaryl group, and R² and R³ in the aforementioned formula (I) form, together with a sulfur atom and a nitrogen atom bonded thereto, an optionally substituted heterocycle, or
(2) R¹ and R³ in the aforementioned formula (I) form, together with a nitrogen atom bonded thereto, an optionally substituted heterocycle, and R² in the aforementioned formula (I) is an optionally substituted aryl group or an optionally substituted heteroaryl group.

### 2-1-1. Compound (IA) and compound (IB)

Among compounds (I),
a compound of the aforementioned formula (I) wherein X¹ is - N(-R³)-, R¹ is an optionally substituted alkyl group, an optionally substituted aryl group, or an optionally substituted heteroaryl group, and R² and R³ form, together with a sulfur atom and a nitrogen atom bonded thereto, an optionally substituted heterocycle (referred to as "compound (IA)" in the present specification), and
a compound of the aforementioned formula (I) wherein X¹ is a sulfur atom, R¹ is an optionally substituted alkyl group, an optionally substituted aryl group, or an optionally substituted heteroaryl group, and R² is an optionally substituted aryl group or an optionally substituted heteroaryl group, provided that 2,2-dipyridyl disulfide is excluded (hereinafter referred to as "compound (IB)") are preferred.

That is, among compounds (I), a compound wherein
X¹ is a sulfur atom or -N(-R³)-,
R¹ is an optionally substituted alkyl group, an optionally substituted aryl group, or an optionally substituted heteroaryl group,
when X¹ is a sulfur atom, R² is an optionally substituted aryl group or an optionally substituted heteroaryl group, and
when X¹ is -N(-R³)-, R² and R³ form, together with a sulfur atom and a nitrogen atom bonded thereto, an optionally substituted heterocycle is preferred provided that 2,2-dipyridyl disulfide is excluded.

Compound (IA) and compound (IB) are described below.

### 2-1-1A. Compound (IA)

Firstly, compound (IA) is described. In compound (IA), R¹ in the aforementioned formula (I) is an optionally substituted alkyl group, an optionally substituted aryl group, or an optionally substituted heteroaryl group, preferably an optionally substituted C₁₋₁₀ alkyl group, an optionally substituted phenyl group, an optionally substituted 5- or 6-membered monocyclic heteroaryl group, or an optionally substituted 8- to 14-membered fused polycyclic heteroaryl group.

In compound (IA), R² and R³ in the aforementioned formula (I) form, together with a sulfur atom and a nitrogen atom bonded thereto, an optionally substituted heterocycle.

### 2-1-1A-1. Compound (Ia)

Compound (IA) is preferably a compound represented by the following formula (Ia). Only one kind of compound (Ia) may be used, or two or more kinds thereof may be used in combination.

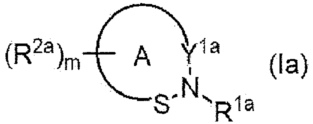

Each group in compound (Ia) is described below.

R^{1a} in the aforementioned formula (Ia) is an optionally substituted alkyl group, an optionally substituted aryl group, or an optionally substituted heteroaryl group, preferably an optionally substituted C₁₋₁₀ alkyl group, an optionally substituted phenyl group, or an optionally substituted 5- or 6-membered monocyclic heteroaryl group.

Ring A in the aforementioned formula (Ia) is a 5- or 6-membered unsaturated heterocycle. Y^{1a} in the aforementioned formula (Ia) is -S(=O)-, -S(=O)₂-, -C(=O)-, or -C(=S)-, preferably -C(=O)-.

m in the aforementioned formula (Ia) is an integer of 0 to 3, and R^{2a} in the number of m are each independently an optionally substituted alkyl group, an optionally substituted alkoxy group, or an electron-withdrawing group, preferably an optionally substituted C₁₋₆ alkyl group, an optionally substituted C₁₋₆ alkoxy group, or an electron-withdrawing group. As used herein, "m is 0" means that R^{2a} is not present.

When m in the aforementioned formula (Ia) is an integer of not less than 2, the adjacent two R^{2a} optionally form, together with ring A, an optionally substituted fused ring.

The 5- or 6-membered unsaturated heterocycle for ring A is a ring represented by any of the following formulas (r2) to (r4) [wherein the symbols are as defined above]. In the following formulas (r2) to (r4), R^{2a} is omitted.

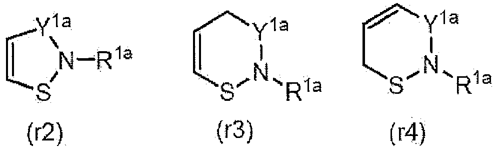

A fused ring formed by the adjacent two R^{2a} and ring A is preferably a bicyclic or tricyclic fused ring, more preferably a bicyclic fused ring. Examples of the fused ring include rings represented by any of the following formulas (r5) to (r25). The fused ring is optionally substituted.

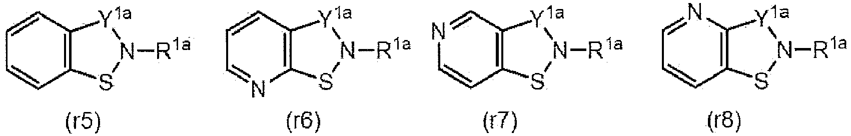

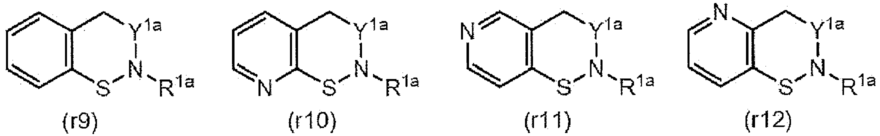

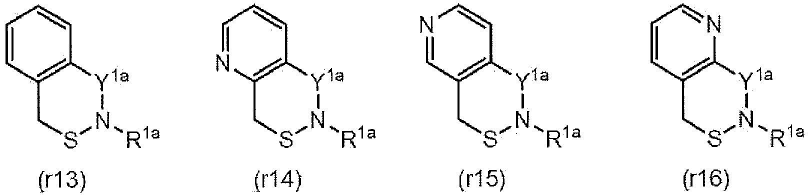

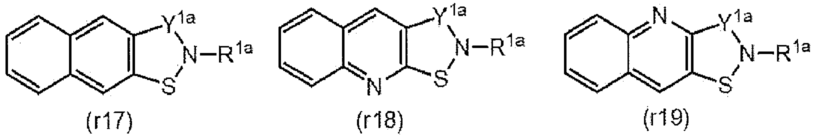

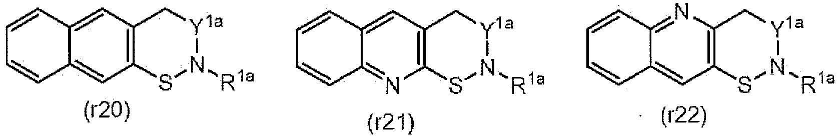

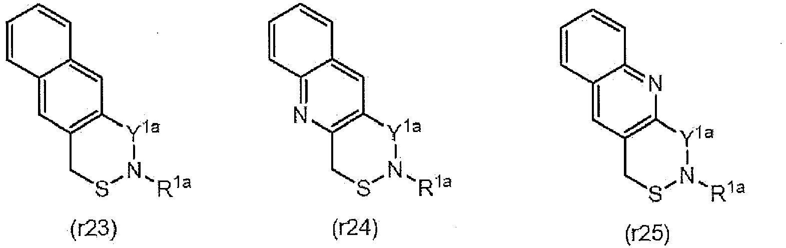

### 2-1-1A-2. Compound (Ic)

Compound (Ia) is preferably a compound represented by the following formula (Ic).

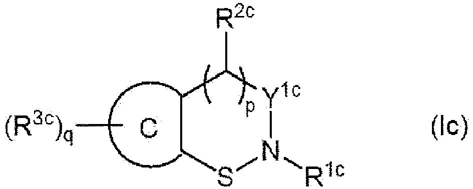

Each group in compound (Ic) is described below.

R^{1c} in the aforementioned formula (Ic) is an optionally substituted alkyl group, an optionally substituted aryl group, or an optionally substituted heteroaryl group, preferably an optionally substituted C₁₋₆ alkyl group, an optionally substituted phenyl group, or an optionally substituted 5- or 6-membered monocyclic heteroaryl group.

In one embodiment of the production method of the present invention, R^{1c} is preferably
(1) a C₁₋₆ alkyl group optionally substituted by 1 or 2 substituents selected from the group consisting of an optionally substituted phenyl group and an optionally substituted 5- or 6-membered monocyclic heteroaryl group,
(2) a phenyl group optionally substituted by 1 to 4 substituents selected from the group consisting of an optionally substituted alkyl group, an optionally substituted alkoxy group, and an electron-withdrawing group, or
(3) a 5- or 6-membered monocyclic heteroaryl group optionally substituted by 1 to 4 substituents selected from the group consisting of an optionally substituted alkyl group, an optionally substituted alkoxy group, and an electron-withdrawing group.

In another embodiment of the production method of the present invention, R^{1c} is preferably
(1) a C₁₋₆ alkyl group optionally substituted by 1 or 2 substituents selected from the group consisting of an optionally substituted phenyl group and an optionally substituted 5- or 6-membered monocyclic heteroaryl group,
(2) a phenyl group optionally substituted by 1 to 4 substituents selected from the group consisting of an optionally substituted C₁₋₆ alkyl group, an optionally substituted C₁₋₆ alkoxy group, and an electron-withdrawing group, or
(3) a pyridyl group optionally substituted by 1 to 4 substituents selected from the group consisting of an optionally substituted C₁₋₆ alkyl group, an optionally substituted C₁₋₆ alkoxy group, and an electron-withdrawing group.

In another embodiment of the production method of the present invention, R^{1c} is preferably
(1) a C₁₋₆ alkyl group optionally substituted by 1 or 2 pyridyl groups,
(2) a phenyl group optionally substituted by 1 to 4 substituents selected from the group consisting of an optionally substituted C₁₋₆ alkyl group, an optionally substituted C₁₋₆ alkoxy group, a halogen atom, and a nitro group, or
(3) a pyridyl group optionally substituted by 1 to 4 substituents selected from the group consisting of an optionally substituted C₁₋₆ alkyl group, an optionally substituted C₁₋₆ alkoxy group, a halogen atom, and a nitro group.

In another embodiment of the production method of the present invention, R^{1c} is preferably
(1) a C₁₋₆ alkyl group optionally substituted by 1 or 2 pyridyl groups,
(2) a phenyl group optionally substituted by 1 or 2 C₁₋₆ alkyl groups, or
(3) a pyridyl group (particularly a 2-pyridyl group or a 4-pyridyl group).

In another embodiment of the production method of the present invention, R^{1c} is preferably
(1) a C₁₋₆ alkyl group,
(2) a phenyl group optionally substituted by 1 or 2 substituents selected from the group consisting of a C₁₋₆ alkyl group, a C₁₋₆ alkoxy group, and a halogen atom, or
(3) a pyridyl group substituted by one C₁₋₆ alkyl group (e.g., 4-methyl-2-pyridyl group).

Y^{1c} in the aforementioned formula (Ic) is -S(=O)-, -S(=O)₂-, -C(=O)-, or -C(=S)-, preferably -C(=O)-.

p in the aforementioned formula (Ic) is 0 or 1, preferably 0. That "p is 0" means that -CH(R^{2c})- is not present in the formula (Ic) .

R^{2c} in the aforementioned formula (Ic) is a hydrogen atom, an optionally substituted alkyl group, an optionally substituted alkoxy group, or an electron-withdrawing group, preferably a hydrogen atom, an optionally substituted C₁₋₆ alkyl group, an optionally substituted C₁₋₆ alkoxy group, or an electron-withdrawing group.

Ring C in the aforementioned formula (Ic) is a 6-membered aromatic hydrocarbon ring, 6-membered nitrogen-containing aromatic heterocycle, or a 10-membered bicyclic fused aromatic heterocycle containing a nitrogen atom.

In one embodiment of the production method of the present invention, the aforementioned ring C is preferably a 6-membered aromatic hydrocarbon ring (i.e., benzene ring) or a 6-membered nitrogen-containing aromatic heterocycle, more preferably a benzene ring or a pyridine ring, further preferably a pyridine ring.

In another embodiment of the production method of the present invention, the aforementioned ring C is preferably a benzene ring, a pyridine ring, or a quinoline ring.

q in the aforementioned formula (Ic) is an integer of 0 to 4, preferably an integer of 0 to 2, more preferably 0 or 1. That "q is 0" means that R^{3c} is not present.

R^{3c} in the number of q in the aforementioned formula (Ic) are each independently an optionally substituted alkyl group, an optionally substituted alkoxy group, or an electron-withdrawing group.

In one embodiment of the production method of the present invention, R^{3c} in the number of q are each independently preferably an optionally substituted C₁₋₆ alkyl group, an optionally substituted C₁₋₆ alkoxy group, or an electron-withdrawing group, more preferably a halogen atom or a nitro group, further preferably a nitro group.

In another embodiment of the production method of the present invention, R^{3c} in the number of q are preferably each independently an electron-withdrawing group, more preferably a halogen atom, a C₁₋₆ perfluoroalkyl group, or a nitro group, further preferably a C₁₋₆ perfluoroalkyl group or a nitro group, particularly preferably a trifluoromethyl group.

As preferred compound (Ic), the following compound can be mentioned. Only one kind of the following compounds may be used, or two or more kinds thereof may be used in combination.

### (Compound (Ic-i))

A compound of the aforementioned formula (Ic), wherein
R^{1c} is
   (1) a C₁₋₆ alkyl group optionally substituted by 1 or 2 substituents selected from the group consisting of an optionally substituted phenyl group and an optionally substituted 5- or 6-membered monocyclic heteroaryl group,
   (2) a phenyl group optionally substituted by 1 to 4 substituents selected from the group consisting of an optionally substituted alkyl group, an optionally substituted alkoxy group, and an electron-withdrawing group, or
   (3) a 5- or 6-membered monocyclic heteroaryl group optionally substituted by 1 to 4 substituents selected from the group consisting of an optionally substituted alkyl group, an optionally substituted alkoxy group, and an electron-withdrawing group,
Y^{1c} is -S(=O)-, -S(=O)₂-, -C(=O)-, or -C(=S)-,
p is 0 or 1,
R^{2c} is a hydrogen atom, an optionally substituted alkyl group, an optionally substituted alkoxy group, or an electron-withdrawing group,
ring C is a 6-membered aromatic hydrocarbon ring or a 6-membered nitrogen-containing aromatic heterocycle,
q is an integer of 0 to 4, and
R^{3c} in the number of q are each independently an optionally substituted alkyl group, an optionally substituted alkoxy group, or an electron-withdrawing group (referred to as "compound (Ic-i)" in the present specification).

### (Compound (Ic-ii))

A compound of the aforementioned formula (Ic), wherein
R^{1c} is
   (1) a C₁₋₆ alkyl group optionally substituted by 1 or 2 substituents selected from the group consisting of an optionally substituted phenyl group and an optionally substituted 5- or 6-membered monocyclic heteroaryl group,
   (2) a phenyl group optionally substituted by 1 to 4 substituents selected from the group consisting of an optionally substituted C₁₋₆ alkyl group, an optionally substituted C₁₋₆ alkoxy group, and an electron-withdrawing group, or
   (3) a pyridyl group optionally substituted by 1 to 4 substituents selected from the group consisting of an optionally substituted C₁₋₆ alkyl group, an optionally substituted C₁₋₆ alkoxy group, and an electron-withdrawing group,
Y^{1c} is -S(=O)-, -S(=O)₂-, -C(=O)-, or -C(=S)-
p is 0 or 1,
R^{2c} is a hydrogen atom, an optionally substituted C₁₋₆ alkyl group, an optionally substituted C₁₋₆ alkoxy group, or an electron-withdrawing group,
ring C is a 6-membered aromatic hydrocarbon ring or a 6-membered nitrogen-containing aromatic heterocycle,
q is an integer of 0 to 2, and
R^{3c} in the number of q are each independently an optionally substituted C₁₋₆ alkyl group, an optionally substituted C₁₋₆ alkoxy group, or an electron-withdrawing group (referred to as "compound (Ic-ii)" in the present specification).

### (Compound (Ic-iii))

A compound of the aforementioned formula (Ic), wherein
R^{1c} is
   (1) a C₁₋₆ alkyl group optionally substituted by 1 or 2 pyridyl groups,
   (2) a phenyl group optionally substituted by 1 to 4 substituents selected from the group consisting of an optionally substituted C₁₋₆ alkyl group, an optionally substituted C₁₋₆ alkoxy group, a halogen atom, and a nitro group, or
   (3) a pyridyl group optionally substituted by 1 to 4 substituents selected from the group consisting of an optionally substituted C₁₋₆ alkyl group, an optionally substituted C₁₋₆ alkoxy group, a halogen atom, and a nitro group,
Y^{1c} is -C (=O) -
p is 0,
ring C is a benzene ring or a pyridine ring,
q is an integer of 0 to 2,
R^{3c} in the number of q are each independently a halogen atom or a nitro group (referred to as "compound (Ic-iii)" in the present specification).

### (Compound (Ic-iv))

A compound of the aforementioned formula (Ic), wherein
R^{1c} is
   (1) a C₁₋₆ alkyl group optionally substituted by 1 or 2 pyridyl groups,
   (2) a phenyl group optionally substituted by 1 or 2 C₁₋₆ alkyl groups, or
   (3) a pyridyl group (particularly a 2-pyridyl group or a 4-pyridyl group),
Y^{1c} is -C(=O)-
p is 0,
ring C is a benzene ring or a pyridine ring,
q is an integer of 0 to 2, and
R^{3c} in the number of q are nitro groups (referred to as "compound (Ic-iv)" in the present specification).

### (Compound (Ic-i'))

A compound of the aforementioned formula (Ic), wherein
R^{1c} is
   (1) a C₁₋₆ alkyl group optionally substituted by 1 or 2 substituents selected from the group consisting of an optionally substituted phenyl group and an optionally substituted 5- or 6-membered monocyclic heteroaryl group,
   (2) a phenyl group optionally substituted by 1 to 4 substituents selected from the group consisting of an optionally substituted alkyl group, an optionally substituted alkoxy group, and an electron-withdrawing group, or
   (3) a 5- or 6-membered monocyclic heteroaryl group optionally substituted by 1 to 4 substituents selected from the group consisting of an optionally substituted alkyl group, an optionally substituted alkoxy group, and an electron-withdrawing group,
Y^{1c} is -S(=O)-, -S(=O)₂-, -C(=O)-, or -C(=S)-,
p is 0 or 1,
R^{2c} is a hydrogen atom, an optionally substituted alkyl group, an optionally substituted alkoxy group, or an electron-withdrawing group,
ring C is a 6-membered aromatic hydrocarbon ring, a 6-membered nitrogen-containing aromatic heterocycle, or a 10-membered bicyclic fused aromatic heterocycle containing a nitrogen atom,
q is an integer of 0 to 4, and
R^{3c} in the number of q are each independently an optionally substituted alkyl group, an optionally substituted alkoxy group, or an electron-withdrawing group (referred to as "compound (Ic-i')" in the present specification).

### (Compound (Ic-ii'))

A compound of the aforementioned formula (Ic), wherein
R^{1c} is
   (1) a C₁₋₆ alkyl group optionally substituted by 1 or 2 substituents selected from the group consisting of an optionally substituted phenyl group and an optionally substituted 5- or 6-membered monocyclic heteroaryl group,
   (2) a phenyl group optionally substituted by 1 to 4 substituents selected from the group consisting of an optionally substituted C₁₋₆ alkyl group, an optionally substituted C₁₋₆ alkoxy group, and an electron-withdrawing group, or
   (3) a pyridyl group optionally substituted by 1 to 4 substituents selected from the group consisting of an optionally substituted C₁₋₆ alkyl group, an optionally substituted C₁₋₆ alkoxy group, and an electron-withdrawing group,
Y^{1c} is -S(=O)-, -S(=O)₂-, -C(=O)-, or -C(=S)-
p is 0 or 1,
R^{2c} is a hydrogen atom, an optionally substituted C₁₋₆ alkyl group, an optionally substituted C₁₋₆ alkoxy group, or an electron-withdrawing group,
ring C is a 6-membered aromatic hydrocarbon ring, a 6-membered nitrogen-containing aromatic heterocycle, or a 10-membered bicyclic fused aromatic heterocycle containing a nitrogen atom,
q is an integer of 0 to 2, and
R^{3c} in the number of q are each independently an optionally substituted C₁₋₆ alkyl group, an optionally substituted C₁₋₆ alkoxy group, or an electron-withdrawing group (referred to as "compound (Ic-ii')" in the present specification).

### (Compound (Ic-iii'))

A compound of the aforementioned formula (Ic), wherein
R^{1c} is
   (1) a C₁₋₆ alkyl group,
   (2) a phenyl group optionally substituted by 1 to 4 substituents selected from the group consisting of an optionally substituted C₁₋₆ alkyl group, an optionally substituted C₁₋₆ alkoxy group, a halogen atom, and a nitro group, or
   (3) a pyridyl group optionally substituted by 1 to 4 substituents selected from the group consisting of an optionally substituted C₁₋₆ alkyl group, an optionally substituted C₁₋₆ alkoxy group, a halogen atom, and a nitro group,
Y^{1c} is -C(=O)-,
p is 0 or 1,
ring C is a benzene ring, a pyridine ring, or a quinoline ring,
q is an integer of 0 to 2,
R^{3c} in the number of q are each independently a halogen atom, a C₁₋₆ perfluoroalkyl group, or a nitro group (referred to as "compound (Ic-iii')" in the present specification).

### (Compound (Ic-iv'))

A compound of the aforementioned formula (Ic), wherein
R^{1c} is
   (1) a C₁₋₆ alkyl group,
   (2) a phenyl group optionally substituted by 1 or 2 substituent selected from the group consisting of a C₁₋₆ alkyl group, a C₁₋₆ alkoxy group, and a halogen atom, or
   (3) a pyridyl group substituted by 1 or 2 C₁₋₆ alkyl groups (e.g., 4-methyl-2-pyridyl group),
Y^{1c} is -C(=O)-,
p is 0 or 1,
ring C is a benzene ring, a pyridine ring, or a quinoline ring,
q is an integer of 0 to 2,
R^{3c} in the number of q are C₁₋₆ perfluoroalkyl groups (referred to as "compound (Ic-iv')" in the present specification).

### (Specific preferable examples of compound (Ic))

Specific preferable examples of compound (Ic) include compounds represented by any of the following formulas (Ic-1) to (Ic-17).

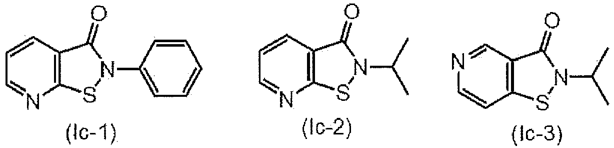

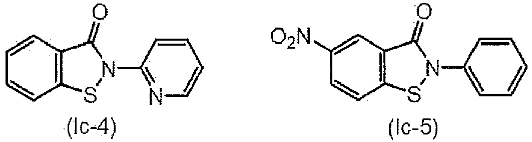

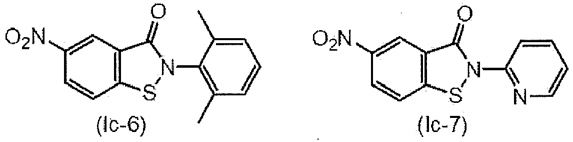

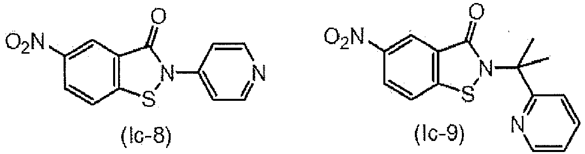

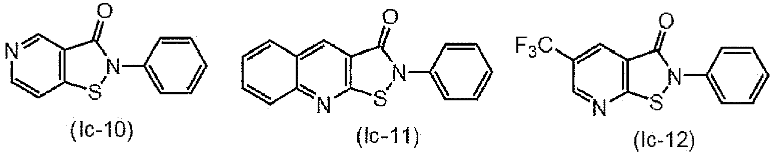

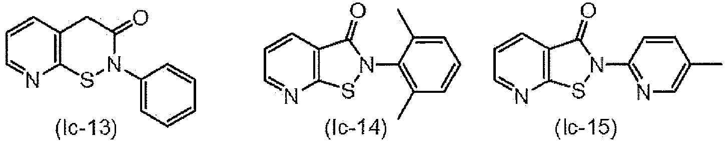

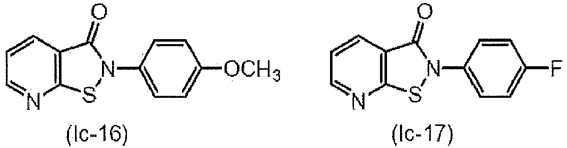

In one embodiment of the production method of the present invention, compound (Ic) is preferably at least one selected from the group consisting of 2-phenylisothiazolo[5,4-b]pyridin-3(2H)-one (compound (Ic-1)), 2-isopropylisothiazolo[5,4-b]pyridin-3(2H)-one (compound (Ic-2)), 2-isopropylisothiazolo[4,5-c]pyridin-3(2H)-one (compound (Ic-3)), 2-(2-pyridyl)-1,2-benzothiazol-3(2H)-one (compound (Ic-4)), 5-nitro-2-phenyl-1,2-benzothiazol-3(2H)-one (compound (Ic-5)), 2-(2,6-dimethylphenyl)-5-nitro-1,2-benzothiazol-3(2H)-one (compound (Ic-6)), 5-nitro-2-(2-pyridyl)-1,2-benzothiazol-3(2H)-one (compound (Ic-7)), 5-nitro-2-(4-pyridyl)-1,2-benzothiazol-3(2H)-one (compound (Ic-8)), 2-[1-methyl-1-(2-pyridyl)ethyl]-5-nitro-1,2-benzothiazol-3(2H)-one (compound (Ic-9)), 2-phenylisothiazolo[4,5-c]pyridin-3(2H)-one (compound (Ic-10)), 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In another embodiment of the production method of the present invention, compound (Ic) is preferably at least one selected from the group consisting of 2-phenylisothiazolo[5,4-b]pyridin-3(2H)-one (compound (Ic-1)), 2-isopropylisothiazolo[5,4-b]pyridin-3(2H)-one (compound (Ic-2)), 2-isopropylisothiazolo[4,5-c]pyridin-3(2H)-one (compound (Ic-3)), 2-(2-pyridyl)-1,2-benzothiazol-3(2H)-one (compound (Ic-4)), 5-nitro-2-phenyl-1,2-benzothiazol-3(2H)-one (compound (Ic-5)), 2-(2,6-dimethylphenyl)-5-nitro-1,2-benzothiazol-3(2H)-one (compound (Ic-6)), 5-nitro-2-(2-pyridyl)-1,2-benzothiazol-3(2H)-one (compound (Ic-7)), 5-nitro-2-(4-pyridyl)-1,2-benzothiazol-3(2H)-one (compound (Ic-8)), and 2-[1-methyl-1-(2-pyridyl)ethyl]-5-nitro-1,2-benzothiazol-3(2H)-one (compound (Ic-9)).

In another embodiment of the production method of the present invention, compound (Ic) is preferably at least one selected from the group consisting of 2-phenylisothiazolo[5,4-b]pyridin-3(2H)-one (compound (Ic-1)), 2-isopropylisothiazolo[5,4-b]pyridin-3(2H)-one (compound (Ic-2)), 5-nitro-2-phenyl-1,2-benzothiazol-3(2H)-one (compound (Ic-5)), 2-phenylisothiazolo[4,5-c]pyridin-3(2H)-one (compound (Ic-10)), 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In another embodiment of the production method of the present invention, compound (Ic) is preferably at least one selected from the group consisting of 2-phenylisothiazolo[5,4-b]pyridin-3(2H)-one (compound (Ic-1)), 2-isopropylisothiazolo[5,4-b]pyridin-3(2H)-one (compound (Ic-2)), 2-phenylisothiazolo[4,5-c]pyridin-3(2H)-one (compound (Ic-10)), 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In the production method of the present invention, compound (Ic) is most preferably 2-phenylisothiazolo[5,4-b]pyridin-3(2H)-one (compound (Ic-1)).

### 2-1-1B. Compound (IB)

Compound (IB) is now described. In compound (IB), R¹ in the aforementioned formula (I) is an optionally substituted alkyl group, an optionally substituted aryl group, or an optionally substituted heteroaryl group, preferably an optionally substituted C₁₋₁₀ alkyl group, an optionally substituted phenyl group, an optionally substituted 5- or 6-membered monocyclic heteroaryl group, or an optionally substituted 8- to 14-membered fused polycyclic heteroaryl group.

In compound (IB), R² in the aforementioned formula (I) is an optionally substituted aryl group or an optionally substituted heteroaryl group, preferably an optionally substituted phenyl group, an optionally substituted 5- or 6-membered monocyclic heteroaryl group, or an optionally substituted 8- to 14-membered fused polycyclic heteroaryl group.

### 2-1-1B-1. Compound (Ib)

Compound (IB) is preferably a compound represented by the following formula (Ib) excluding 2,2-dipyridyl disulfide. Only one kind of compound (Ib) may be used, or two or more kinds thereof may be used in combination.

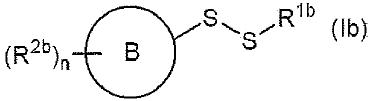

Each group in compound (Ib) is described below.

R^{1b} in the aforementioned formula (Ib) is an optionally substituted alkyl group, an optionally substituted aryl group, or an optionally substituted heteroaryl group, preferably an optionally substituted C₁₋₁₀ alkyl group, an optionally substituted phenyl group, an optionally substituted 5- or 6-membered monocyclic heteroaryl group, or an optionally substituted 8- to 14-membered fused polycyclic heteroaryl group.

In one embodiment of the production method of the present invention (hereinafter referred to as "embodiment (A)"), R^{1b} is more preferably
(1) an alkyl group substituted by one carboxy group,
(2) a 2-benzothiazolyl group optionally substituted by 1 to 4 substituents selected from the group consisting of an optionally substituted alkyl group, an optionally substituted alkoxy group, and an electron-withdrawing group,
(3) a 2-benzoimidazolyl group optionally substituted by 1 to 5 substituents selected from the group consisting of an optionally substituted alkyl group, an optionally substituted alkoxy group, and an electron-withdrawing group,
(4) a 2-benzoxazolyl group optionally substituted by 1 to 4 substituents selected from the group consisting of an optionally substituted alkyl group, an optionally substituted alkoxy group, and an electron-withdrawing group,
(5) a 5-(1,2,3-triazolyl) group optionally substituted by 1 or 2 substituents selected from the group consisting of an optionally substituted alkyl group, an optionally substituted alkoxy group, and an electron-withdrawing group,
(6) a 1H-tetrazol-5-yl group optionally substituted by one substituent selected from the group consisting of an optionally substituted alkyl group and an optionally substituted aryl group,
(7) a (pyridine-N-oxide)-2-yl group optionally substituted by 1 to 4 substituents selected from the group consisting of an optionally substituted alkyl group, an optionally substituted alkoxy group, and an electron-withdrawing group,
(8) a 4-pyridyl group optionally substituted by 1 to 4 substituents selected from the group consisting of an optionally substituted alkyl group, an optionally substituted alkoxy group, and an electron-withdrawing group,
(9) a 2-pyridyl group optionally substituted by 1 to 4 substituents selected from the group consisting of an optionally substituted alkyl group, an optionally substituted alkoxy group, and an electron-withdrawing group,
(10) a phenyl group optionally substituted by 1 to 5 substituents selected from the group consisting of an optionally substituted alkyl group, an optionally substituted alkoxy group, and an electron-withdrawing group, or
(11) a 2-imidazolyl group optionally substituted by 1 to 3 substituents selected from the group consisting of an optionally substituted alkyl group, an optionally substituted alkoxy group, and an electron-withdrawing group.

In embodiment (A), R^{1b} is further preferably
(1) an alkyl group substituted by one carboxy group,
(2) a 2-benzothiazolyl group optionally substituted by 1 to 4 substituents selected from the group consisting of an optionally substituted alkyl group, an optionally substituted alkoxy group, and an electron-withdrawing group,
(3) a 2-benzoimidazolyl group optionally substituted by 1 to 5 substituents selected from the group consisting of an optionally substituted alkyl group, an optionally substituted alkoxy group, and an electron-withdrawing group,
(4) a 2-benzoxazolyl group optionally substituted by 1 to 4 substituents selected from the group consisting of an optionally substituted alkyl group, an optionally substituted alkoxy group, and an electron-withdrawing group,
(5) a 5-(1,2,3-triazolyl) group optionally substituted by 1 or 2 substituents selected from the group consisting of an optionally substituted alkyl group, an optionally substituted alkoxy group, and an electron-withdrawing group,
(6) a 1H-tetrazol-5-yl group optionally substituted by one substituent selected from the group consisting of an optionally substituted alkyl group and an optionally substituted aryl group,
(7) a (pyridine-N-oxide)-2-yl group optionally substituted by 1 to 4 substituents selected from the group consisting of an optionally substituted alkyl group, an optionally substituted alkoxy group, and an electron-withdrawing group,
(8) a 4-pyridyl group optionally substituted by 1 to 4 substituents selected from the group consisting of an optionally substituted alkyl group, an optionally substituted alkoxy group, and an electron-withdrawing group, or
(9) a 2-pyridyl group optionally substituted by 1 to 4 substituents selected from the group consisting of an optionally substituted alkyl group, an optionally substituted alkoxy group, and an electron-withdrawing group.

In embodiment (A), R^{1b} is particularly preferably
(1) a C₁₋₆ alkyl group substituted by one carboxy group,
(2) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
(3) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
(4) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
(5) a 5-(1,2,3-triazolyl) group optionally substituted by 1 or 2 substituents selected from the group consisting of an optionally substituted C₁₋₆ alkyl group, an optionally substituted C₁₋₆ alkoxy group, and an electron-withdrawing group,
(6) a 1H-tetrazol-5-yl group optionally substituted by one substituent selected from the group consisting of an optionally substituted C₁₋₆ alkyl group and an optionally substituted phenyl group,
(7) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
(8) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
(9) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group.

In embodiment (A), R^{1b} is most preferably a C₁₋₆ alkyl group substituted one carboxy group, a 2-benzothiazolyl group, a 2-benzoimidazolyl group, a 2-benzoxazolyl group, a 5-(1,2,3-triazolyl) group, a 1-phenyl-1H-tetrazol-5-yl group, a (pyridine-N-oxide)-2-yl group, a 4-pyridyl group, or a 2-pyridyl group.

In another embodiment of the production method of the present invention (hereinafter referred to as "embodiment (B)"), R^{1b} is more preferably
(1) an alkyl group substituted by one carboxy group,
(2) a 2-benzothiazolyl group optionally substituted by 1 to 4 substituents selected from the group consisting of an optionally substituted alkyl group, an optionally substituted alkoxy group, and an electron-withdrawing group,
(3) a 2-benzoimidazolyl group optionally substituted by 1 to 5 substituents selected from the group consisting of an optionally substituted alkyl group, an optionally substituted alkoxy group, and an electron-withdrawing group,
(4) a 2-benzoxazolyl group optionally substituted by 1 to 4 substituents selected from the group consisting of an optionally substituted alkyl group, an optionally substituted alkoxy group, and an electron-withdrawing group,
(5) a 1H-tetrazol-5-yl group optionally substituted by one substituent selected from the group consisting of an optionally substituted alkyl group and an optionally substituted aryl group,
(6) a (pyridine-N-oxide)-2-yl group optionally substituted by 1 to 4 substituents selected from the group consisting of an optionally substituted alkyl group, an optionally substituted alkoxy group, and an electron-withdrawing group,
(7) a 4-pyridyl group optionally substituted by 1 to 4 substituents selected from the group consisting of an optionally substituted alkyl group, an optionally substituted alkoxy group, and an electron-withdrawing group, or
(8) a 2-pyridyl group optionally substituted by 1 to 4 substituents selected from the group consisting of an optionally substituted alkyl group, an optionally substituted alkoxy group, and an electron-withdrawing group.

In embodiment (B), R^{1b} is further preferably
(1) a C₁₋₆ alkyl group substituted by one carboxy group,
(2) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
(3) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
(4) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
(5) a 1H-tetrazol-5-yl group optionally substituted by one substituent selected from the group consisting of an optionally substituted C₁₋₆ alkyl group and an optionally substituted phenyl group,
(6) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
(7) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
(8) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group.

In embodiment (B), R^{1b} is particularly preferably, a C₁₋₆ alkyl group substituted by one carboxy group, a 2-benzothiazolyl group, a 2-benzoimidazolyl group, a 2-benzoxazolyl group, a 1-phenyl-1H-tetrazol-5-yl group, a (pyridine-N-oxide)-2-yl group, a 4-pyridyl group, or a 2-pyridyl group.

Ring B in the aforementioned formula (Ib) is a 6-membered aromatic hydrocarbon ring (i.e., benzene ring) or a 5- or 6-membered aromatic heterocycle, preferably a 5- or 6-membered aromatic heterocycle, more preferably a triazole ring, a tetrazole ring, a pyridine ring, or a pyridine-N-oxide ring, further preferably a tetrazole ring, a pyridine ring, or a pyridine-N-oxide ring.

In the aforementioned formula (Ib), n is an integer of 0 to 5, R^{2b} in the number of n are each independently an optionally substituted alkyl group, an optionally substituted alkoxy group, or an electron-withdrawing group, more preferably an optionally substituted C₁₋₆ alkyl group, an optionally substituted C₁₋₆ alkoxy group, or an electron-withdrawing group. As used herein, that "n is 0" means that R^{2b} is not present.

In the aforementioned formula (Ib), when n is an integer of not less than 2, the adjacent two R^{2b} may form, together with ring B, an optionally substituted bicyclic fused aromatic heterocycle (preferably an optionally substituted benzoimidazole ring, an optionally substituted benzoxazole ring, or an optionally substituted benzothiazole ring).

In one embodiment of the production method of the present invention (hereinafter referred to as "embodiment (C)"), a group represented by the formula (r1) : 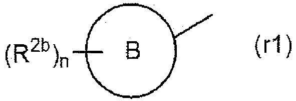 in compound (Ib) is preferably
(1) a 2-benzothiazolyl group optionally substituted by 1 to 4 substituents selected from the group consisting of an optionally substituted alkyl group, an optionally substituted alkoxy group, and an electron-withdrawing group,
(2) a 2-benzoimidazolyl group optionally substituted by 1 to 5 substituents selected from the group consisting of an optionally substituted alkyl group, an optionally substituted alkoxy group, and an electron-withdrawing group,
(3) a 2-benzoxazolyl group optionally substituted by 1 to 4 substituents selected from the group consisting of an optionally substituted alkyl group, an optionally substituted alkoxy group, and an electron-withdrawing group,
(4) a 5-(1,2,3-triazolyl) group optionally substituted by 1 or 2 substituents selected from the group consisting of an optionally substituted alkyl group, an optionally substituted alkoxy group, and an electron-withdrawing group,
(5) a 1H-tetrazol-5-yl group optionally substituted by one substituent selected from the group consisting of an optionally substituted alkyl group and an optionally substituted aryl group,
(6) a (pyridine-N-oxide)-2-yl group optionally substituted by 1 to 4 substituents selected from the group consisting of an optionally substituted alkyl group, an optionally substituted alkoxy group, and an electron-withdrawing group,
(7) a 4-pyridyl group optionally substituted by 1 to 4 substituents selected from the group consisting of an optionally substituted alkyl group, an optionally substituted alkoxy group, and an electron-withdrawing group,
(8) a 2-pyridyl group optionally substituted by 1 to 4 substituents selected from the group consisting of an optionally substituted alkyl group, an optionally substituted alkoxy group, and an electron-withdrawing group,
(9) a phenyl group optionally substituted by 1 to 5 substituents selected from the group consisting of an optionally substituted alkyl group, an optionally substituted alkoxy group, and an electron-withdrawing group, or
(10) a 2-imidazolyl group optionally substituted by 1 to 3 substituents selected from the group consisting of an optionally substituted alkyl group, an optionally substituted alkoxy group, and an electron-withdrawing group.

In embodiment (C), group (r1) is more preferably
(1) a 2-benzothiazolyl group optionally substituted by 1 to 4 substituents selected from the group consisting of an optionally substituted alkyl group, an optionally substituted alkoxy group, and an electron-withdrawing group,
(2) a 2-benzoimidazolyl group optionally substituted by 1 to 5 substituents selected from the group consisting of an optionally substituted alkyl group, an optionally substituted alkoxy group, and an electron-withdrawing group,
(3) a 2-benzoxazolyl group optionally substituted by 1 to 4 substituents selected from the group consisting of an optionally substituted alkyl group, an optionally substituted alkoxy group, and an electron-withdrawing group,
(4) a 5-(1,2,3-triazolyl) group optionally substituted by 1 or 2 substituents selected from the group consisting of an optionally substituted alkyl group, an optionally substituted alkoxy group, and an electron-withdrawing group,
(5) a 1H-tetrazol-5-yl group optionally substituted by one substituent selected from the group consisting of an optionally substituted alkyl group and an optionally substituted aryl group,
(6) a (pyridine-N-oxide)-2-yl group optionally substituted by 1 to 4 substituents selected from the group consisting of an optionally substituted alkyl group, an optionally substituted alkoxy group, and an electron-withdrawing group,
(7) a 4-pyridyl group optionally substituted by 1 to 4 substituents selected from the group consisting of an optionally substituted alkyl group, an optionally substituted alkoxy group, and an electron-withdrawing group, or
(8) a 2-pyridyl group optionally substituted by 1 to 4 substituents selected from the group consisting of an optionally substituted alkyl group, an optionally substituted alkoxy group, and an electron-withdrawing group.

In embodiment (C), group (r1) is further preferably
(1) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
(2) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
(3) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
(4) a 5-(1,2,3-triazolyl) group optionally substituted by 1 or 2 substituents selected from the group consisting of an optionally substituted C₁₋₆ alkyl group, an optionally substituted C₁₋₆ alkoxy group, and an electron-withdrawing group,
(5) a 1H-tetrazol-5-yl group optionally substituted by one substituent selected from the group consisting of an optionally substituted C₁₋₆ alkyl group and an optionally substituted phenyl group,
(6) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
(7) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
(8) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group.

In embodiment (C), group (r1) is particularly preferably a 2-benzothiazolyl group, a 2-benzoimidazolyl group, a 2-benzoxazolyl group, a 5-(1,2,3-triazolyl) group, a 1-phenyl-1H-tetrazol-5-yl group, a (pyridine-N-oxide)-2-yl group, a 4-pyridyl group, or a 2-pyridyl group, most preferably a 2-benzothiazolyl group, a 2-benzoimidazolyl group, a 2-benzoxazolyl group, a 5-(1,2,3-triazolyl) group, a 1-phenyl-1H-tetrazol-5-yl group, or a (pyridine-N-oxide)-2-yl group.

In another embodiment of the production method of the present invention (hereinafter referred to as "embodiment (D)"), group (r1) is preferably
(1) a 2-benzothiazolyl group optionally substituted by 1 to 4 substituents selected from the group consisting of an optionally substituted alkyl group, an optionally substituted alkoxy group, and an electron-withdrawing group,
(2) a 2-benzoimidazolyl group optionally substituted by 1 to 5 substituents selected from the group consisting of an optionally substituted alkyl group, an optionally substituted alkoxy group, and an electron-withdrawing group,
(3) a 2-benzoxazolyl group optionally substituted by 1 to 4 substituents selected from the group consisting of an optionally substituted alkyl group, an optionally substituted alkoxy group, and an electron-withdrawing group,
(4) a 1H-tetrazol-5-yl group optionally substituted by one substituent selected from the group consisting of an optionally substituted alkyl group and an optionally substituted aryl group,
(5) a (pyridine-N-oxide)-2-yl group optionally substituted by 1 to 4 substituents selected from the group consisting of an optionally substituted alkyl group, an optionally substituted alkoxy group, and an electron-withdrawing group,
(6) a 4-pyridyl group optionally substituted by 1 to 4 substituents selected from the group consisting of an optionally substituted alkyl group, an optionally substituted alkoxy group, and an electron-withdrawing group, or
(7) a 2-pyridyl group optionally substituted by 1 to 4 substituents selected from the group consisting of an optionally substituted alkyl group, an optionally substituted alkoxy group, and an electron-withdrawing group.

In embodiment (D), group (r1) is further preferably
(1) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
(2) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
(3) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
(4) a 1H-tetrazol-5-yl group optionally substituted by one substituent selected from the group consisting of an optionally substituted C₁₋₆ alkyl group and an optionally substituted phenyl group,
(5) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
(6) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
(7) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group.

In embodiment (D), group (r1) is particularly preferably a 2-benzothiazolyl group, a 2-benzoimidazolyl group, a 2-benzoxazolyl group, 1-phenyl-1H-tetrazol-5-yl group, a (pyridine-N-oxide)-2-yl group, a 4-pyridyl group, or a 2-pyridyl group, most preferably a 2-benzothiazolyl group, a 2-benzoimidazolyl group, a 2-benzoxazolyl group, a 1-phenyl-1H-tetrazol-5-yl group, or a (pyridine-N-oxide)-2-yl group.

Preferred compound (Ib) includes the following compounds. Only one kind of the following compounds may be used, or two or more kinds thereof may be used in combination.

### (Compound (Ib-i))

A compound of the aforementioned formula (Ib), wherein
R^{1b} is
   (1) an alkyl group substituted by one carboxy group,
   (2) a 2-benzothiazolyl group optionally substituted by 1 to 4 substituents selected from the group consisting of an optionally substituted alkyl group, an optionally substituted alkoxy group, and an electron-withdrawing group,
   (3) a 2-benzoimidazolyl group optionally substituted by 1 to 5 substituents selected from the group consisting of an optionally substituted alkyl group, an optionally substituted alkoxy group, and an electron-withdrawing group,
   (4) a 2-benzoxazolyl group optionally substituted by 1 to 4 substituents selected from the group consisting of an optionally substituted alkyl group, an optionally substituted alkoxy group, and an electron-withdrawing group,
   (5) a 5-(1,2,3-triazolyl) group optionally substituted by 1 or 2 substituents selected from the group consisting of an optionally substituted alkyl group, an optionally substituted alkoxy group, and an electron-withdrawing group,
   (6) a 1H-tetrazol-5-yl group optionally substituted by one substituent selected from the group consisting of an optionally substituted alkyl group and an optionally substituted aryl group,
   (7) a (pyridine-N-oxide)-2-yl group optionally substituted by 1 to 4 substituents selected from the group consisting of an optionally substituted alkyl group, an optionally substituted alkoxy group, and an electron-withdrawing group,
   (8) a 4-pyridyl group optionally substituted by 1 to 4 substituents selected from the group consisting of an optionally substituted alkyl group, an optionally substituted alkoxy group, and an electron-withdrawing group,
   (9) a 2-pyridyl group optionally substituted by 1 to 4 substituents selected from the group consisting of an optionally substituted alkyl group, an optionally substituted alkoxy group, and an electron-withdrawing group,
   (10) a phenyl group optionally substituted by 1 to 5 substituents selected from the group consisting of an optionally substituted alkyl group, an optionally substituted alkoxy group, and an electron-withdrawing group, or
   (11) a 2-imidazolyl group optionally substituted by 1 to 3 substituents selected from the group consisting of an optionally substituted alkyl group, an optionally substituted alkoxy group, and an electron-withdrawing group, and
group (r1) is
   (1) a 2-benzothiazolyl group optionally substituted by 1 to 4 substituents selected from the group consisting of an optionally substituted alkyl group, an optionally substituted alkoxy group, and an electron-withdrawing group,
   (2) a 2-benzoimidazolyl group optionally substituted by 1 to 5 substituents selected from the group consisting of an optionally substituted alkyl group, an optionally substituted alkoxy group, and an electron-withdrawing group,
   (3) a 2-benzoxazolyl group optionally substituted by 1 to 4 substituents selected from the group consisting of an optionally substituted alkyl group, an optionally substituted alkoxy group, and an electron-withdrawing group,
   (4) a 5-(1,2,3-triazolyl) group optionally substituted by 1 or 2 substituents selected from the group consisting of an optionally substituted alkyl group, an optionally substituted alkoxy group, and an electron-withdrawing group,
   (5) a 1H-tetrazol-5-yl group optionally substituted by one substituent selected from the group consisting of an optionally substituted alkyl group and an optionally substituted aryl group,
   (6) a (pyridine-N-oxide)-2-yl group optionally substituted by 1 to 4 substituents selected from the group consisting of an optionally substituted alkyl group, an optionally substituted alkoxy group, and an electron-withdrawing group,
   (7) a 4-pyridyl group optionally substituted by 1 to 4 substituents selected from the group consisting of an optionally substituted alkyl group, an optionally substituted alkoxy group, and an electron-withdrawing group, or
   (8) a 2-pyridyl group optionally substituted by 1 to 4 substituents selected from the group consisting of an optionally substituted alkyl group, an optionally substituted alkoxy group, and an electron-withdrawing group.
   (9) a phenyl group optionally substituted by 1 to 5 substituents selected from the group consisting of an optionally substituted alkyl group, an optionally substituted alkoxy group, and an electron-withdrawing group, or
   (10) a 2-imidazolyl group optionally substituted by 1 to 3 substituents selected from the group consisting of an optionally substituted alkyl group, an optionally substituted alkoxy group, and an electron-withdrawing group (referred to as "compound (Ib-i)" in the present specification).

### (Compound (Ib-ii))

A compound of the aforementioned formula (Ib), wherein
R^{1b} is
   (1) an alkyl group substituted by one carboxy group,
   (2) a 2-benzothiazolyl group optionally substituted by 1 to 4 substituents selected from the group consisting of an optionally substituted alkyl group, an optionally substituted alkoxy group, and an electron-withdrawing group,
   (3) a 2-benzoimidazolyl group optionally substituted by 1 to 5 substituents selected from the group consisting of an optionally substituted alkyl group, an optionally substituted alkoxy group, and an electron-withdrawing group,
   (4) a 2-benzoxazolyl group optionally substituted by 1 to 4 substituents selected from the group consisting of an optionally substituted alkyl group, an optionally substituted alkoxy group, and an electron-withdrawing group,
   (5) a 5-(1,2,3-triazolyl) group optionally substituted by 1 or 2 substituents selected from the group consisting of an optionally substituted alkyl group, an optionally substituted alkoxy group, and an electron-withdrawing group,
   (6) a 1H-tetrazol-5-yl group optionally substituted by one substituent selected from the group consisting of an optionally substituted alkyl group and an optionally substituted aryl group,
   (7) a (pyridine-N-oxide)-2-yl group optionally substituted by 1 to 4 substituents selected from the group consisting of an optionally substituted alkyl group, an optionally substituted alkoxy group, and an electron-withdrawing group,
   (8) a 4-pyridyl group optionally substituted by 1 to 4 substituents selected from the group consisting of an optionally substituted alkyl group, an optionally substituted alkoxy group, and an electron-withdrawing group, or
   (9) a 2-pyridyl group optionally substituted by 1 to 4 substituents selected from the group consisting of an optionally substituted alkyl group, an optionally substituted alkoxy group, and an electron-withdrawing group, and
group (r1) is
   (1) a 2-benzothiazolyl group optionally substituted by 1 to 4 substituents selected from the group consisting of an optionally substituted alkyl group, an optionally substituted alkoxy group, and an electron-withdrawing group,
   (2) a 2-benzoimidazolyl group optionally substituted by 1 to 5 substituents selected from the group consisting of an optionally substituted alkyl group, an optionally substituted alkoxy group, and an electron-withdrawing group,
   (3) a 2-benzoxazolyl group optionally substituted by 1 to 4 substituents selected from the group consisting of an optionally substituted alkyl group, an optionally substituted alkoxy group, and an electron-withdrawing group,
   (4) a 5-(1,2,3-triazolyl) group optionally substituted by 1 or 2 substituents selected from the group consisting of an optionally substituted alkyl group, an optionally substituted alkoxy group, and an electron-withdrawing group,
   (5) a 1H-tetrazol-5-yl group optionally substituted by one substituent selected from the group consisting of an optionally substituted alkyl group and an optionally substituted aryl group,
   (6) a (pyridine-N-oxide)-2-yl group optionally substituted by 1 to 4 substituents selected from the group consisting of an optionally substituted alkyl group, an optionally substituted alkoxy group, and an electron-withdrawing group,
   (7) a 4-pyridyl group optionally substituted by 1 to 4 substituents selected from the group consisting of an optionally substituted alkyl group, an optionally substituted alkoxy group, and an electron-withdrawing group, or
   (8) a 2-pyridyl group optionally substituted by 1 to 4 substituents selected from the group consisting of an optionally substituted alkyl group, an optionally substituted alkoxy group, and an electron-withdrawing group (referred to as "compound (Ib-ii)" in the present specification).

### (Compound (Ib-iii))

A compound of the aforementioned formula (Ib), wherein
R^{1b} is
   (1) a C₁₋₆ alkyl group substituted by one carboxy group,
   (2) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
   (3) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
   (4) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
   (5) a 5-(1,2,3-triazolyl) group optionally substituted by 1 or 2 substituents selected from the group consisting of an optionally substituted C₁₋₆ alkyl group, an optionally substituted C₁₋₆ alkoxy group, and an electron-withdrawing group,
   (6) a 1H-tetrazol-5-yl group optionally substituted by one substituent selected from the group consisting of an optionally substituted C₁₋₆ alkyl group and an optionally substituted phenyl group,
   (7) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
   (8) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
   (9) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group, and
group (r1) is
   (1) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
   (2) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
   (3) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
   (4) a 5-(1,2,3-triazolyl) group optionally substituted by 1 or 2 substituents selected from the group consisting of an optionally substituted C₁₋₆ alkyl group, an optionally substituted C₁₋₆ alkoxy group, and an electron-withdrawing group,
   (5) a 1H-tetrazol-5-yl group optionally substituted by one substituent selected from the group consisting of an optionally substituted C₁₋₆ alkyl group and an optionally substituted phenyl group,
   (6) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
   (7) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
   (8) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group (referred to as "compound (Ib-iii)" in the present specification).

### (Compound (Ib-iv))

A compound of the aforementioned formula (Ib), wherein
R^{1b} is a C₁₋₆ alkyl group substituted one carboxy group, a 2-benzothiazolyl group, a 2-benzoimidazolyl group, a 2-benzoxazolyl group, a 5-(1,2,3-triazolyl) group, a 1-phenyl-1H-tetrazol-5-yl group, a (pyridine-N-oxide)-2-yl group, a 4-pyridyl group, or a 2-pyridyl group, and
group (r1) is a 2-benzothiazolyl group, a 2-benzoimidazolyl group, a 2-benzoxazolyl group, a 5-(1,2,3-triazolyl) group, a 1-phenyl-1H-tetrazol-5-yl group, a (pyridine-N-oxide)-2-yl group, a 4-pyridyl group, or a 2-pyridyl group (referred to as "compound (Ib-iv)" in the present specification).

### (Compound (Ib-v))

A compound of the aforementioned formula (Ib), wherein
R^{1b} is a C₁₋₆ alkyl group substituted by one carboxy group, a 2-benzothiazolyl group, a 2-benzoimidazolyl group, a 2-benzoxazolyl group, a 5-(1,2,3-triazolyl) group, a 1-phenyl-1H-tetrazol-5-yl group, a (pyridine-N-oxide)-2-yl group, a 4-pyridyl group, or a 2-pyridyl group, and
group (r1) is a 2-benzothiazolyl group, a 2-benzoimidazolyl group, a 2-benzoxazolyl group, a 5-(1,2,3-triazolyl) group, a 1-phenyl-1H-tetrazol-5-yl group, or a (pyridine-N-oxide)-2-yl group (referred to as "compound (Ib-v)" in the present specification).

### (Compound (Ib-i'))

A compound of the aforementioned formula (Ib), wherein
R^{1b} is
   (1) an alkyl group substituted by one carboxy group,
   (2) a 2-benzothiazolyl group optionally substituted by 1 to 4 substituents selected from the group consisting of an optionally substituted alkyl group, an optionally substituted alkoxy group, and an electron-withdrawing group,
   (3) a 2-benzoimidazolyl group optionally substituted by 1 to 5 substituents selected from the group consisting of an optionally substituted alkyl group, an optionally substituted alkoxy group, and an electron-withdrawing group,
   (4) a 2-benzoxazolyl group optionally substituted by 1 to 4 substituents selected from the group consisting of an optionally substituted alkyl group, an optionally substituted alkoxy group, and an electron-withdrawing group,
   (5) a 1H-tetrazol-5-yl group optionally substituted by one substituent selected from the group consisting of an optionally substituted alkyl group and an optionally substituted aryl group,
   (6) a (pyridine-N-oxide)-2-yl group optionally substituted by 1 to 4 substituents selected from the group consisting of an optionally substituted alkyl group, an optionally substituted alkoxy group, and an electron-withdrawing group,
   (7) a 4-pyridyl group optionally substituted by 1 to 4 substituents selected from the group consisting of an optionally substituted alkyl group, an optionally substituted alkoxy group, and an electron-withdrawing group, or
   (8) a 2-pyridyl group optionally substituted by 1 to 4 substituents selected from the group consisting of an optionally substituted alkyl group, an optionally substituted alkoxy group, and an electron-withdrawing group, and
group (r1) is
   (1) a 2-benzothiazolyl group optionally substituted by 1 to 4 substituents selected from the group consisting of an optionally substituted alkyl group, an optionally substituted alkoxy group, and an electron-withdrawing group,
   (2) a 2-benzoimidazolyl group optionally substituted by 1 to 5 substituents selected from the group consisting of an optionally substituted alkyl group, an optionally substituted alkoxy group, and an electron-withdrawing group,
   (3) a 2-benzoxazolyl group optionally substituted by 1 to 4 substituents selected from the group consisting of an optionally substituted alkyl group, an optionally substituted alkoxy group, and an electron-withdrawing group,
   (4) a 1H-tetrazol-5-yl group optionally substituted by one substituent selected from the group consisting of an optionally substituted alkyl group and an optionally substituted aryl group,
   (5) a (pyridine-N-oxide)-2-yl group optionally substituted by 1 to 4 substituents selected from the group consisting of an optionally substituted alkyl group, an optionally substituted alkoxy group, and an electron-withdrawing group,
   (6) a 4-pyridyl group optionally substituted by 1 to 4 substituents selected from the group consisting of an optionally substituted alkyl group, an optionally substituted alkoxy group, and an electron-withdrawing group, or
   (7) a 2-pyridyl group optionally substituted by 1 to 4 substituents selected from the group consisting of an optionally substituted alkyl group, an optionally substituted alkoxy group, and an electron-withdrawing group (referred to as "compound (Ib-I')" in the present specification).

### (Compound (Ib-ii'))

A compound of the aforementioned formula (Ib), wherein
R^{1b} is
   (1) a C₁₋₆ alkyl group substituted by one carboxy group,
   (2) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
   (3) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
   (4) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
   (5) a 1H-tetrazol-5-yl group optionally substituted by one substituent selected from the group consisting of an optionally substituted C₁₋₆ alkyl group and an optionally substituted phenyl group,
   (6) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
   (7) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
   (8) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group, and
group (r1) is
   (1) a 2-benzothiazolyl group optionally substituted by 1 to 4 substituents selected from the group consisting of an optionally substituted C₁₋₆ alkyl group, an optionally substituted C₁₋₆ alkoxy group, and an electron-withdrawing group,
   (2) a 2-benzoimidazolyl group optionally substituted by 1 to 5 substituents selected from the group consisting of an optionally substituted C₁₋₆ alkyl group, an optionally substituted C₁₋₆ alkoxy group, and an electron-withdrawing group,
   (3) a 2-benzoxazolyl group optionally substituted by 1 to 4 substituents selected from the group consisting of an optionally substituted C₁₋₆ alkyl group, an optionally substituted C₁₋₆ alkoxy group, and an electron-withdrawing group,
   (4) a 1H-tetrazol-5-yl group optionally substituted by one substituent selected from the group consisting of an optionally substituted C₁₋₆ alkyl group and an optionally substituted phenyl group,
   (5) a (pyridine-N-oxide)-2-yl group optionally substituted by 1 to 4 substituents selected from the group consisting of an optionally substituted C₁₋₆ alkyl group, an optionally substituted C₁₋₆ alkoxy group, and an electron-withdrawing group,
   (6) a 4-pyridyl group optionally substituted by 1 to 4 substituents selected from the group consisting of an optionally substituted C₁₋₆ alkyl group, an optionally substituted C₁₋₆ alkoxy group, and an electron-withdrawing group, or
   (7) a 2-pyridyl group optionally substituted by 1 to 4 substituents selected from the group consisting of an optionally substituted C₁₋₆ alkyl group, an optionally substituted C₁₋₆ alkoxy group, and an electron-withdrawing group (referred to as "compound (Ib-ii')" in the present specification).

### (Compound (Ib-iii'))

A compound of the aforementioned formula (Ib), wherein
R^{1b} is a C₁₋₆ alkyl group substituted by one carboxy group, a 2-benzothiazolyl group, a 2-benzoimidazolyl group, a 2-benzoxazolyl group, a 1-phenyl-1H-tetrazol-5-yl group, a (pyridine-N-oxide)-2-yl group, a 4-pyridyl group, or a 2-pyridyl group, and
group (r1) is a 2-benzothiazolyl group, a 2-benzoimidazolyl group, a 2-benzoxazolyl group, a 1-phenyl-1H-tetrazol-5-yl group, a (pyridine-N-oxide)-2-yl group, a 4-pyridyl group, or a 2-pyridyl group (referred to as "compound (Ib-iii')" in the present specification).

### (Compound (Ib-iv'))

A compound of the aforementioned formula (Ib), wherein
R^{1b} is a C₁₋₆ alkyl group substituted by one carboxy group, a 2-benzothiazolyl group, a 2-benzoimidazolyl group, a 2-benzoxazolyl group, a 1-phenyl-1H-tetrazol-5-yl group, a (pyridine-N-oxide)-2-yl group, a 4-pyridyl group, or a 2-pyridyl group, and
group (r1) is a 2-benzothiazolyl group, a 2-benzoimidazolyl group, a 2-benzoxazolyl group, a 1-phenyl-1H-tetrazol-5-yl group, or a (pyridine-N-oxide)-2-yl group (referred to as "compound (Ib-iv')" in the present specification).

### (Specific preferable examples of compound (Ib))

Specific preferable examples of compound (Ib) include compounds represented by any of the following formulas (Ib-1) to (Ib-12) (Ph in the following formula (Ib-7) is a phenyl group).

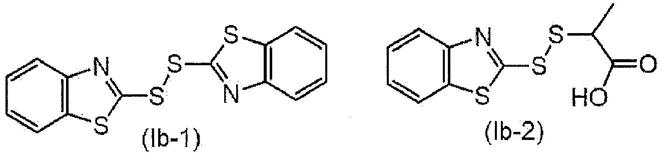

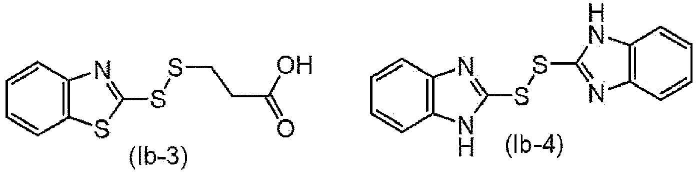

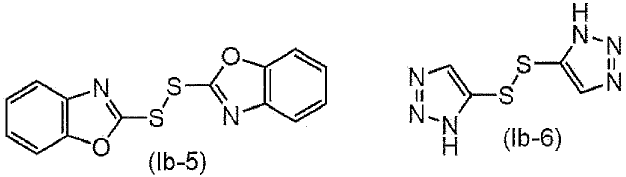

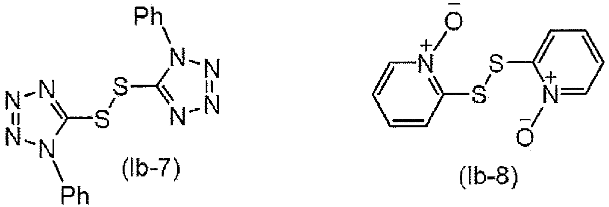

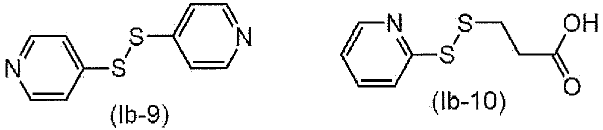

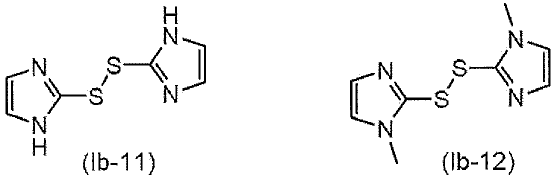

In one embodiment of the production method of the present invention, compound (Ib) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound•(Ib-5)), 5,5'-di(1,2,3-triazolyl) disulfide (compound (Ib-6)), 5,5'-dithiobis(1-phenyl-1H-tetrazole) (compound (Ib-7)), 2,2'-dithiobis(pyridine-N-oxide) (compound (Ib-8)), 4,4'-dipyridyl disulfide (compound (Ib-9)), 3-(2-pyridyldithio)propanoic acid (compound (Ib-10)), 2,2'-dithiobis(1H-imidazole) (compound (Ib-11)), and 2,2'-dithiobis(1-methyl-1H-imidazole) (compound (Ib-12)).

In another embodiment of the production method of the present invention, compound (Ib) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-di(1,2,3-triazolyl) disulfide (compound (Ib-6)), 5,5'-dithiobis(1-phenyl-1H-tetrazole) (compound (Ib-7)), 2,2'-dithiobis(pyridine-N-oxide) (compound (Ib-8)), 4,4'-dipyridyl disulfide (compound (Ib-9)), and 3-(2-pyridyldithio)propanoic acid (compound (Ib-10)).

In another embodiment of the production method of the present invention, compound (Ib) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-dithiobis(1-phenyl-1H-tetrazole) (compound (Ib-7)),- 2,2'-dithiobis(pyridine-N-oxide) (compound (Ib-8)), 4,4'-dipyridyl disulfide (compound (Ib-9)), and 3-(2-pyridyldithio)propanoic acid (compound (Ib-10)).

In another embodiment of the production method of the present invention, compound (Ib) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-di(1,2,3-triazolyl) disulfide (compound (Ib-6)), 5,5'-dithiobis(1-phenyl-1H-tetrazole) (compound (Ib-7)), and 2,2'-dithiobis(pyridine-N-oxide) (compound (Ib-8)).

In another embodiment of the production method of the present invention, compound (Ib) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-dithiobis(1-phenyl-1H-tetrazole) (compound (Ib-7)), and 2,2'-dithiobis(pyridine-N-oxide) (compound (Ib-8)).

In the production method of the present invention, compound (Ib) is most preferably 2,2'-dibenzothiazolyl disulfide (compound (Ib-1)).

### 2-1-2. Compound (ID) to compound (IG)

Compounds (I) other than the aforementioned compound (IA) and compound (IB) can be classified into the following compounds:
a compound of the aforementioned formula (I), wherein X¹ is a single bond, R¹ is a halogen atom, and R² in the aforementioned formula (I) is an optionally substituted aryl group or an optionally substituted heteroaryl group (referred to as "compound (ID)" in the present specification),
a compound of the aforementioned formula (I), wherein X¹ is -S(=O)₂-, R¹ is an optionally substituted alkyl group, an optionally substituted aryl group, or an optionally substituted heteroaryl group, and R² is an optionally substituted aryl group or an optionally substituted heteroaryl group (referred to as "compound (IE)" in the present specification),
a compound of the aforementioned formula (I), wherein X¹ is an oxygen atom, and R¹ and R² form an optionally substituted heterocycle together with a sulfur atom and an oxygen atom bonded thereto (referred to as "compound (IF)" in the present specification), and
a compound of the aforementioned formula (I), wherein X¹ is -N(-R³)-, R¹ and R³ form an optionally substituted heterocycle together with a nitrogen atom bonded thereto, and R² is an optionally substituted aryl group or an optionally substituted heteroaryl group (referred to as "compound (IG)" in the present specification).

Only one kind of compound (ID), compound (IE), compound (IF), and compound (IG) may be used, or two or more kinds thereof may be used in combination. Compound (ID), compound (IE), compound (IF), and compound (IG) are described in order below.

### 2-1-2D. Compound (ID)

Compound (ID) in which X¹ in the aforementioned formula (I) is a single bond is described. Compound (ID) can be represented by the following formula (Id):

R^{2d}-S-R^{1d} (Id)

wherein R^{1d} is a halogen atom and R^{2d} is an optionally substituted aryl group or an optionally substituted heteroaryl group. Only one kind of compound (Id) (= compound (ID)) may be used, or two or more kinds thereof may be used in combination.

R^{1d} in the aforementioned formula (Id) is preferably a chlorine atom. R^{2d} in the aforementioned formula (Id) is preferably an optionally substituted heteroaryl group, more preferably a 5- or 6-membered monocyclic heteroaryl group optionally substituted by an electron-withdrawing group, further preferably a pyridyl group optionally substituted by a nitro group.

As preferred compound (Id), the following compounds can be mentioned. Only one kind of the following compounds may be used, or two or more kinds thereof may be used in combination.

### (Compound (Id-i))

A compound of the aforementioned formula (Id) wherein R^{1d} is a halogen atom, and R^{2d} is an optionally substituted heteroaryl group (referred to as "compound (Id-i)" in the present specification).

### (Compound (Id-ii))

A compound of the aforementioned formula (Id) wherein R^{1d} is a chlorine atom, and R^{2d} is a 5- or 6-membered monocyclic heteroaryl group optionally substituted by an electron-withdrawing group (referred to as "compound (Id-ii)" in the present specification).

### (Compound (Id-iii))

A compound of the aforementioned formula (Id) wherein R^{1d} is a chlorine atom, and R^{2d} is a pyridyl group optionally substituted by a nitro group (referred to as "compound (Id-iii)" in the present specification).

### (Specific preferable example of compound (Id))

Specific preferable example of compound (Id) is 3-nitro-2-pyridinesulfenyl chloride represented by the following formula (Id-1).

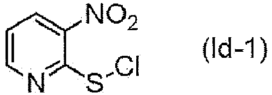

### 2-1-2E. Compound (IE)

Compound (IE) of the aforementioned formula (I), wherein X¹ is -S(=O)₂- is described. Compound (IE) can be represented by the following formula (Ie): 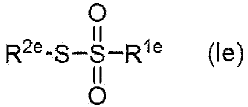 wherein R^{1e} and R^{2e} are each independently an optionally substituted alkyl group, an optionally substituted aryl group, or an optionally substituted heteroaryl group. Only one kind of compound (Ie) (= compound (IE)) may be used, or two or more kinds thereof may be used in combination.

In the aforementioned formula (Ie), R^{1e} and R^{2e} are preferably each independently an optionally substituted aryl group, more preferably an optionally substituted phenyl group.

### (Specific preferable example of compound (Ie))

Specific preferable example of compound (Ie) is (S)-phenyl benzenethiosulfonate represented by the following formula (Ie-1).

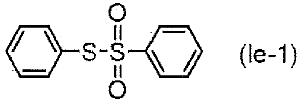

### 2-1-2F. Compound (IF)

Compound (IF) of the aforementioned formula (I) wherein X¹ is an oxygen atom, and R¹ and R² form an optionally substituted heterocycle together with a sulfur atom and an oxygen atom bonded thereto is described. Compound (IF) is preferably a compound represented by the following formula (If): 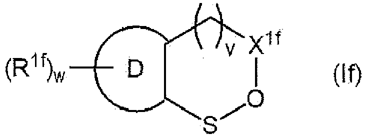 wherein
ring D is a 6-membered aromatic hydrocarbon ring or a 5- or 6-membered aromatic heterocycle,
w is an integer of 0 to 4,
R^{1f} in the number of w are each independently an optionally substituted alkyl group, an optionally substituted alkoxy group, or an electron-withdrawing group,
X^{1f} is -C(=O)-, -C(=S)-, -S(=O)₂-, or -CHR^{2f}-,
R^{2f} is a hydrogen atom, an optionally substituted alkyl group, an optionally substituted alkoxy group, or an electron-withdrawing group, and
v is an integer of 0 to 3. Only one kind of compound (If) may be used, or two or more kinds thereof may be used in combination.

In the aforementioned formula (If), ring D is preferably a 6-membered aromatic hydrocarbon ring (i.e., benzene ring).

In the aforementioned formula (If), w is preferably an integer of 0 to 2, more preferably 0 or 1. That "w is 0" means that R^{1f} is not present.

In the aforementioned formula (If), R^{1f} in the number of w are preferably each independently an optionally substituted C₁₋₆ alkyl group, an optionally substituted C₁₋₆ alkoxy group, a halogen atom, or a nitro group.

In the aforementioned formula (If), R^{2f} is preferably a hydrogen atom, an optionally substituted C₁₋₆ alkyl group, an optionally substituted C₁₋₆ alkoxy group, or an electron-withdrawing group, more preferably a hydrogen atom, an optionally substituted C₁₋₆ alkyl group, an optionally substituted C₁₋₆ alkoxy group, a halogen atom, or a nitro group.

In the aforementioned formula (If), X^{1f} is preferably -C(=O)-.

In the aforementioned formula (If), v is preferably 0. That "v is 0" means that -CH₂- is not present in the parentheses in the aforementioned formula (If).

As preferred compound (If), the following compounds can be mentioned. Only one kind of the following compounds may be used, or two or more kinds thereof may be used in combination.

### (Compound (If-i)

A compound of the aforementioned formula (If), wherein
ring D is a benzene ring,
w is preferably an integer of 0 to 2, and
R^{1f} in the number of w are each independently an optionally substituted alkyl group, an optionally substituted alkoxy group, or an electron-withdrawing group,
X^{1f} is -C(=O)-, and
v is an integer of 0 to 3(referred to as "compound (If-i)" in the present specification).

### (Compound (If-ii)

A compound of the aforementioned formula (If), wherein
ring D is a benzene ring,
w is preferably an integer of 0 to 2, and
R^{1f} in the number of w are each independently an optionally substituted C₁₋₆ alkyl group, an optionally substituted C₁₋₆ alkoxy group, a halogen atom, or a nitro group,
X^{1f} is -C (=O) -, and
v is an integer of 0 to 3 (referred to as "compound (If-ii)" in the present specification).

### (Compound (If-iii)

A compound of the aforementioned formula (If), wherein
ring D is a benzene ring,
w is preferably an integer of 0 or 1,
R^{1f} is an optionally substituted C₁₋₆ alkyl group, an optionally substituted C₁₋₆ alkoxy group, a halogen atom, or a nitro group,
X^{1f} is -C(=O)-, and
v is 0 (referred to as "compound (If-iii)" in the present specification).

### (Specific preferable example of compound (If))

Specific preferable example of compound (If) is 3H-2,1-benzooxathiol-3-one represented by the following formula (If-1).

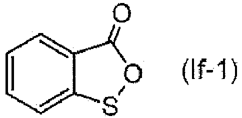

### 2-1-2G. Compound (IG)

Compound (IG) of the aforementioned formula (I), wherein X¹ is -N(-R³)-, R¹ and R³ form, together with a nitrogen atom bonded thereto, an optionally substituted heterocycle, and R² is an optionally substituted aryl group or an optionally substituted heteroaryl group is described. Compound (IG) is preferably a compound represented by the following formula (Ig): 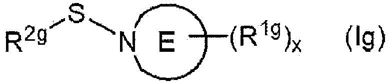 wherein
ring E is a 4 to 8-membered nitrogen-containing heterocycle,
x is an integer of 0 to 6,
R^{1g} in the number of x are each independently an optionally substituted alkyl group, an optionally substituted alkoxy group, an oxo group, or an electron-withdrawing group,
when x is an integer of not less than 2, the adjacent two R^{1g} may form, together with ring E, an optionally substituted bicyclic nitrogen-containing fused heterocycle, and
R^{2g} is an optionally substituted aryl group or an optionally substituted heteroaryl group. Only one kind of compound (Ig) may be used, or two or more kinds thereof may be used in combination.

In the aforementioned formula (Ig), R^{2g} is preferably an optionally substituted heteroaryl group, more preferably an optionally substituted 5- or 6-membered monocyclic nitrogen-containing heteroaryl group, or an optionally substituted 8- to 14-membered fused polycyclic nitrogen-containing heteroaryl group.

R^{2g} is further preferably
   (1) a 2-pyridyl group optionally substituted by 1 to 4 substituents selected from the group consisting of an optionally substituted alkyl group, an optionally substituted alkoxy group, and an electron-withdrawing group, or
   (2) a 2-benzothiazolyl group optionally substituted by 1 to 4 substituents selected from the group consisting of an optionally substituted alkyl group, an optionally substituted alkoxy group, and an electron-withdrawing group.
R^{2g} is particularly preferably
   (1) a 2-pyridyl group optionally substituted by 1 to 4 substituents selected from the group consisting of an optionally substituted C₁₋₆ alkyl group, an optionally substituted C₁₋₆ alkoxy group, a halogen atom, and a nitro group, or
   (2) a 2-benzothiazolyl group optionally substituted by 1 to 4 substituents selected from the group consisting of an optionally substituted C₁₋₆ alkyl group, an optionally substituted C₁₋₆ alkoxy group, a halogen atom, and a nitro group.
R^{2g} is most preferably a 2-pyridyl group or a 2-benzothiazolyl group.

In the aforementioned formula (Ig), x is an integer of 0 to 6, preferably an integer of 2 to 6. That "x is 0" means that R^{1g} is not present.

In the aforementioned formula (Ig), R^{1g} in the number of x are preferably each independently an optionally substituted C₁₋₆ alkyl group, an optionally substituted C₁₋₆ alkoxy group, an oxo group, a halogen atom, or a nitro group.

In compound (Ig), a group represented by the formula (r17): 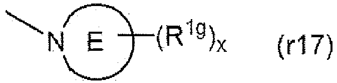 is preferably a group represented by the formula (r17'): 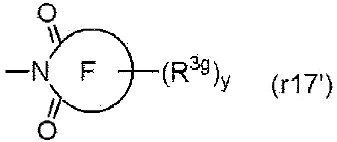 wherein
ring F is a 4- to 8-membered nitrogen-containing heterocycle,
y is an integer of 0 to 4, and
R^{3g} in the number of y are each independently an optionally substituted alkyl group, an optionally substituted alkoxy group, or an electron-withdrawing group, and
when y is an integer of not less than 2, the adjacent two R^{3g} may form, together with ring F, an optionally substituted bicyclic nitrogen-containing fused heterocycle.

In the aforementioned formula (r17'), y is 0 to 4. That "y is 0" means that R^{3g} is not present.

In the aforementioned formula (r17'), R^{3g} in the number of y are preferably each independently a substituted C₁₋₆ alkyl group, an optionally substituted C₁₋₆ alkoxy group, a halogen atom, or a nitro group, or adjacent two R^{3g} preferably form, together with ring F, an optionally substituted bicyclic nitrogen-containing fused heterocycle. The aforementioned bicyclic nitrogen-containing fused heterocycle is preferably an isoindole ring optionally substituted by 1 to 4 substituents selected from the group consisting of an optionally substituted alkyl group, an optionally substituted alkoxy group, and an electron-withdrawing group, more preferably an isoindole ring optionally substituted by 1 to 4 substituents selected from the group consisting of an optionally substituted C₁₋₆ alkyl group, an optionally substituted C₁₋₆ alkoxy group, a halogen atom, and a nitro group.

Ring F is preferably a pyrrolidine ring, or form, together with the adjacent two R^{3g}, an isoindole ring optionally substituted by 1 to 4 substituents selected from the group consisting of an optionally substituted alkyl group, an optionally substituted alkoxy group, and an electron-withdrawing group.

Ring F is more preferably a pyrrolidine ring, or form, together with the adjacent two R^{3g}, an isoindole ring optionally substituted by 1 to 4 substituents selected from the group consisting of an optionally substituted C₁₋₆ alkyl group, an optionally substituted C₁₋₆ alkoxy group, a halogen atom, and a nitro group.

Compound (Ig) is preferably a compound having an imide bond (-C(=O)-N(-S-R^{2g})-C(=O)-) which is represented by the following formula (Ig') (symbols in the following formula are as defined above).

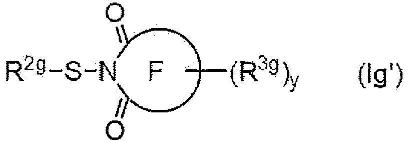

As preferred compound (Ig'), the following compounds can be mentioned. Only one kind of the following compounds may be used, or two or more kinds thereof may be used in combination.

### (Compound (Ig-i-a))

A compound of the aforementioned formula (Ig'), wherein
ring F is a pyrrolidine ring,
y is an integer of 0 to 4,
R^{3g} in the number of y are each independently an optionally substituted alkyl group, an optionally substituted alkoxy group, or an electron-withdrawing group, and
R^{2g} is an optionally substituted heteroaryl group (referred to as "compound (Ig-i-a)" in the present specification).

### (Compound (Ig-i-b))

A compound of the aforementioned formula (Ig'), wherein
ring F forms, together with the adjacent two R^{3g}, an isoindole ring optionally substituted by 1 to 4 substituents selected from the group consisting of an optionally substituted alkyl group, an optionally substituted alkoxy group, and an electron-withdrawing group, and
R^{2g} is an optionally substituted heteroaryl group (referred to as "compound (Ig-i-b)" in the present specification).

### (Compound (Ig-ii-a))

A compound of the aforementioned formula (Ig'), wherein
ring F is a pyrrolidine ring,
y is an integer of 0 to 4,
R^{3g} in the number of y are each independently an optionally substituted alkyl group, an optionally substituted alkoxy group, or an electron-withdrawing group, and
R^{2g} is an optionally substituted 5- or 6-membered monocyclic nitrogen-containing heteroaryl group, or an optionally substituted 8- to 14-membered fused polycyclic nitrogen-containing heteroaryl group (referred to as "compound (Ig-ii-a)" in the present specification).

### (Compound (Ig-ii-b))

A compound of the aforementioned formula (Ig'), wherein
ring F forms, together with the adjacent two R^{3g}, an isoindole ring optionally substituted by 1 to 4 substituents selected from the group consisting of an optionally substituted alkyl group, an optionally substituted alkoxy group, and an electron-withdrawing group, and
R^{2g} is an optionally substituted 5- or 6-membered monocyclic nitrogen-containing heteroaryl group, or an optionally substituted 8- to 14-membered fused polycyclic nitrogen-containing heteroaryl group (referred to as "compound (Ig-ii-b)" in the present specification).

### (Compound (Ig-iii-a))

A compound of the aforementioned formula (Ig'), wherein
ring F is a pyrrolidine ring,
y is an integer of 0 to 4,
R^{3g} in the number of y are each independently an optionally substituted alkyl group, an optionally substituted alkoxy group, or an electron-withdrawing group, and
R^{2g} is
   (1) a 2-pyridyl group optionally substituted by 1 to 4 substituents selected from the group consisting of an optionally substituted alkyl group, an optionally substituted alkoxy group, and an electron-withdrawing group, or
   (2) a 2-benzothiazolyl group optionally substituted by 1 to 4 substituents selected from the group consisting of an optionally substituted alkyl group, an optionally substituted alkoxy group, and an electron-withdrawing group (referred to as "compound (Ig-iii-a)" in the present specification).

### (Compound (Ig-iii-b))

A compound of the aforementioned formula (Ig'), wherein
ring F forms, together with the adjacent two R^{3g}, an isoindole ring optionally substituted by 1 to 4 substituents selected from the group consisting of an optionally substituted alkyl group, an optionally substituted alkoxy group, and an electron-withdrawing group, and
R^{2g} is
   (1) a 2-pyridyl group optionally substituted by 1 to 4 substituents selected from the group consisting of an optionally substituted alkyl group, an optionally substituted alkoxy group, and an electron-withdrawing group, or
   (2) a 2-benzothiazolyl group optionally substituted by 1 to 4 substituents selected from the group consisting of an optionally substituted alkyl group, an optionally substituted alkoxy group, and an electron-withdrawing group (referred to as "compound (Ig-iii-b)" in the present specification).

### (Compound (Ig-iv-a))

A compound of the aforementioned formula (Ig'), wherein
ring F is a pyrrolidine ring,
y is an integer of 0 to 4,
R^{3g} in the number of y are each independently a substituted C₁₋₆ alkyl group, an optionally substituted C₁₋₆ alkoxy group, a halogen atom, or a nitro group,
R^{2g} is
   (1) a 2-pyridyl group optionally substituted by 1 to 4 substituents selected from the group consisting of an optionally substituted C₁₋₆ alkyl group, an optionally substituted C₁₋₆ alkoxy group, a halogen atom, and a nitro group, or
   (2) a 2-benzothiazolyl group optionally substituted by 1 to 4 substituents selected from the group consisting of an optionally substituted C₁₋₆ alkyl group, an optionally substituted C₁₋₆ alkoxy group, a halogen atom, and a nitro group (referred to as "compound (Ig-iv-a)" in the present specification).

### (Compound (Ig-iv-b))

A compound of the aforementioned formula (Ig'), wherein
ring F forms, together with the adjacent two R^{3g}, an isoindole ring optionally substituted by 1 to 4 substituents selected from the group consisting of an optionally substituted C₁₋₆ alkyl group, an optionally substituted C₁₋₆ alkoxy group, a halogen atom, and a nitro group, and
R^{2g} is
   (1) a 2-pyridyl group optionally substituted by 1 to 4 substituents selected from the group consisting of an optionally substituted C₁₋₆ alkyl group, an optionally substituted C₁₋₆ alkoxy group, a halogen atom, and a nitro group, or
   (2) a 2-benzothiazolyl group optionally substituted by 1 to 4 substituents selected from the group consisting of an optionally substituted C₁₋₆ alkyl group, an optionally substituted C₁₋₆ alkoxy group, a halogen atom, and a nitro group (referred to as "compound (Ig-iv-b)" in the present specification).

### (Specific preferable examples of compound (Ig))

Specific preferable examples of compound (Ig) include 1-(2-pyridinylthio)-2,5-pyrrolidinedione represented by the following formula (Ig-1), 1-(2-benzothiazolylthio)-2,5-pyrrolidinedione represented by the following formula (Ig-2), 2-(2-pyridinylthio)-1H-isoindole-1,3(2H)-dione represented by the following formula (Ig-3), and 2-(2-benzothiazolylthio)-1H-isoindole-1,3(2H)-dione represented by the following formula (Ig-4).

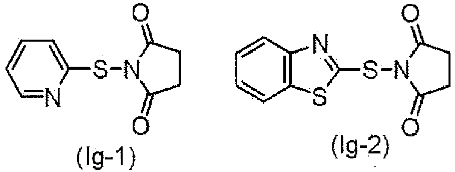

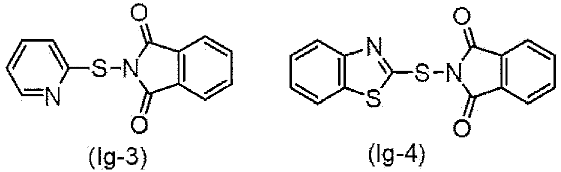

### 2-1-3. Preferred compound (I)

In the production method of the present invention, compound (I) preferably includes compound (Ia) and/or compound (Ib). Among compounds (Ia), compound (Ic) is preferred.

In one embodiment of the production method of the present invention, compound (I) preferably includes compound (Ib) and/or compound (Ic), more preferably compound (Ib-i) and/or compound (Ic-i), still more preferably compound (Ib-ii) and/or compound (Ic-ii), further preferably compound (Ib-iii) and/or compound (Ic-iii), further more preferably compound (Ib-iv) and/or compound (Ic-iv), particularly preferably compound (Ib-v) and/or compound (Ic-iv).

In another embodiment of the production method of the present invention, compound (I) preferably includes compound (Ib-i') and/or compound (Ic-i'), more preferably compound (Ib-ii') and/or compound (Ic-ii'), still more preferably compound (Ib-iii') and/or compound (Ic-iii'), further preferably compound (Ib-iv') and/or compound (Ic-iv').

In another embodiment of the production method of the present invention, compound (I) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-di(1,2,3-triazolyl) disulfide (compound (Ib-6)), 5,5'-dithiobis(1-phenyl-1H-tetrazole) (compound (Ib-7)), 2,2'-dithiobis(pyridine-N-oxide) (compound (Ib-8)), 4,4'-dipyridyl disulfide (compound (Ib-9)), 3-(2-pyridyldithio)propanoic acid (compound (Ib-10)), 2,2'-dithiobis(1H-imidazole) (compound (Ib-11)), 2,2'-dithiobis(1-methyl-1H-imidazole) (compound (Ib-12)), 2-phenylisothiazolo[5,4-b]pyridin-3(2H)-one (compound (Ic-1)), 2-isopropylisothiazolo[5,4-b]pyridin-3(2H)-one (compound (Ic-2)), 2-isopropylisothiazolo[4,5-c]pyridin-3(2H)-one (compound (Ic-3)), 2-(2-pyridyl)-1,2-benzothiazol-3(2H)-one (compound (Ic-4)), 5-nitro-2-phenyl-1,2-benzothiazol-3(2H)-one (compound (Ic-5)), 2-(2,6-dimethylphenyl)-5-nitro-1,2-benzothiazol-3(2H)-one (compound (Ic-6)), 5-nitro-2-(2-pyridyl)-1,2-benzothiazol-3(2H)-one (compound (Ic-7)), 5-nitro-2-(4-pyridyl)-1,2-benzothiazol-3(2H)-one (compound (Ic-8)), and 2-[1-methyl-1-(2-pyridyl)ethyl]-5-nitro-1,2-benzothiazol-3(2H)-one (compound (Ic-9)).

In another embodiment of the production method of the present invention, compound (I) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-di(1,2,3-triazolyl) disulfide (compound (Ib-6)), 5,5'-dithiobis(1-phenyl-1H-tetrazole) (compound (Ib-7)), 2,2'-dithiobis(pyridine-N-oxide) (compound (Ib-8)), 4,4'-dipyridyl disulfide (compound (Ib-9)), 3-(2-pyridyldithio)propanoic acid (compound (Ib-10)), 2-phenylisothiazolo[5,4-b]pyridin-3(2H)-one (compound (Ic-1)), 2-isopropylisothiazolo[5,4-b]pyridin-3(2H)-one (compound (Ic-2)), 2-isopropylisothiazolo[4,5-c]pyridin-3(2H)-one (compound (Ic-3)), 2-(2-pyridyl)-1,2-benzothiazol-3(2H)-one (compound (Ic-4)), 5-nitro-2-phenyl-1,2-benzothiazol-3(2H)-one (compound (Ic-5)), 2-(2,6-dimethylphenyl)-5-nitro-1,2-benzothiazol-3(2H)-one (compound (Ic-6)), 5-nitro-2-(2-pyridyl)-1,2-benzothiazol-3(2H)-one (compound (Ic-7)), 5-nitro-2-(4-pyridyl)-1,2-benzothiazol-3(2H)-one (compound (Ic-8)), and 2-[1-methyl-1-(2-pyridyl)ethyl]-5-nitro-1,2-benzothiazol-3(2H)-one (compound (Ic-9)).

In another embodiment of the production method of the present invention, compound (I) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-di(1,2,3-triazolyl) disulfide (compound (Ib-6)), 5,5'-dithiobis(1-phenyl-1H-tetrazole) (compound (Ib-7)), 2,2'-dithiobis(pyridine-N-oxide) (compound (Ib-8)), 2-phenylisothiazolo[5,4-b]pyridin-3(2H)-one (compound (Ic-1)), 2-isopropylisothiazolo[5,4-b]pyridin-3(2H)-one (compound (Ic-2)), 2-isopropylisothiazolo[4,5-c]pyridin-3(2H)-one (compound (Ic-3)), 2-(2-pyridyl)-1,2-benzothiazol-3(2H)-one (compound (Ic-4)), 5-nitro-2-phenyl-1,2-benzothiazol-3(2H)-one (compound (Ic-5)), 2-(2,6-dimethylphenyl)-5-nitro-1,2-benzothiazol-3(2H)-one (compound (Ic-6)), 5-nitro-2-(2-pyridyl)-1,2-benzothiazol-3(2H)-one (compound (Ic-7)), 5-nitro-2-(4-pyridyl)-1,2-benzothiazol-3(2H)-one (compound (Ic-8)), and 2-[1-methyl-1-(2-pyridyl)ethyl]-5-nitro-1,2-benzothiazol-3(2H)-one (compound (Ic-9)).

In another embodiment of the production method of the present invention, compound (I) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-di(1,2,3-triazolyl) disulfide (compound (Ib-6)), 5,5'-dithiobis(1-phenyl-1H-tetrazole) (compound (Ib-7)), 2,2'-dithiobis(pyridine-N-oxide) (compound (Ib-8)), 4,4'-dipyridyl disulfide (compound (Ib-9)), 3-(2-pyridyldithio)propanoic acid (compound (Ib-10)), 2,2'-dithiobis(1H-imidazole) (compound (Ib-11)), 2,2'-dithiobis(1-methyl-1H-imidazole) (compound (Ib-12)), 2-phenylisothiazolo[5,4-b]pyridin-3(2H)-one (compound (Ic-1)), 2-isopropylisothiazolo[5,4-b]pyridin-3(2H)-one (compound (Ic-2)), 2-isopropylisothiazolo[4,5-c]pyridin-3(2H)-one (compound (Ic-3)), 2-(2-pyridyl)-1,2-benzothiazol-3(2H)-one (compound (Ic-4)), 5-nitro-2-phenyl-1,2-benzothiazol-3(2H)-one (compound (Ic-5)), 2-(2,6-dimethylphenyl)-5-nitro-1,2-benzothiazol-3(2H)-one (compound (Ic-6)), 5-nitro-2-(2-pyridyl)-1,2-benzothiazol-3(2H)-one (compound (Ic-7)), 5-nitro-2-(4-pyridyl)-1,2-benzothiazol-3(2H)-one (compound (Ic-8)), 2-[1-methyl-1-(2-pyridyl)ethyl]-5-nitro-1,2-benzothiazol-3(2H)-one (compound (Ic-9)), 2-phenylisothiazolo[4,5-c]pyridin-3(2H)-one (compound (Ic-10)), 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In another embodiment of the production method of the present invention, compound (I) is preferably at least one selected from the group consisting of 2,2'-dibenzothiazolyl disulfide (compound (Ib-1)), 2-(2-benzothiazolyldithio)propanoic acid (compound (Ib-2)), 3-(2-benzothiazolyldithio)propanoic acid (compound (Ib-3)), 2,2'-dibenzoimidazolyl disulfide (compound (Ib-4)), 2,2'-dibenzoxazolyl disulfide (compound (Ib-5)), 5,5'-dithiobis(1-phenyl-1H-tetrazole) (compound (Ib-7)), 2,2'-dithiobis(pyridine-N-oxide) (compound (Ib-8)), 4,4'-dipyridyl disulfide (compound (Ib-9)), 3-(2-pyridyldithio)propanoic acid (compound (Ib-10)), 2-phenylisothiazolo[5,4-b]pyridin-3(2H)-one (compound (Ic-1)), 2-isopropylisothiazolo[5,4-b]pyridin-3(2H)-one (compound (Ic-2)), 5-nitro-2-phenyl-1,2-benzothiazol-3(2H)-one (compound (Ic-5)), 2-phenylisothiazolo[4,5-c]pyridin-3(2H)-one (compound (Ic-10)), 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In another embodiment of the production method of the present invention, compound (I) is preferably at least one selected from the group consisting of 2,2'-dibenzothiazolyl disulfide (compound (Ib-1)), 2-phenylisothiazolo[5,4-b]pyridin-3(2H)-one (compound (Ic-1)), 2-isopropylisothiazolo[5,4-b]pyridin-3(2H)-one (compound (Ic-2)), 2-phenylisothiazolo[4,5-c]pyridin-3(2H)-one (compound (Ic-10)), 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In another embodiment of the production method of the present invention, compound (I) is preferably at least one selected from the group consisting of 2,2'-dibenzothiazolyl disulfide (compound (Ib-1)), 2-phenylisothiazolo[5,4-b]pyridin-3(2H)-one (compound (Ic-1)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), 2-(2,6-dimethylphenyl)isothiazolo[5,4-b]pyridin-3(2H)-one (compound (Ic-14)), 2-(4-methyl-2-pyridinyl)isothiazolo[5,4-b]pyridin-3(2H)-one (compound (Ic-15)), 2-(4-methoxyphenyl)isothiazolo[5,4-b]pyridin-3(2H)-one (compound (Ic-16)), and 2-(4-fluorophenyl)isothiazolo[5,4-b]pyridin-3(2H)-one (compound (Ic-17)).

In the production method of the present invention, compound(s) (I) are/is particularly preferably 2,2'-dibenzothiazolyl disulfide (compound (Ib-1)) and/or 2-phenylisothiazolo[5,4-b]pyridin-3(2H)-one (compound (Ic-1)), most preferably 2-phenylisothiazolo[5,4-b]pyridin-3(2H)-one (compound (Ic-1)).

### 2-1-4. Production method of compound (I)

A commercially available product may be used as compound (I), or compound (I) may be produced by a known method. For example, compound (Ib-1) (i.e., 2,2'-dibenzothiazolyl disulfide, MBTS) and the like are commercially available.

A representative production methods are shown in the following as exemplary production methods of compound (I); however, the production method is not limited to them.

Compound (I) can be produced by the following representative production method, the below-mentioned Synthetic Example, a known method (e.g., the method described in Supporting Information of Non Patent Literature 2) or a method analogous thereto.

A schematic diagram of the reaction in each step in the representative production method is shown below. Each symbol in the schematic diagram means the same as described above.

### (Production method of compound (Ib))

Compound (Ib) can be produced, for example, by oxidation and coupling of thiol as shown in the following formula.

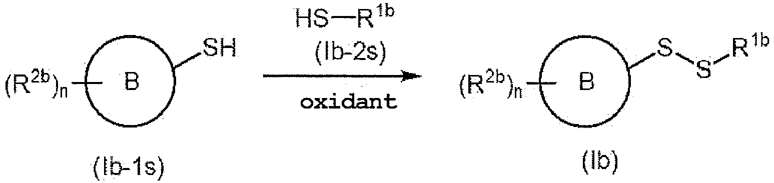

Examples of the oxidant for oxidizing compound (Ib-1s) and compound (Ib-2s) include hydrogen peroxide, iodine, oxygen, N-bromosuccinimide, N-chlorosuccinimide, and the like. Only one kind of the oxidant may be used, or two or more kinds thereof may be used in combination.

The amount of the oxidant to be used is preferably 1 to 10 mol, more preferably 1 to 5 mol, per 1 mol in total of compound (Ib-1s) and compound (Ib-2s). It is preferable to use equal amounts of compound (Ib-2s) and compound (Ib-1s).

This reaction is performed in an appropriate solvent that does not inhibit the reaction. Examples of the aforementioned solvent include ester solvents such as ethyl acetate, isopropyl acetate, and the like; polar ether solvents such as 1,4-dioxane, tetrahydrofuran, and the like; halogenated solvents such as dichloromethane, chloroform, and the like; aromatic solvents such as benzene, toluene, xylene, mesitylene, and the like, and the like. The amount of the aforementioned solvent to be used is preferably 1 to 20 mL, more preferably 3 to 10 mL, per 1 mmol in total of compound (Ib-1s) and compound (Ib-2s).

The reaction temperature is preferably -10°C to 50°C, more preferably 0°C to 40°C, and the reaction time is preferably 1 to 24 hr, more preferably 1 to 10 hr.

After the aforementioned reaction, compound (Ib) can be recovered by a known method (e.g., extraction, etc.). The recovered compound (Ib) can be purified by a known means (e.g., recrystallization, chromatography, etc.).

### (Production method of compound (Ic) wherein Y^{1c} is carbonyl)

Compound (Ic) wherein Y^{1c} is carbonyl can be produced, for example, by the steps shown in the following.

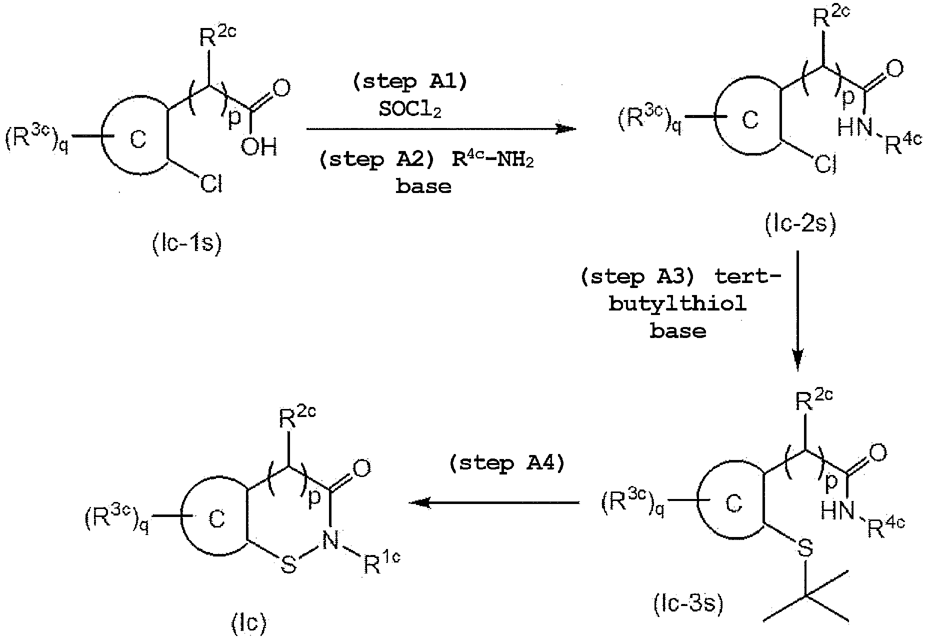

### Step A1

In this step, an acid chloride of compound (Ic-1s) is synthesized. To be specific, an acid chloride of compound (Ic-1s) can be obtained by adding compound (Ic-1s) and an excess amount of thionyl chloride (e.g., 10 mol per 1 mol of compound (Ic-1s)) into a reactor and refluxing the mixture for 1 hr to one night.

This step is preferably performed under non-active atmosphere (e.g., under argon atmosphere). After completion of the reaction, thionyl chloride is evaporated to recover a solid containing the acid chloride of compound (Ic-1s).

### Step A2

In this step, compound (Ic-2s) is synthesized by reacting an acid chloride of compound (Ic-1s) with appropriate amine (R^{4c}-NH₂). To be specific, the solid containing the acid chloride of compound (Ic-1s) obtained in step A1, appropriate amine (R^{4c}-NH₂), and a base are added to an appropriate solvent and reacted to give compound (Ic-2s).

The base may be either an organic base or an inorganic base, preferably triethylamine. The amounts of R^{4c}-NH₂ and base to be used are each preferably about 1 to 1.5 mol per 1 mol of the acid chloride of compound (Ic-1s).

This step is performed in an appropriate solvent that does not inhibit the reaction. Examples of the aforementioned solvent include polar ether solvents such as 1,4-dioxane, tetrahydrofuran (THF), and the like, and THF is preferred. The amount of the aforementioned solvent to be used is not particularly limited as long as it can dissolve the aforementioned solid. It is preferably about 5 to 10 mL per 1 mmol of the acid chloride of compound (Ic-1s).

This step is preferably performed at low temperatures (e.g., about 0°C). Completion of the reaction can be confirmed by TLC or the like. After completion of the reaction, compound (Ic-2s) can be recovered by a known means (e.g., concentration and extraction).

### Step A3

In this step, compound (Ic-3s) is synthesized by reacting compound (Ic-2s) with tert-butylthiol. To be specific, compound (Ic-2s) is reacted with tert-butylthiol in the presence of a base in an appropriate solvent to give compound (Ic-3s).

The amount of tert-butylthiol to be used is preferably about 1 to 1.5 mol per 1 mol of compound (Ic-2s).

As a base to be used in this step, alkali metal hydroxides such as sodium hydroxide, potassium hydroxide, and the like can be mentioned, and sodium hydroxide is preferred. The amount of the base to be used is preferably about 1 to 2 mol per 1 mol of compound (Ic-3s).

This step is performed in an appropriate solvent that does not inhibit the reaction. Examples of the aforementioned solvent include amide solvents such as dimethylformamide (DMF), dimethylacetamide, N-methylpiperidone, and the like, and DMF is preferred. The amount of the aforementioned solvent to be used is not particularly limited as long as it can dissolve compound (Ic-2s). It is preferably about 5 to 10 mL per 1 mmol of compound (Ic-2s) .

In this step, the reaction temperature is around room temperature (e.g., 25°C) and the time thereof is, for example, 1 hr to one night, preferably about 3 to 20 hr. After completion of the reaction, compound (Ic-3s) can be recovered by a known means (e.g., solid-liquid separation).

### Step A4

In this step, compound (Ic) wherein Y^{1c} is carbonyl is synthesized by a ring closure reaction of compound (Ic-3s). This step can be performed in two ways, the following step A4-1 and step A4-2.

### Step A4-1

In this step, compound (Ic-3s), dimethyl sulfoxide (DMSO), and chlorotrimethylsilane (TMSCl) are added to an appropriate solvent, and the obtained mixture is reacted at around room temperature (e.g., 25°C) to synthesize compound (Ic) wherein Y^{1c} is carbonyl.

The amount of DMSO to be used is preferably about 1 to 2 mol per 1 mol of compound (Ic-3s). The amount of TMSCl to be used is preferably about 1 to 1.5 mol per 1 mol of compound (Ic-3s).

This step is performed in an appropriate solvent that does not inhibit the reaction. Examples of the aforementioned solvent include halogenated solvents such as chloroform, dichloromethane, 1,2-dichloroethane, and the like, and dichloromethane is preferred. The amount of the aforementioned solvent to be used is not particularly limited as long as it can dissolve compound (Ic-3s). It is preferably about 2 to 5 mL per 1 mmol of compound (Ic-3s).

The reaction time in this step is, for example, 1 hr to one night, preferably about 5 to 10 hr. After completion of the reaction, compound (Ic) wherein Y^{1c} is carbonyl can be recovered by a known means (e.g., solid-liquid separation). The recovered compound (Ic) can be purified by a known means (e.g., recrystallization, chromatography, etc.).

### Step A4-2

In this step, compound (Ic-3s) and meta-chloroperoxybenzoic acid (mCPBA) are reacted in an appropriate solvent to give a sulfoxide intermediate, and the intermediate is heated to synthesize compound (Ic) wherein Y^{1c} is carbonyl.

The amount of mCPBA to be used is preferably about 1 to 1.5 mol per 1 mol of compound (Ic-3s).

Compound (Ic-3s) and meta-chloroperoxybenzoic acid (mCPBA) are reacted in an appropriate solvent that does not inhibit the reaction. Examples of the aforementioned solvent include halogenated solvents such as chloroform, dichloromethane, 1,2-dichloroethane, and the like, and dichloromethane is preferred. The amount of the aforementioned solvent to be used is not particularly limited as long as it can dissolve compound (Ic-3s). It is preferably about 8 to 15 mL per 1 mmol of compound (Ic-3s).

Compound (Ic-3s) and meta-chloroperoxybenzoic acid (mCPBA) are preferably reacted at low temperatures (e.g., about 0°C). The reaction time thereof is, for example, about 30 min to 1 hr. After completion of the reaction, a sulfoxide intermediate can be recovered by a known means (e.g., concentration and recrystallization after extraction, etc.).

The recovered sulfoxide intermediate is heated in an appropriate solvent that does not inhibit the cyclization reaction, whereby compound (Ic) wherein Y^{1c} is carbonyl can be synthesized. Examples of the aforementioned solvent include toluene, xylene, pyridine, and a combination of these, and a combination of toluene and pyridine is preferred. The aforementioned reaction temperature is preferably 70°C to 120°C. The synthesized compound (Ic) can be purified by a known means (e.g., recrystallization, chromatography, etc.).

The aforementioned compound (Ic-2s) (i.e., intermediate to synthesize compound (Ic) wherein Y^{1c} is carbonyl) can also be produced, for example, by the following step.

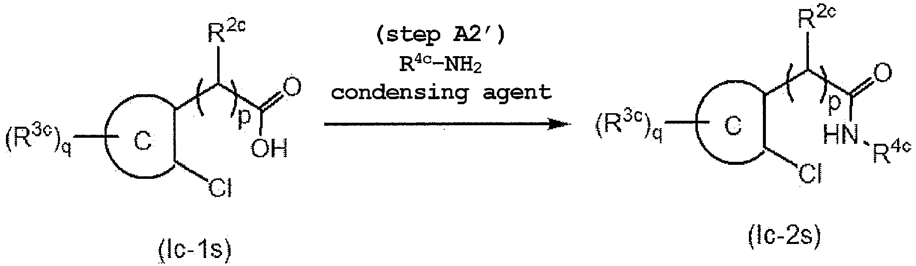

### Step A2'

In this step, compound (Ic-1s) is reacted with appropriate amine (R^{4c}-NH₂) using a condensing agent to synthesize compound (Ic-2s) .

Examples of the condensing agent to be used in this step include dicyclohexylcarbodiimide (DCC), diisopropylcarbodiimide (DIC), 1-ethyl-3-[3-(dimethylamino)propyl]carbodiimide (EDC) and hydrochloride thereof (EDC·HCl), (benzotriazol-1-yloxy)tripyrrolidinophosphonium hexafluorophosphate (PyBop), O-(benzotriazol-1-yl)-N,N,N',N'-tetramethyluronium tetrafluoroborate (TBTU), 1-[bis(dimethylamino)methylene]-5-chloro-1H-benzotriazolium-3-oxide hexafluorophosphate (HCTU), O-benzotriazole-N,N,N',N'-tetramethyluronium hexafluorophosphate (HBTU), and the like. Among these, EDC·HCl is preferred.

The amounts of R^{4c}-NH₂ and the condensing agent to be used are each preferably about 1 to 5 mol per 1 mol of compound (Ic-1s).

A base may be used in this step. The base may be either an organic base or an inorganic base, and triethylamine is preferred. When a base is used, the amount thereof is preferably about 1 to 5 mol per 1 mol of compound (Ic-1s).

This step is performed in an appropriate solvent that does not inhibit the reaction. Examples of the aforementioned solvent include polar ether solvents such as 1,4-dioxane, tetrahydrofuran (THF), and the like, and THF is preferred. The amount of the aforementioned solvent to be used is not particularly limited and it is preferably about 1 to 10 mL per 1 mmol of compound (Ic-1s).

In this step, the reaction temperature is around room temperature (e.g., 25°C), and the time thereof is, for example, 1 hr to one night, preferably about 3 to 20 hr. This step is preferably performed under non-active atmosphere (e.g., under argon atmosphere). Completion of the reaction can be confirmed by TLC or the like. After completion of the reaction, compound (Ic-2s) can be recovered by a known means (e.g., concentration and extraction).

### 2-2. Oxidation

The production method of the present invention is characterized in that an oligonucleotide precursor having a phosphite ester bond or a phosphonate ester bond (hereinafter sometimes to be abbreviated as "oligonucleotide precursor") is oxidized using compound (I) excluding 2,2-dipyridyl disulfide.

Production of byproducts (defective byproduct and desulfurized byproduct) can be suppressed by using compound (I) as an oxidant. In order to suppress production of byproducts, the amount of compound (I) to be used in the production method of the present invention is preferably not less than 80 mol%, more preferably not less than 90 mol%, with respect to the whole oxidant. In the production method of the present invention, the oxidant further preferably consists of compound (I).

From the aspect of completion of the oxidation reaction, the amount of compound (I) to be used is preferably 1 to 15 mol, more preferably 1 to 10 mol, further preferably 1 to 5 mol, per 1 mol of the oligonucleotide precursor.

The oligonucleotide precursor is preferably
(1) an oligonucleotide precursor having a phosphite ester bond which is obtained by condensation of a nucleoside, nucleotide, or oligonucleotide with a phosphoramidited nucleoside, nucleotide, or oligonucleotide, or
(2) an oligonucleotide precursor having a phosphite ester bond which is obtained by condensation of an oligonucleotide with a phosphoramidite. Examples of the aforementioned oligonucleotide precursor (1) include the aforementioned compound (c-I) (i.e., oligonucleotide precursor having a phosphite ester bond which is obtained by condensation of the aforementioned compound (a-I) and the aforementioned compound (b-I)), and the aforementioned compound (c-I') (i.e., oligonucleotide precursor having a phosphite ester bond which is obtained by condensation of the aforementioned compound (a-I') and the aforementioned compound (bI')). Examples of the aforementioned oligonucleotide precursor (2) include the aforementioned compound (y-I) and the aforementioned compound (γ-I').

The oligonucleotide precursor having a phosphite ester bond is more preferably an oligonucleotide precursor having a phosphite ester bond which is obtained by condensation of a nucleoside, nucleotide, or oligonucleotide with a phosphoramidited nucleoside, nucleotide, or oligonucleotide.

The oligonucleotideprecursor is further preferably an oligonucleotide precursor having a phosphite ester bond, which is obtained by condensation of nucleoside, nucleotide, or oligonucleotide (a), each having a hydrophobic protecting group, and nucleoside, nucleotide, or oligonucleotide (b), each of which is phosphoramidited and in which a hydroxy group is protected by a temporary protecting group removable under acidic conditions.

The oligonucleotide precursor is particularly preferably the aforementioned compound (c-I) wherein Pg is **L-Y-Z (i.e., oligonucleotide precursor having a phosphite ester bond which is obtained by condensation of the aforementioned compound (a-I) wherein Pg is **L-Y-Z and the aforementioned compound (b-I)), or the aforementioned compound (c-I') wherein Pg is **L-Y-Z (i.e., oligonucleotide precursor having a phosphite ester bond which is obtained by condensation of the aforementioned compound (a-I') wherein Pg is **L-Y-Z and the aforementioned compound (b-I')).

The oligonucleotide precursor is preferably an oligonucleotide precursor having a thiophosphate ester bond in addition to a phosphite ester bond or a phosphonate ester bond. When an oligonucleotide precursor having a thiophosphate ester bond is oxidized with iodine, thiophosphate ester bond is converted to a phosphate ester bond to produce the resulting byproduct (desulfurized byproduct). According to the present invention using compound (I) as an oxidant, the production of the desulfurized byproduct can be suppressed.

The oligonucleotide precursor is preferably oxidized in the presence of water. The water present in the system acts as a source of oxygen atom, and promotes oxidation of the oligonucleotide precursor. The amount of water is preferably 0.5 to 200 mol, more preferably 1 to 100 mol, further preferably 1 to 50 mol, per 1 mol of the oligonucleotide precursor.

The temperature of oxidation reaction of the oligonucleotide precursor is preferably 10 to 50°C, more preferably 15 to 40°C, and the reaction time thereof is preferably 5 min to 5 hr, more preferably 30 min to 3 hr.

The oligonucleotide precursor is preferably oxidized in a solution containing a non-polar solvent. The concentration of the oligonucleotide precursor in the solution is not particularly limited as long as it is dissolved in the solvent. It is preferably 1 to 30 wt%.

Examples of the non-polar solvent include halogenated solvents such as chloroform, dichloromethane, 1,2-dichloroethane, and the like; aromatic solvents such as benzene, toluene, xylene, mesitylene, and the like; ester solvents such as ethyl acetate, isopropyl acetate, and the like; aliphatic solvents such as hexane, pentane, heptane, octane, nonane, cyclohexane, and the like; non-polar ether solvents such as diethyl ether, cyclopentyl methyl ether, tert-butyl methyl ether, and the like. Only one kind of the non-polar solvent may be used, or two or more kinds thereof may be used in combination. The non-polar solvent is preferably at least one selected from the group consisting of halogenated solvents, aromatic solvents, ester solvents, and aliphatic solvents, more preferably halogenated solvents and toluene, further preferably at least one selected from the group consisting of chloroform, dichloromethane, and toluene, particularly preferably dichloromethane.

The solution containing a non-polar solvent may further containing an aprotic polar solvent. As the aprotic polar solvent, nitrile solvents such as acetonitrile, propionitrile, and the like; polar ether solvents such as tetrahydrofuran and the like; pyridine, and the like can be mentioned. Among these, nitrile solvents are preferred, and acetonitrile and pyridine are more preferred. The amount of the aprotic polar solvent to be used is preferably 10 to 100 mL, more preferably 10 to 50 mL, per 100 mL of the non-polar solvent.

### 2-3. One-pot synthesis

The production method of the present invention can also be performed in an one-pot synthesis.

The one-pot synthesis includes
step (1) in which nucleoside, nucleotide, or oligonucleotide (a), each having a hydrophobic protecting group, and nucleoside, nucleotide, or oligonucleotide (b), each of which is phosphoramidited and in which a hydroxy group is protected by a temporary protecting group removable under acidic conditions are condensed in a solution containing a non-polar solvent to form oligonucleotide precursor (c) having a phosphite ester bond and the hydrophobic protecting group, in which the hydroxy group is protected by the temporary protecting group removable under acidic conditions,
step (2) in which a quencher (i) for the aforementioned phosphoramidited nucleoside, nucleotide, or oligonucleotide (b) was added to the solution after step (1) to quench the aforementioned phosphoramidited nucleoside, nucleotide, or oligonucleotide (b),
step (3) in which an oxidant is added to the solution after step (2) to oxidize the aforementioned oligonucleotide precursor (c) to form oligonucleotide (d) having a phosphate ester bond and the hydrophobic protecting group, in which the hydroxy group is protected by the temporary protecting group removable under acidic conditions,
step (4) in which a quencher (ii) for the oxidant is added to the solution after step (3) to quench the oxidant,
step (5) in which an acid is added to the solution after step (4) to remove the temporary protecting group removable under acidic conditions from the aforementioned oligonucleotide (d) to form ligonucleotide (e) having an unprotected hydroxy group and the hydrophobic protecting group,
step (6) in which a base is added to the solution after step (5) as necessary, and
step (7) in which a polar solvent is added to the solution containing the aforementioned oligonucleotide (e) to allow precipitation of the aforementioned oligonucleotide (e), and is characterized in that the aforementioned oxidant is compound (I) excluding 2,2-dipyridyl disulfide. As used herein, the production method of the present invention including the one-pot synthesis is characterized in that the intermediates (i.e., oligonucleotide precursor (c) obtained in step (1) and oligonucleotide (d) obtained in step (3)) are not isolated. Each step of the one-pot synthesis is described in order below.

### (Step (1) (condensation))

In step (1), nucleoside, nucleotide, or oligonucleotide (a), each having a hydrophobic protecting group, and nucleoside, nucleotide, or oligonucleotide (b), each of which is phosphoramidited and in which a hydroxy group is protected by a temporary protecting group removable under acidic conditions are condensed in a solution containing a non-polar solvent to form oligonucleotide precursor (c) having a phosphite ester bond and the hydrophobic protecting group, in which the hydroxy group is protected by the temporary protecting group removable under acidic conditions.

The hydrophobic protecting group of the aforementioned nucleoside, nucleotide, or oligonucleotide (a) is the aforementioned protecting group (Pg-5). The protecting group (Pg-5) is as described above.

The temporary hydroxy-protecting group of the aforementioned phosphoramidited nucleoside, nucleotide, or oligonucleotide (b) is preferably a 4,4'-dimethoxytrityl group or a 4-monomethoxytrityl group, more preferably a 4,4'-dimethoxytrityl group, from the aspects of easy deprotection and the like.

The combination of the aforementioned nucleoside, nucleotide, or oligonucleotide (a) and the aforementioned phosphoramidited nucleoside, nucleotide, or oligonucleotide (b) used in step (1) is preferably a combination of the aforementioned compound (a-I) and the aforementioned compound (b-I), or a combination of the aforementioned compound (a-I') and the aforementioned compound (bI'), more preferably a combination of the aforementioned compound (a-I) in which Pg is **L-Y-Z and the aforementioned compound (b-I), or a combination of aforementioned compound (a-I') in which Pg is **L-Y-Z and the aforementioned compound (b-I').

The step (1) is performed in a solution containing a non-polar solvent. Examples of the non-polar solvent include halogenated solvents such as chloroform, dichloromethane, 1,2-dichloroethane, and the like; aromatic solvents such as benzene, toluene, xylene, mesitylene, and the like; ester solvents such as ethyl acetate, isopropyl acetate, and the like; aliphatic solvents such as hexane, pentane, heptane, octane, nonane, cyclohexane, and the like; and non-polar ether solvents such as diethyl ether, cyclopentyl methyl ether, tert-butyl methyl ether, and the like. Only one kind of non-polar solvent may be used, or two or more kinds thereof may be used in combination. The non-polar solvent is preferably at least one selected from the group consisting of halogenated solvents, aromatic solvents, ester solvents, and aliphatic solvents, more preferably at least one selected from the group consisting of halogenated solvents and toluene, further preferably at least one selected from the group consisting of chloroform, dichloromethane, and toluene, particularly preferably dichloromethane and/or toluene. The steps after step (1) are also similarly performed in the solution containing a non-polar solvent.

The solution containing a non-polar solvent may further containing an aprotic polar solvent. As the aprotic polar solvent, nitrile solvents such as acetonitrile, propionitrile, and the like; polar ether solvents such as tetrahydrofuran and the like; and the like can be mentioned. Among these, nitrile solvents are preferred, and acetonitrile is more preferred. The amount of the aprotic polar solvent to be used is preferably 10 to 100 mL, more preferably 10 to 50 mL, per 100 mL of the non-polar solvent.

The amount of the aforementioned phosphoramidited nucleoside, nucleotide, or oligonucleotide (b) to be used is, for example, 1 to 10 mol, preferably 1 to 5 mol, per 1 mol of the amount of the aforementioned nucleoside, nucleotide, or oligonucleotide (a) to be used. The concentration of the aforementioned nucleoside, nucleotide, or oligonucleotide (a) in the solution is preferably 1 to 30 wt%.

The reaction temperature in step (1) is not particularly limited as long as the condensation proceeds, and is, for example, 0 to 100°C, preferably 20 to 50°C. The reaction time varies depending on the kind of the material to be used, reaction temperature, and the like and is, for example, 5 min to 24 hr.

In order to promote condensation, a known activator may be used. Examples of the activator include pyridine·trifluoroacetate, tetrazole, 5-ethylthio-1H-tetrazole, 5-benzylthio-1H-tetrazole, 4,5-dicyanoimidazole, and the like. Only one kind of the activator may be used, or two or more kinds thereof may be used in combination. When an activator is used, the amount thereof to be used is preferably 0.5 to 10 mol, more preferably 1 to 5 mol, per 1 mol of the aforementioned phosphoramidited nucleoside, nucleotide, or oligonucleotide (b).

### (Step (2) (Quench of phosphoramidited nucleoside, nucleotide, or oligonucleotide (b)))

In step (2), quencher (i) for the aforementioned phosphoramidited nucleoside, nucleotide, or oligonucleotide (b) is added to the solution after step (1) to quench the aforementioned phosphoramidited nucleoside, nucleotide, or oligonucleotide (b).

Only one kind of quencher (i) may be used, or two or more kinds thereof may be used in combination. Examples of quencher (i) include water, alcohols, phenols, and amines.

Examples of the alcohols usable as quencher (i) include optionally halogenated monovalent alcohols such as methanol, 2-propanol, t-butanol, 2,2,2,-trifluoroethanol, tetrahydrofurfuryl alcohol, furfurylalcohol, 2,3-O-isopropylidene-D-ribofuranose, 3'-O-triisopropylsilyl-thymidine, and the like, optionally halogenated polyhydric alcohols such as ethylene glycol, diethylene glycol, and the like.

Examples of the phenols usable as quencher (i) include 4-nitrophenol and pentafluorophenol. Examples of the amines usable as quencher (i) include morpholine.

The amount of quencher (i) is preferably 1 to 20 mol, more preferably 1 to 10 mol, further preferably 1 to 5 mol, per 1 mol of the amount of the phosphoramidited nucleoside, nucleotide, or oligonucleotide (b) to be used in step (1).

The quencher (i) is preferably water. Using water as quencher (i), oxidation of subsequent step (3) can be promoted. When water is used as quencher (i), the amount thereof to be used is preferably 1 to 20 mol, more preferably 2 to 15 mol, further preferably 2 to 10 mol, per 1 mol of the amount of the phosphoramidited nucleoside, nucleotide, or oligonucleotide (b) to be used in step (1).

The temperature of the solution after addition of quencher (i) is not particularly limited as long as the phosphoramidited nucleoside, nucleotide, or oligonucleotide (b) can be quenched, and is preferably 5 to 40°C, preferably 15 to 30°C. The stirring time of the solution after addition of quencher (i) varies depending on the kind of quencher (i) to be used, temperature and the like, and is, for example, 10 min to 3 hr.

### (Step (3) (Oxidation))

Step (3) is characterized by conversion of the phosphite ester bond of the aforementioned oligonucleotide precursor (c) into a phosphate ester bond by oxidation using compound (I) excluding 2,2-dipyridyl disulfide as an oxidant. To be specific, in step (3), an oxidant (i.e., compound (I)) is added to the solution after step (2) to oxidize the aforementioned oligonucleotide precursor (c) to form oligonucleotide (d) having a phosphate ester bond and the hydrophobic protecting group, in which the hydroxy group is protected by the temporary protecting group removable under acidic conditions. The oxidant and oxidation are as described above.

In the production method of the present invention, production of a desulfurized byproduct can be suppressed. Therefore, the aforementioned oligonucleotide precursor (c) is preferably an oligonucleotide precursor having a thiophosphate ester bond in addition to a phosphite ester bond.

### (Addition of aromatic amine)

It is preferable to add an aromatic amine to the solution after step (3). Only one kind of the aromatic amine may be used, or two or more kinds thereof may be used in combination. Addition of the aromatic amine enables further suppression of the production of defective byproduct. The aromatic amine may be added after step (3) and before addition of a quencher in step (4). A quencher (ii) and the aromatic amine may be simultaneously added in step (4), or quencher (ii) and the aromatic amine may be successively added in step (4).

Examples of the aromatic amine include aniline, 2-chloroaniline, 3-chloroaniline, 2,4-dichloroaniline, 2-fluoroaniline, 4-methoxyaniline, 4-nitroaniline, 2,6-dichloroaniline, 2,6-xylidine, and the like.

The aromatic amine is preferably at least one selected from a county of aniline, 2-chloroaniline, 3-chloroaniline, 2,4-dichloroaniline, 2-fluoroaniline, 4-methoxyaniline, 4-nitroaniline, 2,6-dichloroaniline, and 2,6-xylidine, more preferably 2-chloroaniline and/or 2,6-xylidine, further preferably 2,6-xylidine (i.e., 2,6-dimethylaniline).

From the aspect of suppression of defective byproducts, the amount of the aromatic amine to be used is preferably 1 to 20 mol, more preferably 1 to 10 mol, further preferably 1 to 5 mol, per 1 mol of the oxidant used in step (3).

### (Step (4) (Quench of oxidant))

In step (4), quencher (ii) for the oxidant is added to the solution after step (3) to quench the oxidant. Only one kind of quencher (ii) may be used, or two or more kinds thereof may be used in combination.

The quencher (ii) is preferably an organic phosphorus compound. Only one kind of the organic phosphorus compound may be used, or two or more kinds thereof may be used in combination.

The organic phosphorus compound is preferably at least one selected from the group consisting of a phosphine, a phosphorous acid triester, a phosphinite ester, a phosphonous acid diester, and a phosphinate ester, more preferably a phosphine. Only one kind of the aforementioned phosphine and the like may be used, or two or more kinds thereof may be used in combination.

Phosphine is a compound represented by formula: P(R)₃ wherein three Rs are each independently a hydrogen atom, an alkyl group, or an aryl group. Examples of the phosphine include triphenylphosphine, methyldiphenylphosphine, and the like.

Phosphorous acid triester is a compound represented by, formula: P(OR')₃ wherein three Rs are each independently an alkyl group or an aryl group. Examples of the phosphorous acid triester include triethyl phosphite and the like.

Phosphinite ester is a compound represented by formula: P(OR') (R)₂ wherein two Rs are each independently a hydrogen atom, an alkyl group, or an aryl group, and R' is an alkyl group or an aryl group. Examples of the phosphinite ester include ethoxydiphenylphosphine and the like.

Phosphonous acid diester is a compound represented by formula: P(R) (OR')₂ wherein R is a hydrogen atom, an alkyl group, or an aryl group, and two R's are each independently an alkyl group or an aryl group. Examples of the phosphonous acid diester include diethoxyphenylphosphine and the like.

Phosphinate ester is a compound represented by formula: P(=O) (R)₂(OR') wherein two Rs are each independently a hydrogen atom, an alkyl group or an aryl group, R' is an alkyl group or an aryl group, and one of R and R' may form, together with a phosphorus atom and an oxygen atom bonded thereto, a heterocycle. Examples of the phosphinate ester include 9,10-dihydro-9-oxa-10-phosphaphenanthrene 10-oxide represented by the following formula, and the like.

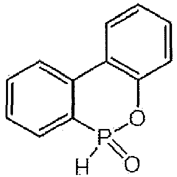

In one embodiment of the production method of the present invention, quencher (ii) is preferably at least one selected from the group consisting of triphenylphosphine, methyldiphenylphosphine, triethyl phosphite, ethoxydiphenylphosphine, diethoxyphenylphosphine, and 9,10-dihydro-9-oxa-10-phosphaphenanthrene 10-oxide, more preferably triphenylphosphine and/or methyldiphenylphosphine, further preferably triphenylphosphine.

The amount of the quencher (ii) to be used is preferably 1 to 10 mol, more preferably 1 to 5 mol, per 1 mol of the surplus of the oxidant in step (3) (i.e., "amount of oxidant used in step (3)" - "amount of phosphoramidated nucleoside, nucleotide or oligonucleotide (b) used in step (1)"), as long as it can quench the surplus of the oxidant.

The temperature of the solution after addition of quencher (ii) is not particularly limited as long as the oxidant can be quenched, and is preferably 0°C to 50°C, more preferably 10°C to 40°C. The stirring time of the solution after addition of quencher (i) varies depending on the kind of quencher (ii) to be used, temperature and the like, and is, for example, 5 min to 5 hr, more preferably 5 min to 2 hr.

### (Step (5) (Removal of temporary protecting group))

In step (5), an acid is added to the solution after step (4) to remove the temporary protecting group removable under acidic conditions from the aforementioned oligonucleotide (d) to form ligonucleotide (e) having an unprotected hydroxy group and the hydrophobic protecting group. Only one kind of acid may be used, or two or more kinds thereof may be used in combination.

The acid is not particularly limited as long as the temporary protecting group can be removed finely. Examples thereof include trifluoroacetic acid, dichloroacetic acid, trifluoromethanesulfonic acid, trichloroacetic acid, methanesulfonic acid, hydrochloric acid, acetic acid, p-toluene sulfonic acid, and the like. From the aspect of good removal of the temporary protecting group, trifluoroacetic acid, dichloroacetic acid, trifluoromethanesulfonic acid, and trichloroacetic acid are more preferred, trifluoroacetic acid, dichloroacetic acid, and trifluoromethanesulfonic acid are further preferred, trifluoroacetic acid and trifluoromethanesulfonic acid are still further preferred, and trifluoroacetic acid is particularly preferred.

The amount of the acid to be used is, for example 1 to 100 mol, preferably 1 to 40 mol, per 1 mol of the oligonucleotide (d).

The reaction temperature in step (5) is not particularly limited as long as the reaction proceeds, and is, for example, - 10°C to 50°C, preferably 0°C to 40°C. The reaction time varies depending on the oligonucleotide (d) used, the kind of acid, the kind of non-polar solvent, reaction temperature, and the like, and it is, for example 5 min to 5 hr.

A cation scavenger is preferably added to the solution before, during, or after removal of the temporary protecting group of oligonucleotide (d). That is, the temporary protecting group is preferably removed in the presence of a cation scavenger or a cation scavenger is preferably added to the reaction solution after the removal of the temporary protecting group. Only one kind of the cation scavenger may be used, or two or more kinds thereof may be used in combination.

The cation scavenger is not particularly limited as long as re-protection by a temporary protecting group removed or a side reaction with the deprotected functional group does not proceed. Pyrrole derivatives such as pyrrole, 2-methylpyrrole, 3-methylpyrrole, 2,3-dimethylpyrrole, 2,4-dimethylpyrrole and the like; indole derivatives such as indole, 3-methylindole, 4-methylindole, 5-methylindole, 6-methylindole, 7-methylindole, 5,6-dimethylindole, 6,7-dimethylindole, 5-methoxyindole, and the like; furan derivatives such as 2-methylfuran, 2,3-dimethylfuran, 2-methyl-3-(methylthio)furan, menthofuran, and the like can be used. The amount of the cation scavenger to be used is preferably 1 to 50 mol, more preferably 5 to 20 mol per 1 mol of the aforementioned oligonucleotide (d).

### (Step (6) (Neutralization))

In order to neutralize the acid used in step (5), the aforementioned one-pot synthesis may further include, after step (5) and before step (7), step (6) of adding a base to the solution after step (5). However, step (7) (solid-liquid separation) and washing are performed, whereby the acid used in step (5) can be removed from the oligonucleotide (e). Therefore, step (6) (neutralization) is not essential.

Only one kind of base may be used, or two or more kinds thereof may be used in combination. As the base to be used, an organic base is preferred. Examples of the organic base include pyridine, 2,4,6-trimethylpyridine, benzimidazole, 1,2,4-triazole, N-phenylimidazole, 2-amino-4,6-dimethylpyrimidine, 1,10-phenanthrolin, imidazole, N-methylimidazole, 2-chlorobenzimidazole, 2-bromobenzimidazole, 2-methylimidazole, 2-phenylbenzimidazole, N-phenylbenzimidazole, 5-nitrobenzimidazolep, and the like. Among them, pyridine, 2,4,6-trimethylpyridine, benzimidazole, 1,2,4-triazole, N-phenylimidazole, N-methylimidazole, 2-amino-4,6-dimethylpyrimidine, and 1,10-phenanthrolin are more preferable, pyridine, 2,4,6-trimethylpyridine, benzimidazole, 1,2,4-triazole, and N-phenylimidazole are more preferred, pyridine, 2,4,6-trimethylpyridine, benzimidazole, and 1,2,4-triazole are further preferred, and pyridine, 2,4,6-trimethylpyridine, and benzimidazole are particularly preferred.

The amount of the base to be used in step (6) is preferably 1 to 10 mol, more preferably 1 to 3 mol, per 1 mol of the amount of the acid used in step (5).

### (Step (7) (Solid-liquid separation))

In step (7), a polar solvent is added to the solution containing the aforementioned oligonucleotide (e) (i.e., solution after step (5), or solution after step (6) where necessary) to allow precipitation of the aforementioned oligonucleotide (e).

Examples of the polar solvent to be used in step (7) include alcohol solvents such as methanol, ethanol, isopropanol, and the like; nitrile solvents such as acetonitrile, propionitrile, and the like; ketone solvents such as acetone, 2-butanone, and the like; polar ether solvents such as 1,4-dioxane, tetrahydrofuran, and the like; amide solvents such as dimethylformamide, dimethylacetamide, N-methylpiperidone, and the like, sulfoxide solvents such as dimethyl sulfoxide and the like; water, and the like. Only one kind of the polar solvent may be used, or two or more kinds thereof may be used in combination. Of these, nitrile solvents are preferred, and acetonitrile is more preferred.

To increase the collection rate of the aforementioned oligonucleotide (e), the amount of the polar solvent to be added in the solid-liquid separation is preferably 1 to 20 mL, more preferably 5 to 20 mL, further preferably 5 to 10 mL, per 1 mL of the non-polar solvent contained in the solution.

To increase collection rate of the aforementioned oligonucleotide (e), a precipitation promoter (e.g., 3,4,5-tris(octadecyloxy)benzyl pivalate) described in WO 2016/117663 may also be used.

The aforementioned precipitated oligonucleotide (e) can be recovered by a known means such as filtration and the like.

The oligonucleotide chain can be elongated by repeating the one-pot synthesis containing steps (1) to (5) and (7) (preferably steps (1) to (7)). A production method of oligonucleotide, including repeating such one-pot synthesis is also encompassed in the production method of the present invention.

### (Deprotection)

The production method of the present invention may include, after step (7) (solid-liquid separation), a step of removing the protecting group (e.g., phosphate-protecting group, hydrophobic protecting group) of the obtained oligonucleotide (e) by a known method. For example, a 2-cyanoethyl group that is a phosphate-protecting group, the aforementioned protecting group (Pg-5), and the like can be removed by treating with aqueous ammonia, an aqueous ammonia/ethanol solution, or a mixture of aqueous ammonia and methylamine aqueous solution.

### 3. Novel compound

The present invention also provides a compound represented by the formula (In): 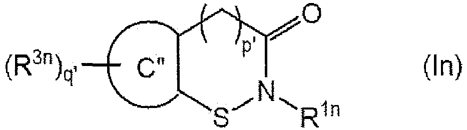 wherein
(1) R¹ⁿ is an optionally substituted phenyl group, p' is 0, ring C" is a 10-membered bicyclic fused aromatic heterocycle containing a nitrogen atom, and q' is 0, or
(2) R¹ⁿ is an optionally substituted phenyl group, p' is 0, ring C" is a 6-membered nitrogen-containing aromatic heterocycle, q' is 1, and R³ⁿ is a C₁₋₆ perfluoroalkyl group, or
(3) R¹ⁿ is an optionally substituted phenyl group, p' is 1, ring C" is a 6-membered nitrogen-containing aromatic heterocycle, and q' is 0, or
(4) R¹ⁿ is a pyridyl group substituted by one C₁₋₆ alkyl group, p' is 0, ring C" is a 6-membered nitrogen-containing aromatic heterocycle, and q' is 0, which is useful for the production method of oligonucleotide.

The compound (In) is useful as an oxidant for oxidizing an oligonucleotide precursor having a phosphite ester bond or a phosphonate ester bond (particularly, an oligonucleotide precursor having a phosphite ester bond) in the production method of oligonucleotide. Only one kind of compound (In) may be used, or two or more kinds thereof may be used in combination.

In the aforementioned formula (In), "p' is 0" means that - CH₂- is not present in the parentheses in the formula (In).

In the aforementioned formula (In), "q' is 0" means that R³ⁿ is not present in the formula (In).

The "optionally substituted phenyl group" for R¹ⁿ is preferably a phenyl group.

The "pyridyl group substituted by one C₁₋₆ alkyl group" for R¹ⁿ is preferably a pyridyl group substituted by one methyl group, more preferably a 4-methyl-2-pyridyl group.

The "10-membered bicyclic fused aromatic heterocycle containing a nitrogen atom" for ring C" is preferably a quinoline ring.

The "6-membered nitrogen-containing aromatic heterocycle" for ring C" is preferably a pyridine ring.

The "C₁₋₆ perfluoroalkyl group" for R³ⁿ is preferably a trifluoromethyl group.

The compound (In) is preferably 2-phenylisothiazolo[5,4-b]quinolin-3(2H)-one (compound (Ic-11)), 2-phenyl-5-trifluoromethylisothiazolo[5,4-b]pyridin-3(2H)-one (compound (Ic-12)), 2-phenyl-2H-1,2-benzothiazin-3(4H)-one (compound (Ic-13)), or 2-(4-methyl-2-pyridinyl)isothiazolo[5,4-b]pyridin-3(2H)-one (compound (Ic-15)).

The compound (In) can be produced by the aforementioned "production method of compound (Ic) wherein Y^{1c} is carbonyl", the below-mentioned Synthetic Example, a known method (e.g., the method described in Supporting Information of Non Patent Literature 2) or a method analogous thereto.

### [Example]

The present invention is explained in more detail in the following by referring to Examples and the like, which do not limit the present invention. It is also possible to carry out the present invention by making appropriate modifications within the range that can conform to the above and the following gist, all of which are encompassed in the technical scope of the present invention.

The "room temperature" described in the following Examples and the like means "20°C to 30°C".

In addition, the meanings of the abbreviations used in the following Examples and the like are as described below.

DMF: dimethylformamide
THF: tetrahydrofuran
SUC: succinyl
TOB: 3,4,5-tris(octadecyloxy)benzyloxy
Piv-TOB: 3,4,5-tris(octadecyloxy)benzyl pivalate
DDTT: [(N,N-dimethylaminomethylidene)amino]-3H-1,2,4-di thiazoline-3-thione
POS: 5-phenyl-3H-1,2,4-dithiazol-3-one
DMTr: 4,4'-dimethoxytrityl

### (Phosphoramidite or H-phosphonate)

2'-OMe-C-CE phosphoramidite: 5'-O-(4,4'-dimethoxytrityl)-N⁴-benzoyl-2'-O-methylcytidine-3'-[O-(2-cyanoethyl)-(N,N-diisopropyl)]-phosphoramidite
dT-CE phosphoramidite: 5'-O-(4,4'-dimethoxytrityl)-deoxythymidine-3'-[O-(2-cyanoethyl)-(N,N-diisopropyl)]-phosphoramidite
dC-CE phosphoramidite: 5'-O-(4,4'-dimethoxytrityl)-N⁴-benzoyl-deoxycytidine-3'-[O-(2-cyanoethyl)-(N,N-diisopropyl)]-phosphoramidite
5-Me-dC(Bz)-CE phosphoramidite: 5'-O-(4,4'-dimethoxytrityl)-N⁴-benzoyl-2'-deoxy-5-methylcytidine-3'-[O-(2-cyanoethyl)-(N,N-diisopropyl)]-phosphoramidite
dA-CE phosphoramidite: 5'-O-(4,4'-dimethoxytrityl)-N⁶-[1-(dimethylamino)ethylidene]-deoxyadenosine-3'-[O-(2-cyanoethyl)-(N,N-diisopropyl)]-phosphoramidite
dG-CE phosphoramidite: 5'-O-(4,4'-dimethoxytrityl)-N²-isobutyryl-2'-O-methylguanosine-3'-[O-(2-cyanoethyl)-(N,N-diisopropyl)]-phosphoramidite
2'-F-A-CE phosphoramidite: 5'-O-(4,4'-dimethoxytrityl)-N⁴-benzoyl-2'-fluoro-deoxyadenosine-3'-[O-(2-cyanoethyl)-(N,N-diisopropyl)]-phosphoramidite
dT-H-phosphonate TEA salt: 5'-O-(4,4'-dimethoxytrityl)-deoxythymidine-3'-H-phosphonate triethylamine salt

### (Oligonucleotide)

HO-T-SUC-TOB: deoxythymidine-3'-yl 3,4,5-tris(octadecyloxy)benzyl succinate
HO-T(S)T-SUC-TOB: deoxythymidine-3'-[O-(2-cyanoethyl)]phosphorothionyl deoxythymidine-3'-yl-[3,4,5-tris(octadecyloxy)benzyl] succinate
HO-Am(S)Cm-SUC-TOB: N⁶-benzoyl-2'-O-methyladenosine-3'-[O-(2-cyanoethyl)]phosphorothionyl N⁴-benzoyl-2'-O-methylcytidine-3'-yl-[3,4,5-tris(octadecyloxy)benzyl] succinate
HO-Cm(O)Am(S)Cm-SUC-TOB: N⁴-benzoyl-2'-O-methylcytidine-3'-[O-(2-cyanoethyl)]phosphoryl N⁶-benzoyl-2'-O-methyladenosine-3'-[O-(2-cyanoethyl)]phosphorothionyl N⁴-benzoyl-2'-O-methylcytidine-3'-yl-[3,4,5-tris(octadecyloxy)benzyl] succinate
HO-T(O)T-SUC-TOB: deoxythymidine-3'-[O-(2-cyanoethyl)]phosphoryl deoxythymidine-3'-yl-[3,4,5-tris(octadecyloxy)benzyl] succinate
HO-Cm(O)T(O)T-SUC-TOB: N⁴-benzoyl-2'-O-methylcytidine 3'-[O-(2-cyanoethyl)]phosphoryl deoxythymidine-3'-[O-(2-cyanoethyl)]phosphoryl deoxythymidine-3'-yl-[3,4,5-tris(octadecyloxy)benzyl] succinate
HO-Af(O)Cm(O)T(O)T-SUC-TOB: N⁶-benzoyl-2'-fluoro-deoxyadenosine-3'-[O-(2-cyanoethyl)]phosphoryl N⁴-benzoyl-2'-O-methylcytidine 3'-[O-(2-cyanoethyl)]phosphoryl deoxythymidine-3'-[O-(2-cyanoethyl)]phosphoryl deoxythymidine-3'-yl-[3,4,5-tris(octadecyloxy)benzyl] succinate
DMTrO-Af(O)Cm(O)T(O)T-SUC-TOB: 5'-O-(4,4'-dimethoxytrityl)-N⁶-benzoyl-2'-fluoro-deoxyadenosine-3'-[O-(2-cyanoethyl)]phosphoryl N⁴-benzoyl-2'-O-methylcytidine 3'-[O-(2-cyanoethyl)]phosphoryl deoxythymidine-3'-[O-(2-cyanoethyl)]phosphoryl deoxythymidine-3'-yl-[3,4,5-tris(octadecyloxy)benzyl] succinate
DMTrO-Af(O)Cm(O)T(O)T-OH: 5'-O-(4,4'-dimethoxytrityl)-2'-fluoro-deoxyadenosine-3'-phosphoryl-2'-O-methylcytidine-3'-phosphoryl-deoxythymidine-3'-phosphoryl-deoxythymidine
DMTrO-Af(O)Cm(O)T-OH: 5'-O-(4,4'-dimethoxytrityl)-2'-fluoro-deoxyadenosine-3'-phosphoryl-2'-O-methylcytidine-3'-phosphoryl-deoxythymidine
DMTrO-Af(O)T(O)T-OH: 5'-O-(4,4'-dimethoxytrityl)-2'-fluoro-deoxyadenosine-3'-phosphoryl-deoxythymidine-3'-phosphoryl-deoxythymidine
HO-A(S)C(S)T(S)T-SUC-TOB: N⁶-[1-(dimethylamino)ethylidene]-deoxyadenosine-3'-[O-(2-cyanoethyl)]phosphorothionyl N⁴-benzoyl-deoxycytidine 3'-[O-(2-cyanoethyl)]phosphorothionyl deoxythymidine-3'-[O-(2-cyanoethyl)]phosphorothionyl deoxythymidine-3'-yl-[3,4,5-tris(octadecyloxy)benzyl] succinate
DMTrO-G(III)A(S)C(S)T(S)T-SUC-TOB: 5'-O-(4,4'-dimethoxytrityl)-N²-isobutyryl-deoxyguanosine-3'-[O-(2-cyanoethyl)]phosphityl N⁶-[1-(dimethylamino)ethylidene]-deoxyadenosine-3'-[O-(2-cyanoethyl)]phosphorothionyl N⁴-benzoyl-deoxycytidine 3'-[O-(2-cyanoethyl)]phosphorothionyl deoxythymidine-3'-[O-(2-cyanoethyl)]phosphorothionyl deoxythymidine-3'-yl-[3,4,5-tris(octadecyloxy)benzyl] succinate
DMTrO-G(O)A(S)C(S)T(S)T-SUC-TOB: 5'-O-(4,4'-dimethoxytrityl)-N²-isobutyryl-deoxyguanosine-3'-[O-(2-cyanoethyl)]phosphoryl N⁶-[1-(dimethylamino)ethylidene]-deoxyadenosine-3'-[O-(2-cyanoethyl)]phosphorothionyl N⁴-benzoyl-deoxycytidine 3'-[O-(2-cyanoethyl)]phosphorothionyl deoxythymidine-3'-[O-(2-cyanoethyl)]phosphorothionyl deoxythymidine-3'-yl-[3,4,5-tris(octadecyloxy)benzyl] succinate
DMTrO-G(O)A(S)C(S)T(S)T-OH: 5'-O-(4,4'-dimethoxytrityl)-deoxyguanosine-3'-phosphoryl-deoxyadenosine-3'-phosphorothionyl-deoxycytidine-3'-phosphorothionyl-deoxythymidine-3'-phosphorothionyl-deoxythymidine
DMTrO-T(OH)T-SUC-TOB: 5'-O-(4,4'-dimethoxytrityl)-deoxythymidine-3'-phosphoryl deoxythymidine-3'-yl-[3,4,5-tris(octadecyloxy)benzyl] succinate
HO-G(O)T(S)T-SUC-TOB: N²-isobutyryl-deoxyguanosine-3'-[O-(2-cyanoethyl)]phosphoryl deoxythymidine-3'-[O-(2-cyanoethyl)]phosphorothionyl deoxythymidine-3'-yl-[3,4,5-tris(octadecyloxy)benzyl] succinate
HO-A(O)G(O)T(S)T-SUC-TOB: N⁶-[1-(dimethylamino)ethylidene]-deoxyadenosine-3'-[O-(2-cyanoethyl)]phosphoryl N²-isobutyryl-deoxyguanosine-3'-[O-(2-cyanoethyl)]phosphoryl deoxythymidine-3'-[O-(2-cyanoethyl)]phosphorothionyl deoxythymidine-3'-yl-[3,4,5-tris(octadecyloxy)benzyl] succinate
HO-^{Me}C(O)A(O)G(O)T(S)T-SDC-TOB: N⁴-benzoyl-2'-deoxy-5-methylcytidine-3'-[O-(2-cyanoethyl)]phosphoryl N⁶-[1-(dimethylamino)ethylidene]-deoxyadenosine-3'-[O-(2-cyanoethyl)]phosphoryl N²-isobutyryl-deoxyguanosine-3'-[O-(2-cyanoethyl)]phosphoryl deoxythymidine-3'-[O-(2-cyanoethyl)]phosphorothionyl deoxythymidine-3'-yl-[3,4,5-tris(octadecyloxy)benzyl] succinate
HO-T(S)T(S)T-SUC-TOB: deoxythymidine-3'-[O-(2-cyanoethyl)]phosphorothionyl deoxythymidine-3'-[O-(2-cyanoethyl)]phosphorothionyl deoxythymidine-3'-yl-[3,4,5-tris(octadecyloxy)benzyl] succinate
HO-T(S)T(S)T-OH: deoxythymidine-3'-[O-(2-cyanoethyl)]phosphorothionyl-deoxythymidine-3'-[O-(2-cyanoethyl)]phosphorothionyl-deoxythymidine
DMTrO-C(O)T(S)T-SUC-TOB: 5'-O-(4,4'-dimethoxytrityl)-N⁴-benzoyl-deoxycytidine 3'-[O-(2-cyanoethyl)]phosphoryl deoxythymidine-3'-[O-(2-cyanoethyl)]phosphorothionyl deoxythymidine-3'-yl-[3,4,5-tris(octadecyloxy)benzyl] succinate
DMTrO-C(S)T(S)T-SUC-TOB: 5'-O-(4,4'-dimethoxytrityl)-N⁴-benzoyl-deoxycytidine-3'-[O-(2-cyanoethyl)]phosphorothionyl deoxythymidine-3'-[O-(2-cyanoethyl)]phosphorothionyl deoxythymidine-3'-yl-[3,4,5-tris(octadecyloxy)benzyl] succinate
HO-^{Me}C-SUC-TOB: N⁴-benzoyl-5-methyl-2'-deoxycytidine-3'-yl-[3,4,5-tris(octadecyloxy)benzyl] succinate
HO-G(S)^{Me}C-SUC-TOB: N²-isobutyryl-deoxyguanosine-3'-[O-(2-cyanoethyl)]phosphorothionyl N⁴-benzoyl-5-methyl-2'-deoxycytidine-3'-yl-[3,4,5-tris(octadecyloxy)benzyl] succinate
HO-A(S)G(S)^{Me}C-SUC-TOB: N⁶-[1-(dimethylamino)ethylidene]-deoxyadenosine-3'-[O-(2-cyanoethyl)]phosphorothionyl N²-isobutyryl-deoxyguanosine-3'-[O-(2-cyanoethyl)]phosphorothionyl N⁴-benzoyl-5-methyl-2'-deoxycytidine-3'-yl-[3,4,5-tris(octadecyloxy)benzyl] succinate
DMTrO-G(O)A(S)G(S)^{Me}C-SUC-TOB: 5'-O-(4,4'-dimethoxytrityl)-N²-isobutyryl-deoxyguanosine-3'-[O-(2-cyanoethyl)]phosphoryl N⁶-[1-(dimethylamino)ethylidene]-deoxyadenosine-3'-[O-(2-cyanoethyl)]phosphorothionyl N²-isobutyryl-deoxyguanosine-3'-[O-(2-cyanoethyl)]phosphorothionyl N⁴-benzoyl-5-methyl-2'-deoxycytidine-3'-yl-[3,4,5-tris(octadecyloxy)benzyl] succinate
DMTrO-G(S)A(S)G(S)^{Me}C-SUC-TOB: 5'-O-(4,4'-dimethoxytrityl)-N²-isobutyryl-deoxyguanosine-3'-[O-(2-cyanoethyl)]phosphorothionyl N⁶-[1-(dimethylamino)ethylidene]-deoxyadenosine-3'-[O-(2-cyanoethyl)]phosphorothionyl N²-isobutyryl-deoxyguanosine-3'-[O-(2-cyanoethyl)]phosphorothionyl N⁴-benzoyl-5-methyl-2'-deoxycytidine-3'-yl-[3,4,5-tris(octadecyloxy)benzyl] succinate
DMTrO-G(O)A(S)G(S)^{Me}C-OH: 5'-O-(4,4'-dimethoxytrityl)-deoxyguanosine-3'-phosphoryl-deoxyadenosine-3'-phosphorothionyl-deoxyguanosine-3'-phosphorothionyl-5-methyl-2'-deoxycytidine

### (Oligonucleotide precursor)

DMTrO-G(III)A(S)C(S)T(S)T-SUC-TOB: 5'-O-(4,4'-dimethoxytrityl)-N²-isobutyryl-deoxyguanosine-3'-[O-(2-cyanoethyl)]phosphityl N⁶-[1-(dimethylamino)ethylidene]-deoxyadenosine-3'-[O-(2-cyanoethyl)]phosphorothionyl N⁴-benzoyl-deoxycytidine 3'-[O-(2-cyanoethyl)]phosphorothionyl deoxythymidine-3'-[O-(2-cyanoethyl)]phosphorothionyl deoxythymidine-3'-yl-[3,4,5-tris(octadecyloxy)benzyl] succinate
DMTrO-C(III)T(S)T-SUC-TOB: 5'-O-(4,4'-dimethoxytrityl)-N⁴-benzoyl-deoxycytidine-3'-[O-(2-cyanoethyl)]phosphityl deoxythymidine-3'-[O-(2-cyanoethyl)]phosphorothionyl deoxythymidine-3'-yl-[3,4,5-tris(octadecyloxy)benzyl] succinate
DMTrO-T(H)T-SUC-TOB: 5'-O-(4,4'-dimethoxytrityl)-deoxythymidine-3'-phosphonyl deoxythymidine-3'-yl-[3,4,5-tris(octadecyloxy)benzyl] succinate
DMTrO-G(III)A(S)G(S)^{Me}C-SUC-TOB: 5'-O-(4,4'-dimethoxytrityl)-N²-isobutyryl-deoxyguanosine-3'-[O-(2-cyanoethyl)]phosphityl N⁶-[1-(dimethylamino)ethylidene]-deoxyadenosine-3'-[O-(2-cyanoethyl)]phosphorothionyl N²-isobutyryl-deoxyguanosine-3'-[O-(2-cyanoethyl)]phosphorothionyl N⁴-benzoyl-5-methyl-2'-deoxycytidine-3'-yl-[3,4,5-tris(octadecyloxy)benzyl] succinate

### Synthetic Example 1: Synthesis of oligonucleotide precursor having phosphite ester bond

Under an argon atmosphere, HO-T-SUC-TOB (2.5 g, 2.0 mmol), Piv-TOB (5.0 g, 5.0 mmol), dehydrated dichloromethane (100 mL), and dehydrated acetonitrile (30 mL) were added to a 1000 mL flask to prepare a solution. Thereafter, dT-CE phosphoramidite (3.0 g, 4.0 mmol) and triphenylphosphine (270 mg, 1.0 mmol) were added, and the solution was stirred for 30 min. Then, 5-ethylthio-1H-tetrazole (530 mg, 4.0 mmol) was added, and the solution was stirred at room temperature for 45 min. Thereafter, 2,2,2-trifluoroethanol (1.5 mL, 20 mmol) was added to the solution, and the mixture was stirred at room temperature for 30 min. 2,6-Dimethylaniline (1.8 mL, 15 mmol) and POS (1.0 g, 5.1 mmol) were successively added, and the solution was stirred at room temperature for 1.0 hr. Thereafter, 2,3-dimethylfuran (2.1 mL, 20 mmol), trifluoroacetic acid (5.2 mL, 68 mmol), and 2,6-dimethylaniline (83.5 µL, 0.7 µmοl) were successively added, and the solution was stirred at room temperature for 55 min. Furthermore, trifluoroacetic acid (620 µL, 8.1 mmol) and 2,6-dimethylaniline (10 µL, 0.10 µmοl) were added, and the solution was stirred at room temperature for 15 min. Then, pyridine (19 mL, 230 mmol) and methanol (870 µL) were successively added to the solution, and the solution was stirred at room temperature for 115 min. Thereafter, acetonitrile (1 L) was added, and the precipitated solid was recovered by suction filtration using a Kiriyama funnel and then dried to give Piv-TOB and a dimer, oligonucleotide HO-T(S)T-SUC-TOB, as a mixture (8.1 g, yield 98%). m/z: Calcd. 1610.03, Found 1611.06 [M+H]⁺

In the same manner as above except that the solid (8.1 g) obtained above and dC-CE phosphoramidite instead of dT-CE phosphoramidite were used, Piv-TOB and a trimer, oligonucleotide HO-C(S)T(S)T-SUC-TO was obtained, as a mixture (8.7 g, yield 97%). m/z: Calcd. 2072.11, Found 2073.14 [M+H]⁺

In the same manner as above except that the solid (2.8 g) obtained above and dT-dA-CE phosphoramidite instead of dT-CE phosphoramidite were used, Piv-TOB and a tetramer, oligonucleotide HO-A(S)C(S)T(S)T-SUC-TOB was obtained, as a mixture (3.0 g, yield 98%).
m/z: Calcd. 2523.23, Found 1262.63 [M+2H]²⁺

Under an argon atmosphere, the solid (400 mg) obtained above, dehydrated dichloromethane (4.0 mL), and dehydrated acetonitrile (1.2 mL) were added to a 100 mL two-necked flask to prepare a solution. Thereafter, dG-CE phosphoramidite (136 mg, 161 pmol) and 5-ethylthio-1H-tetrazole (21.1 mg, 162 µmοl) were successively added, and the solution was stirred at room temperature for 60 min. Thereafter, acetonitrile (30 mL) was added, and the precipitated solid was recovered by suction filtration using a Kiriyama funnel, and dried to give Piv-TOB and a pentamer, oligonucleotide precursor DMTrO-G(III)A(S)C(S)T(S)T-SUC-TOB, as a mixture (453 mg, yield 99%).
m/z: Calcd. 3261.49, Found 1631.67 [M+2H]²⁺

### Example 1: Oxidation using compound (Ib-1)

Under an argon atmosphere, the solid (100.0 mg: 56.9 mg, 17.4 µmol as DMTrO-G(III)A(S)C(S)T(S)T-SUC-TOB) obtained in Synthetic Example 1, dehydrated dichloromethane (0.9 mL), and dehydrated acetonitrile (0.3 mL) were added to a 20 mL two-necked flask to prepare a solution. Thereafter, water (9 µL, 0.5 mmol) and compound (Ib-1) (12.1 mg, 36.4 µmοl) were successively added, and the solution was stirred at room temperature for 60 min. Thereafter, triphenylphosphine (4.6 mg, 18 µmol) was added, and the solution was stirred for 30 min. Then, acetonitrile (10 mL) was added, and the precipitated solid was recovered by suction filtration using a Kiriyama funnel and then dried to give Piv-TOB and pentamer, oligonucleotide DMTrO-G(O)A(S)C(S)T(S)T-SUC-TOB, as a mixture (82 mg, yield 82%).

As compound (Ib-1), a commercially available product was used.
m/z: Calcd. 3277.48, Found 1639.75 [M+2H]²⁺

### Example 2: Oxidation using compound (Ic-1)

Under an argon atmosphere, the solid (100 mg: 57 mg, 17 µmol as DMTrO-G(III)A(S)C(S)T(S)T-SUC-TOB) obtained in Synthetic Example 1, dehydrated dichloromethane (0.9 mL), and dehydrated acetonitrile (0.3 mL) were added to a 20 mL two-necked flask to prepare a solution. Thereafter, water (9 µL, 0.5 mmol) and compound (Ic-1) (8.0 mg, 35 µmοl) were successively added, and the solution was stirred at room temperature for 60 min. Thereafter, triphenylphosphine (4.7 mg, 18 µmοl) was added, and the solution was stirred for 30 min. Then, acetonitrile (10 mL) was added, and the precipitated solid was recovered by suction filtration using a Kiriyama funnel and then dried to give Piv-TOB and pentamer, oligonucleotide DMTrO-G(O)A(S)C(S)T(S)T-SUC-TOB, as a mixture (83 mg, yield 83%).

Compound (Ic-1) used was synthesized by the method described in the Supporting Information of Non Patent Literature 2.
m/z: Calcd. 3277.48, Found 1639.75 [M+2H]²⁺

### Comparative Example 1: Oxidation using iodine

Under an argon atmosphere, the solid (100 mg: 57 mg, 17 µmol as DMTrO-G(III)A(S)C(S)T(S)T-SUC-TOB) obtained in Synthetic Example 1, dehydrated dichloromethane (0.9 mL), and dehydrated acetonitrile (0.3 mL) were added to a 20 mL two-necked flask to prepare a solution. Thereafter, water (9 µL, 0.5 mmol), pyridine (5.7 µL, 71 µmol), and iodine (8.8 mg, 35 µmοl) were successively added, and the solution was stirred at room temperature for 60 min. Thereafter, triphenylphosphine (4.8 mg, 18 µmol) was added, and the solution was stirred for 30 min. Then, acetonitrile (10 mL) was added, and the precipitated solid was recovered by suction filtration using a Kiriyama funnel and then dried to give Piv-TOB and pentamer, oligonucleotide DMTrO-G(O)A(S)C(S)T(S)T-SUC-TOB, as a mixture (83 mg, yield 83%).
m/z: Calcd. 3277.48, Found 1639.75 [M+2H]²⁺

### Comparison of Example 1, Example 2, and Comparative Example 1

As described below, an oligonucleotide having a thiophosphate ester bond, and a byproduct obtained by conversion of the thiophosphate ester bond to a phosphate ester bond (hereinafter referred to as "desulfurized byproduct") were analyzed, and the proportion of the desulfurized byproduct was calculated.

The solid (10 mg) obtained in Example 1, Example 2, or Comparative Example 1 and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure by a centrifugation evaporator to give an oligonucleotide having a thiophosphate ester bond, DMTrO-G(O)A(S)C(S)T(S)T-OH (hereinafter referred to as "desired oligonucleotide").
m/z: Calcd. 1827.36, Found 1826.34 [M-H]⁻

The obtained mixture containing the desired oligonucleotide and desulfurized byproduct was analyzed by liquid chromatography-mass spectrometry (LC-MS). Using the extraction ion chromatogram (EIC) of the observed each compound (desired oligonucleotide and desulfurized byproduct), the peak area of each compound was calculated, and the proportion (%) of the desulfurized byproduct was calculated by the following formula: $proportion \left(%\right) of desulfurized byproduct = \left(\begin{matrix}peak area of \\ desulfurized byproduct / peak area of desired oligonucleotide\end{matrix}\right) \times 100 .$ The results are shown in Table 1.

**[Table 1]**

| | oxidant used | proportion of desulfurized byproduct |
|---|---|---|
| Example 1 | 2,2'-dibenzothiazolyl disulfide (compound (Ib-1)) | 0.3% |
| Example 2 | 2-phenylisothiazolo[5,4-b]pyridin-3(2H)-one (compound (Ic-1)) | 0.4% |
| Comparative Example 1 | iodine | 4.5% |

As is clear from the results of Table 1, production of desulfurized byproducts can be suppressed by using compound (Ib-1) or compound (Ic-1) as an oxidant instead of iodine.

### Synthetic Example 2: Synthesis of oligonucleotide precursor having phosphonate ester bond

Under an argon atmosphere, HO-T-SUC-TOB (500 mg, 400 µmol), dT-H-phosphonate TEA salt (568 mg, 800 µmol), dehydrated dichloromethane (10 mL), and dehydrated pyridine (10 mL) were added to a 200 mL three-necked flask to prepare a solution. Then, pivaloyl chloride (0.2 mL, 1.6 mmol) was added, and the solution was stirred for 1 hr. Thereafter, acetonitrile (140 mL) was added, and the precipitated solid was recovered by suction filtration using a Kiriyama funnel and dried to give a dimer, oligonucleotide precursor DMTrO-T(H)T-SUC-TOB (727 mg, yield 98%).

### Example 3: Oxidation using compound (Ic-1)

Under an argon atmosphere, the oligonucleotide precursor (171 mg, 93 pmol) obtained in Synthetic Example 2, dichloromethane (4.7 mL), and acetonitrile (1.4 mL) were added to a 50 mL two-necked flask to prepare a solution. Then, water (60 µL), compound (Ic-1) (43 mg, 189 µmol), and diazabicycloundecene (DBU) (55 µL, 372 µmοl) were added, and the solution was stirred at room temperature for 2 hr. Thereafter, acetonitrile (42 mL) was added, and the precipitated solid was recovered by suction filtration using a Kiriyama funnel and then dried to quantitatively give a dimer, oligonucleotide DMTrO-T(OH)T-SUC-TOB.
m/z: Calcd. 1843.16, Found 1842.15 [M-H]⁻

### Example 4: Oxidation using compound (Ib-1)

Under an argon atmosphere, the oligonucleotide precursor (170 mg, 93 µmοl) obtained in Synthetic Example 2, dichloromethane (4.7 mL), and acetonitrile (1.4 mL) were added to a 50 mL two-necked flask to prepare a solution. Then, compound (Ib-1) (62 mg, 186 µmοl) and DBU (55 µL, 372 pmol) were added, and the solution was stirred at room temperature for 2 hr. Thereafter, acetonitrile (42 mL) was added, and the precipitated solid was recovered by suction filtration using a Kiriyama funnel and then dried to quantitatively give a dimer, oligonucleotide DMTrO-T(OH)T-SUC-TOB.
m/z: Calcd. 1843.16, Found 1842.15 [M-H]⁻

### Experimental Example 1

Non Patent Literature 1(ARKIVOC 2009 (iii) 264-273) discloses, as shown in the following formula, that H-phosphonate (compound 1) and nucleoside (compound 2) are condensed and oxidized in the presence of a large amount of triphenylphosphine and a large amount of 2,2'-dipyridyl disulfide to produce dinucleotide (compound 4).

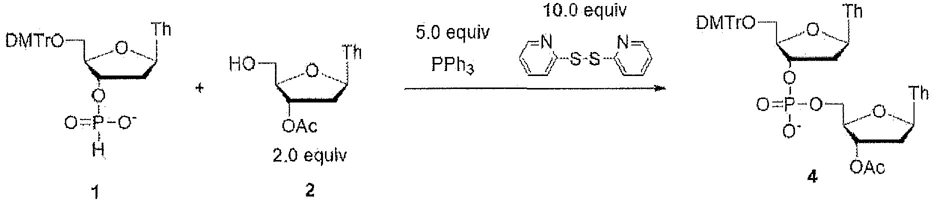

In addition, repeated extension in oligonucleotide synthesis may unintentionally remove the thiophosphate moiety-protecting group (e.g., 2-cyanoethyl group) of the oligonucleotide. Thus, an oligonucleotide from which the thiophosphate moiety-protecting group was removed was reacted with 2,2'-dipyridyl disulfide used in Non Patent Literature 1 or compound (Ib-1) used in the present invention, and 2,2'-dipyridyl disulfide and compound (Ib-1) were evaluated. In the following experiment, a large amount of 2,2'-dipyridyl disulfide or a large amount of compound (Ib-1) was used for a substrate (i.e., oligonucleotide from which the thiophosphate moiety-protecting group was removed) in accordance with the reaction conditions described in Non Patent Literature 1.

### (1) Experiment using 2,2'-dipyridyl disulfide and triphenylphosphine

A mixture of HO-T(S)T(S)T-SUC-TOB from which 2-cyanoethyl group was removed and Piv-TOB (40 mg: 20 mg, 0.012 mmol as HOT(S)T(S)T-SUC-TOB) were dissolved in dehydrated dichloromethane (0.3 mL) and dehydrated pyridine (0.3 mL) to prepare a solution. Triphenylphosphine (15 mg, 0.059 mmol) and 2,2'-dipyridyl disulfide (25 mg, 0.12 mmol) were added, and the solution was stirred at room temperature for 4.5 hr. Thereafter, acetonitrile (7 mL) was added to the solution, and the precipitated solid was recovered by suction filtration using a Kiriyama funnel, and dried under reduced pressure to give a solid (31.2 mg).

The obtained solid (10 mg) and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure by a centrifugation evaporator. The obtained concentrate was subjected to LC-MS analysis.

### (2) Experiment using compound (Ib-1) and water

A mixture of HO-T(S)T(S)T-SUC-TOB from which 2-cyanoethyl group was removed and Piv-TOB (40 mg:20 mg, 0.012 mmol as HOT(S)T(S)T-SUC-TOB) were dissolved in dehydrated dichloromethane (0.3 mL) and dehydrated pyridine (0.3 mL) to prepare a solution. Water (6 µL, 0.3 mmol) and compound (Ib-1) (39 mg, 0.12 mmol) were added, and the solution was stirred at room temperature for 4.5 hr. Thereafter, acetonitrile (7 mL) was added to the solution, and the precipitated solid was recovered by suction filtration using a Kiriyama funnel, and dried under reduced pressure to quantitatively give a solid.

The obtained solid (10 mg) and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure by a centrifugation evaporator. The obtained concentrate was subjected to LC-MS analysis.

### (3) Evaluation

The amount of an oligonucleotide having a thiophosphate ester bond, HO-T(S)T(S)T-OH (hereinafter referred to as "desired oligonucleotide"), and the amount of a byproduct formed by reaction of the desired oligonucleotide and an oxidant (2,2'-dipyridyl disulfide or compound (Ib-1)) (hereinafter referred to as "byproduct") were measured as follows, and the proportion of the byproduct was calculated. To be specific, the concentrate obtained by the above-mentioned experiment was analyzed by liquid chromatography-mass spectrometry (LC-MS). Using the extraction ion chromatogram (EIC) of the observed each compound (desired oligonucleotide and byproduct), the peak area of each compound was calculated, and the proportion of the byproduct was calculated by the following formula: $proportion \left(%\right) of byproduct = \left(\begin{matrix}peak area of byproduct / peak \\ area of desired oligonucleotide + peak area of byproduct\end{matrix}\right) \times 100 .$ The results are shown in Table 2.

**[Table 2]**

| | proportion of byproduct |
|---|---|
| (1) 2,2'-dipyridyl disulfide and triphenylphosphine | 81% |
| (2) 2,2'-dibenzothiazolyl disulfide (compound (Ib-1)) and water | not detected |

As shown in Table 2, a large amount of byproduct was produced by using 2,2'-dipyridyl disulfide and triphenylphosphine. On the other hand, byproduct was not detected even when large amounts of compound (Ib-1) and water were used. In the method described in Non Patent Literature 1 using 2,2'-dipyridyl disulfide and triphenylphosphine, a large amount of byproduct is produced, similar to the results shown in Experimental Example 1.

### Synthetic Example 3: Synthesis of 2-(2-benzothiazolyldithio)propanoic acid (compound (Ib-2))

Tetrahydrofuran (100 mL), 2-mercaptopropionic acid (10 mmol), and compound (Ib-1) (20 mmol) were added to a 200 mL eggplant flask, and the solution was stirred for 24 hr. Thereafter, the solution was filtered, the solvent of the filtrate was evaporated under reduced pressure, and the obtained concentrate was purified by silica gel column chromatography to give the desired product (1.6 g).
¹H-NMR (400 MHz, methanol-d₄) δ 7.91 (d, J = 8.0, 1H), 7.81 (d, J = 8.0, 1H), 7.47 (dd, J = 8.0, 1H), 7.38 (dd, J = 8.0, 1H), 3.89 (q, J = 7.1, 1H), 1.58 (d, J = 7.1, 3H)

### Synthetic Example 4: Synthesis of 3-(2-benzothiazolyldithio)propanoic acid (compound (Ib-3))

Tetrahydrofuran (100 mL), 3-mercaptopropionic acid (10 mmol), and compound (Ib-1) (20 mmol) were added to a 200 mL eggplant flask to prepare a solution, and the solution was stirred for 24 hr. Thereafter, the solution was filtered, the solvent of the filtrate was evaporated under reduced pressure, and the obtained concentrate was purified by silica gel column chromatography to give the desired product (1.3 g).
¹H-NMR (400 MHz, chloroform-d) δ 7.93 (d, J = 8.0, 1H), 7.81 (d, J = 8.0, 1H), 7.48 (dd, J = 8.0, 1H), 7.39 (dd, J = 8.0, 1H), 3.24 (t, J = 6.8, 2H), 2.81 (t, J = 6.8, 2H).

### Synthetic Example 5: Synthesis of 2,2'-dibenzoimidazolyl disulfide (compound (Ib-4))

Dichloromethane (150 mL), 2-mercaptobenzoimidazole (20 mmol), and triethylamine (22 mmol) were added to a 300 mL eggplant flask, then iodine (11 mmol) was added, and the solution was stirred for 3 hr. Thereafter, the solution was filtered, the solvent of the filtrate was evaporated under reduced pressure, and the obtained concentrate was recrystallized with diethyl ether and ethanol to give the desired product (2.5 g).
¹H-NMR (400 MHz, DMSO-d⁶) δ = 7.58-7.56 (m, 4H), 7.26-7.22 (m, 4H).

### Synthetic Example 6: Synthesis of 2,2'-dibenzoxazolyl disulfide (compound (Ib-5))

2-Mercaptobenzoxazole (1.5 g, 10 mmol) and ethyl acetate (18 mL) were added to a 30 mL eggplant flask to prepare a solution. Hydrogen peroxide (33 wt%, 489 µL) was added dropwise to the solution at 0°C over 60 min, and the solution was heated to room temperature and stirred for 2.5 hr. Water (1.4 mL) was added to the solution, and the mixture was stirred for 10 min. The organic layer was separated, and the aqueous layer was extracted twice with ethyl acetate (5 mL). The solution obtained by extraction was dried over sodium sulfate, and the solvent was removed under reduced pressure. The obtained solid was washed with ice-cooled diethyl ether and recrystallized with diethyl ether. The precipitated solid was recovered by filtration and dried under reduced pressure to give the desired product (210 mg).
¹H-NMR (400 MHz, Chloroform-d) δ = 7.76-7.69(m, 2H), 7.58-7.50 (m, 2H), 7.42-7.32 (m, 4H).

### Synthetic Example 7: Synthesis of 2,2'-dithiobis(pyridine-N-oxide) (compound (Ib-8))

2-Mercaptopyridine-N-oxide (3.8 g, 30 mmol) and water (27 mL) were added to a 100 mL eggplant flask to prepare a solution. Hydrogen peroxide (33 wt%, 3.2 mL) was added dropwise to the solution at 0°C over 60 min, and the solution was heated to 40°C and stirred for 1 hr. The solution was filtered to recover a solid, and the recovered solid was washed with methanol and dried under reduced pressure to give the desired product (1.2 g).
¹H-NMR (400 MHz, Chloroform-d) δ = 8.31 (d, J = 5.2, 2H), 7.62 (d, J = 6.4, 2H), 7.34-7.24 (m, 2H), 7.20 (dd, J = 6.4, 2H).

### Synthetic Example 8: Synthesis of oligonucleotide precursor having phosphite ester bond

Under an argon atmosphere, HO-T-SUC-TOB (2.1 g, 1.7 mmol), dehydrated dichloromethane (87 mL), and dehydrated acetonitrile (26 mL) were added to a 200 mL two-necked flask to prepare a solution. Thereafter, molecular sieve 3A (1.7 g), triphenylphosphine (227 mg, 867 µmol), dT-CE phosphoramidite (2.6 g, 3.5 mmol), and 5-ethylthio-1H-tetrazole (452 mg, 3.47 mmol) were successively added, and the mixture was stirred at room temperature for 60 min. Trifluoroethanol (1.3 mL, 17 mmol) was added to the reaction solution and the mixture was stirred at room temperature for 30 min. Thereafter, 2,6-xylidine (2.6 mL, 21 mmol) and DDTT (892, 4.34 mmol) were added, and the solution was stirred at room temperature for 60 min. Molecular sieve 3A was removed by filtration, indole (2.0 g, 17 mmol) and trifluoroacetic acid (4.8 mL, 63 mmol) were successively added, and the solution was stirred at room temperature for 30 min. Trifluoroacetic acid (265 µL, 3.46 mmol) was further added, and the solution was stirred at room temperature for 30 min. Thereafter, pyridine (16 mL, 200 mmol) and water (742 µL) were successively added to the solution, and the solution was stirred at room temperature for 60 min. Thereafter, acetonitrile (858 mL) was added, and the precipitated solid was recovered by suction filtration using a Kiriyama funnel and then dried to give a dimer, oligonucleotide HO-T(S)T-SUC-TOB (2.6 g, yield 94%).
m/z: Calcd. 1610.03, Found 1611.05 [M+H]⁺

Under an argon atmosphere, HO-T(S)T-SUC-TOB (1.3 g, 811 µmol), dehydrated dichloromethane (40 mL), and dehydrated acetonitrile (12 mL) were added to a 100 mL two-necked flask to prepare a solution. Thereafter, molecular sieve 3A (816 mg), triphenylphosphine (109 mg, 417 µmol), dC(Bz)-CE phosphoramidite (1.4 g, 1.6 mmol), and 5-ethylthio-1H-tetrazole (212 mg, 1.63 µmοl) were successively added, and the solution was stirred at room temperature for 60 min. Molecular sieve 3A was removed by filtration, acetonitrile (30 mL) was added, and the precipitated solid was recovered by suction filtration using a Kiriyama funnel and then dried to give a trimer, oligonucleotide precursor DMTrO-C(III)T(S)T-SUC-TOB (1.8 g, yield 96%).
m/z: Calcd. 2342.26, Found 2343.28 [M+H]⁺

### Examples 5 to 15 and Comparative Example 2: Oxidation using various oxidants

DMTrO-C(III)T(S)T-SUC-TOB (60 mg, 25 µmol), dehydrated dichloromethane (1.3 mL), and dehydrated acetonitrile (0.4 mL) were added to a flask to prepare a solution. Water (17 µL) was added to the solution, various oxidants (38 µmοl) shown in the following Table 3 were added, and the mixture was stirred at room temperature for 3 hr to perform oxidation to synthesize a trimer, oligonucleotide DMTrO-C(O)T(S)T-SUC-TOB (hereinafter referred to as "oxidized product"). As shown in the following Table 3, Examples 6 and 7 using 2-(2-benzothiazolyldithio)propanoic acid (compound (Ib-2)) or 3-(2-benzothiazolyldithio)propanoic acid (compound (Ib-3)) as the oxidant, oxidation under anhydrous conditions without adding water to the solution in the aforementioned operation was also performed in addition to the oxidation in the presence of water as mentioned above.

As compound (Ib-2) to compound (Ib-5) and compound (Ib-8), those obtained in the aforementioned Synthetic Examples 3 to 7 were used.

Compound (Ic-1) used was synthesized by the method described in the Supporting Information of Non Patent Literature 2.

Other oxidants used were commercially available products.

### Comparison of Examples 5 to 15 and Comparative Example 2

The solution (10 µL) after oxidation in Examples 5 to 15 or Comparative Example 2 and DDTT (1 mg) were placed in a 1 mL vial, diluted with tetrahydrofuran (450 µL), DBU (20 µL) was added, and the solution was stirred at room temperature for 30 sec to sulfurize unreacted DMTrO-C(III)T(S)T-SUC-TOB to synthesize DMTrO-C(S)T(S)T-SUC-TOB (hereinafter "sulfurized product"), whereby a test solution containing the oxidized product and the sulfurized product was prepared. The obtained test solution was subjected to MS analysis and, using the abundance of the observed each compound (oxidized product (i.e., desired oligonucleotide DMTrO-C(O)T(S)T-SUC-TOB) and sulfurized product), the proportion of the oxidized product was calculated by the following formula: $proportion \left(%\right) of oxidized product = \left(\begin{matrix}abundance of oxidized \\ product / \left(\begin{matrix}abundance of oxidized product + abundance of sulfurized \\ product\end{matrix}\right)\end{matrix}\right) \times 100 .$ The results are shown in Table 3. The numerical values in parentheses in Examples 6 and 7 in Table 3 indicate the proportion of the oxidized product when oxidation was performed under anhydrous conditions.

**[Table 3]**

| | oxidant used | proportion of oxidized product |
|---|---|---|
| Example 5 | 2,2'-dibenzothiazolyl disulfide (compound (Ib-1)) | >90% |
| Example 6 | 2-(2-benzothiazolyldithio)propanoic acid (compound (Ib-2)) | >90% (>90%) |
| Example 7 | 3-(2-benzothiazolyldithio)propanoic acid (compound (Ib-3)) | >90% (>90%) |
| Example 8 | 2,2'-dibenzoimidazolyl disulfide (compound (Ib-4)) | >90% |
| Example 9 | 2,2'-dibenzoxazolyl disulfide (compound (Ib-5)) | >90% |
| Example 10 | 5,5'-di(1,2,3-triazolyl) disulfide (compound (Ib-6)) | >90% |
| Example 11 | 5,5'-dithiobis(1-phenyl-1H-tetrazole) (compound (Ib-7)) | >90% |
| Example 12 | 2,2'-dithiobis(pyridine-N-oxide) (compound (Ib-8)) | >90% |
| Example 13 | 4,4'-dipyridyl disulfide (compound (Ib-9)) | 61% |
| Example 14 | 3-(2-pyridyldithio)propanoic acid (compound (Ib-10)) | 59% |
| Example 15 | 2-phenylisothiazolo[5,4-b]pyridin-3(2H)-one (compound (Ic-1)) | >90% |
| Comparative Example 2 | 2,2'-dipyridyl disulfide | 23% |

As shown in Table 3, oligonucleotide precursors can be efficiently oxidized by using compound (Ib-1) to compound (Ib-10) or compound (Ic-1) as an oxidant, as compared with 2,2'-dipyridyl disulfide used in Non Patent Literature 1 (ARKIVOC 2009 (iii) 264-273).

### Example 16: Synthesis of DMTrO-Af(O)Cm(O)T(O)T-OH using compound (Ib-1) as oxidant

Under an argon atmosphere, HO-T-SUC-TOB (351 mg, 284 µmol), Piv-TOB (350 mg, 350 µmol), dehydrated dichloromethane (14 mL), and dehydrated acetonitrile (4 mL) were added to a 300 mL two-necked flask to prepare a solution. Thereafter, dT-CE phosphoramidite (402 mg, 540 µmοl) and 5-ethylthio-1H-tetrazole (69.9 mg, 537 pmol) were successively added, and the solution was stirred at room temperature for 45 min. dT-CE phosphoramidite (21 mg, 29 µmοl) and 5-ethylthio-1H-tetrazole (3.7 mg, 28 pmol) were further added, and the solution was stirred at room temperature for 35 min. Thereafter, water (184 µL, 10.2 mmol) was added, and the solution was stirred at room temperature for 30 min. Compound (Ib-1) (282 mg, 848 µmol) was added to the solution, and the solution was stirred at room temperature for 60 min. Thereafter, triphenylphosphine (74.2 mg, 283 pmol) was added, and the solution was stirred for 30 min, after which 2,3-dimethylfuran (298 µL, 2.83 mmol) and trifluoroacetic acid (563 µL, 7.35 mmol) were successively added, and the solution was stirred at room temperature for 30 min. Trifluoroacetic acid (86.6 µL, 1.13 mmol) was further added, and the solution was stirred at room temperature for 30 min. Then, pyridine (2.0 mL, 25 mmol) and water (120 µL) were successively added to the solution, and the solution was stirred at room temperature for 60 min. Thereafter, acetonitrile (170 mL) was added, and the precipitated solid was recovered by suction filtration using a Kiriyama funnel and dried under reduced pressure to give Piv-TOB and a dimer, oligonucleotide HO-T(O)T-SUC-TOB, as a mixture (766 mg, yield 96%).
m/z: Calcd. 1594.06, Found 1595.07 [M+H]⁺

In the same manner as above except that the solid obtained above (766 mg) and 2'-OMe-C-CE phosphoramidite instead of dT-CE phosphoramidite were used to give Piv-TOB and a trimer, oligonucleotide HO-Cm(O)T(O)T-SUC-TOB, as a mixture (875 mg, yield 98%).
m/z: Calcd. 2070.17, Found 2071.19 [M+H]⁺

Under an argon atmosphere, the solid obtained above (875 mg), dehydrated dichloromethane (13 mL), and dehydrated acetonitrile (4 mL) were added to a 200 mL two-necked flask to prepare a solution. Thereafter, 2'-F-A-CE phosphoramidite (441 mg, 503 pmol) and 5-ethylthio-1H-tetrazole (65.7 mg, 505 pmol) were successively added, and the solution was stirred at room temperature for 45 min. 2'-F-A-CE phosphoramidite (25 mg, 28 pmol) and 5-ethylthio-1H-tetrazole (3.4 mg, 26 µmοl) were further added, and the solution was stirred at room temperature for 60 min. Then, water (172 µL, 9.54 mmol) was added, and the solution was stirred at room temperature for 60 min, after which compound (Ib-1) (266 mg, 800 µmοl) was added to the solution, and the solution was stirred at room temperature for 60 min. Thereafter, triphenylphosphine (71.1 mg, 271 pmol) was added, and the solution was stirred at room temperature for 30 min. Thereafter, acetonitrile (129 mL) was added and the precipitated solid was recovered by suction filtration using a Kiriyama funnel and dried under reduced pressure to give Piv-TOB and a tetramer, oligonucleotide DMTrO-Af(O)Cm(O)T(O)T-SUC-TOB as a mixture (1007 mg, yield 93%).
m/z: Calcd. 2860.41, Found 2861.42 [M+H]⁺

The solid (9.3 mg) obtained above and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure by a centrifugation evaporator to give an aqueous solution of DMTrO-Af(O)Cm(O)T(O)T-OH.
m/z: Calcd. 1498.39, Found 1497.37 [M-H]⁻

### Example 17: Synthesis of DMTrO-Af(O)Cm(O)T(O)T-OH using compound (Ic-1) as oxidant

In the same manner as in Example 16 except that compound (Ib-1) was changed to compound (Ic-1), an aqueous solution of DMTrO-Af(O)Cm(O)T(O)T-OH was obtained.
m/z: Calcd. 1498.39, Found 1497.37 [M-H]⁻

### Comparative Example 3: Synthesis of DMTrO-Af(O)Cm(O)T(O)T-OH using iodine as oxidant

Under an argon atmosphere, HO-T-SUC-TOB (354 mg, 286 µmol) and Piv-TOB (351 mg, 352 µmol) were added, and then dehydrated dichloromethane (14 mL) and dehydrated acetonitrile (4.2 mL) were added to a 200 mL two-necked flask to prepare a solution. Thereafter, dT-CE phosphoramidite (406 mg, 545 µmol) and 5-ethylthio-1H-tetrazole (70.1 mg, 539 pmol) were successively added, and the solution was stirred at room temperature for 45 min. dT-CE phosphoramidite (23 mg, 31 pmol) and 5-ethylthio-1H-tetrazole (4.0 mg, 31 µmοl) were further added, and the solution was stirred at room temperature for 35 min. Then, water (180 µL, 10.2 mmol) was added, and the solution was stirred at room temperature for 30 min, after which pyridine (91 µL, 1.1 mmol) and iodine (145 mg, 569 µmοl) were successively added to the solution, and the solution was stirred at room temperature for 90 min. Thereafter, 2,3-dimethylfuran (298 µL, 2.83 mmol) and trifluoroacetic acid (650 µL, 8.48 mmol) were successively added, and the solution was stirred at room temperature for 30 min. Trifluoroacetic acid (86.6 µL, 1.13 mmol) was further added, and the solution was stirred at room temperature for 30 min. Pyridine (2.3 mL, 29 mmol) and water (120 µL) were successively added to the solution, and the solution was stirred at room temperature for 60 min. Thereafter, acetonitrile (140 mL) was added and the precipitated solid was recovered by suction filtration using a Kiriyama funnel and dried under reduced pressure to quantitatively give Piv-TOB and a dimer, oligonucleotide HO-T(O)T-SUC-TOB as a mixture (827 mg).
m/z: Calcd. 1594.06, Found 1595.07 [M+H]⁺

In the same manner as above except that the solid obtained above (812 mg) and 2'-OMe-C-CE phosphoramidite instead of dT-CE phosphoramidite were used, Piv-TOB and a trimer, oligonucleotide HO-Cm(O)T(O)T-SUC-TOB were obtained as a mixture (893 mg, yield 94%).
m/z: Calcd. 2070.17, Found 2071.19 [M+H]⁺

Under an argon atmosphere, the solid obtained above (889 mg), dehydrated dichloromethane (13 mL), and dehydrated acetonitrile (4.0 mL) were added to a 200 mL two-necked flask to prepare a solution. Thereafter, 2'-F-A-CE phosphoramidite (729 mg, 833 µmοl) and 5-ethylthio-1H-tetrazole (107 mg, 819 pmol) were successively added, and the solution was stirred at room temperature for 105 min. Then, water (174 µL, 9.63 mmol) was added, and the solution was stirred at room temperature for 30 min, after which pyridine (200 µL, 2.48 mmol) and iodine (317 mg, 1.25 mmol) were successively added to the solution, and the solution was stirred at room temperature for 60 min. Triphenylphosphine (109 mg, 414 µmοl) was further added, and the solution was stirred at room temperature for 30 min. Thereafter, acetonitrile (137 mL) was added and the precipitated solid was recovered by suction filtration using a Kiriyama funnel and dried under reduced pressure to give Piv-TOB and a tetramer, oligonucleotide DMTrO-Af(O)Cm(O)T(O)T-SUC-TOB as a mixture (1052 mg, yield 95%).
m/z: Calcd. 2860.41, Found 2861.42 [M+H]⁺

The solid (9.8 mg) obtained above and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure in a centrifugation evaporator to give an aqueous solution of DMTrO-Af(O)Cm(O)T(O)T-OH.
m/z: Calcd. 1498.39, Found 1497.37 [M-H]⁻

### Comparison of Example 16, Example 17 and Comparative Example 3

DMTrO-Af(O)Cm(O)T(O)T-OH (hereinafter referred to as "desired oligonucleotide"), and DMTrO-Af(O)Cm(O)T-OH or DMTrO-Af(O)T(O)T-OH (hereinafter referred to as "defective byproduct"), which is a trimer lacking the 2nd residue or 3rd residue from the desired oligonucleotide, in the aqueous solutions obtained in Example 16, Example 17, and Comparative Example 3 were measured by MS analysis. In the MS analysis, the peak area of each compound was calculated using the extraction ion chromatogram (EIC) of the observed each compound (desired oligonucleotide and defective byproduct), and the proportion (%) of the defective byproduct was calculated by the following formula: $proportion \left(%\right) of defective byproduct = \left(\begin{matrix}total peak area of \\ defective byproducts / peak area of desired oligonucleotide\end{matrix}\right) \times 100 .$ The results are shown in Table 4.

**[Table 4]**

| | oxidant used | proportion of defective byproduct |
|---|---|---|
| Example 16 | 2,2'-dibenzothiazolyl disulfide (compound (Ib-1)) | <0.4% |
| Example 17 | 2-phenylisothiazolo[5,4-b]pyridin-3(2H)-one (compound (Ic-1)) | <0.4% |
| Comparative Example 3 | iodine | 1.2% |

As is clear from the results of Table 4, production of defective byproducts can be suppressed by using compound (Ib-1) or compound (Ic-1) as an oxidant instead of iodine.

### Example 18: Synthesis of HO-Af(O)Cm(O)T(O)T-SUC-TOB using compound (Ic-1) as oxidant (with addition of aromatic amine)

Under an argon atmosphere, HO-T-SUC-TOB (6.4 g, 5.2 mmol), Piv-TOB (13 g, 13 mmol), dehydrated dichloromethane (349 mL) and dehydrated acetonitrile (98 mL) were added to a 3 L three-necked flask to prepare a solution. Thereafter, dT-CE phosphoramidite (7.3 g, 9.8 mmol) and 5-ethylthio-1H-tetrazole (1.3 g, 9.8 mmol) were successively added, and the solution was stirred at room temperature for 65 min. dT-CE phosphoramidite (385 mg, 517 pmol) and 5-ethylthio-1H-tetrazole (67.3 mg, 517 µmοl) were further added, and the solution was stirred at room temperature for 30 min, after which water (932 µL, 51.7 mmol) was added, and the solution was stirred at room temperature for 40 min. Then, compound (Ic-1) (3.5 g, 15 mmol) was added, and the solution was stirred for 60 min. Triphenylphosphine (1.4 g, 5.2 mmol) and 2,6-xylidine (5.5 mL, 45 mmol) as aromatic amine were successively added to the solution, and the solution was stirred at room temperature for 30 min. Thereafter, 2,3-dimethylfuran (5.4 mL, 52 mmol) and trifluoroacetic acid (13.8 mL, 180 mmol) were successively added, and the mixture was stirred at room temperature for 80 min. Then, pyridine (43.7 mL, 541 mmol) and water (2.5 mL) were successively added to the solution, and the solution was stirred at room temperature for 90 min. Thereafter, acetonitrile was added, and the precipitated solid was recovered by suction filtration using a Kiriyama funnel and then dried to give Piv-TOB and a dimer, oligonucleotide HO-T(O)T-SUC-TOB, as a mixture (21 g, yield 98%).
m/z: Calcd. 1594.06, Found 1595.07 [M+H]⁺

In the same manner as above except that the solid obtained above (21 g) and 2'-OMe-C-CE phosphoramidite instead of dT-CE phosphoramidite were used, Piv-TOB and a trimer, oligonucleotide HO-Cm(O)T(O)T-SUC-TOB were quantitatively obtained as a mixture (23 g).
m/z: Calcd. 2070.17, Found 2071.19 [M+H]⁺

In the same manner as above except that the solid obtained above (21 g) and 2'-F-A-CE phosphoramidite instead of 2'-OMe-C-CE phosphoramidite were used, Piv-TOB and a tetramer, HO-Af(O)Cm(O)T(O)T-SUC-TOB, were obtained as a mixture (23 g, yield 99%).
m/z: Calcd. 2558.28, Found 2559.29 [M+H]⁺

In addition, in order to confirm the effect of the addition of aromatic amine, acetonitrile (9 mL) was added to the solution obtained by extracting 1.3 mL from the reaction solution before addition of 2,3-dimethylfuran, and the precipitated solid was suction-filtered with a Kiriyama funnel and then dried to obtain Piv-TOB and a tetramer, oligonucleotide DMTrO-Af(O)Cm(O)T(O)T-SUC-TOB, as a mixture (69 mg).
m/z: Calcd. 2860.41, Found 2861.42 [M+H]⁺

DMTrO-Af(O)Cm(O)T(O)T-SUC-TOB (3.4 mg) obtained above and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure in a centrifugation evaporator to give an aqueous solution of DMTrO-Af(O)Cm(O)T(O)T-OH.
m/z: Calcd. 1498.39, Found 1497.37 [M-H]⁻

### Comparison of Example 17 and Example 18

DMTrO-Af(O)Cm(O)T(O)T-OH (hereinafter referred to as "desired oligonucleotide"), and DMTrO-Af(O)Cm(O)T-OH or DMTrO-Af(O)T(O)T-OH (hereinafter referred to as "defective byproduct"), which is a trimer lacking the 2nd residue or 3rd residue from the desired oligonucleotide, in the aqueous solutions obtained in Example 17 and Example 18 were measured by MS analysis. In the MS analysis, the peak area of each compound was calculated using the extraction ion chromatogram (EIC) of the observed each compound (desired oligonucleotide and defective byproduct), and the proportion (%) of the defective byproduct was calculated by the following formula: $proportion \left(%\right) of defective byproduct = \left(\begin{matrix}total peak area of \\ defective byproducts / peak area of desired oligonucleotide\end{matrix}\right) \times 100 .$ The results are shown in Table 5.

**[Table 5]**

| | oxidant used | addition of aromatic amine | proportion of defective byproduct |
|---|---|---|---|
| Example 17 | 2-phenylisothiazolo[5,4-b]pyridin-3(2H)-one (compound (Ic-1)) | not added | <0.4% |
| Example 18 | 2-phenylisothiazolo[5,4-b]pyridin-3(2H)-one (compound (Ic-1)) | added | <0.2% |

As is clear from the results of Table 5, production of defective byproducts can be further suppressed by adding aromatic amine (2,6-xylidine) after an oxidation step using compound (Ic-1).

### Example 19: Synthesis of HO-^{Me}C(O)A(O)G(O)T(S)T-SUC-TOB using compound (Ib-1) as oxidant

Under an argon atmosphere, HO-T(S)T-SUC-TOB (250 mg, 88 µmol), dehydrated dichloromethane (4.4 mL), and dehydrated acetonitrile (1.3 mL) were added to a 30 mL two-necked flask to prepare a solution. Thereafter, dG-CE phosphoramidite (149 mg, 176 pmol) and 5-ethylthio-1H-tetrazole (23 mg, 0.18 mmol) were successively added, and the solution was stirred at room temperature for 45 min. Thereafter, 2,2,2-trifluoroethanol (38 µL, 0.53 mmol) was added to the solution, and the solution was stirred at room temperature for 30 min. Then, water (70 µL) and compound (Ib-1) (59 mg, 18 mmol) were added, and the solution was stirred at room temperature for 60 min. A part of the solution was sampled, 2,3-dimethylfuran (46 µL, 0.88 mmol) and trifluoroacetic acid (102 µL, 1.32 mmol) were successively added to the solution, and the solution was stirred at room temperature for 80 min. Then, pyridine (319 µL, 3.95 mmol) and water (19 µL) were successively added to the solution, and the solution was stirred at room temperature for 90 min. Thereafter, acetonitrile was added, and the precipitated solid was recovered by suction filtration using a Kiriyama funnel and then dried to give a trimer, oligonucleotide HO-G(O)T(O)T-SUC-TOB (111 mg).
m/z: Calcd. 2062.15, Found 2063.16 [M+H]⁺

In the same manner as above except that the solid obtained above (111 mg, 34 µmοl) and dA-CE phosphoramidite instead of dG-CE phosphoramidite were used, a tetramer, oligonucleotide HO-A(O)G(O)T(S)T-SUC-TOB, was obtained (113 mg, yield 90%).
m/z: Calcd. 2497.30, Found 2498.32 [M+H]⁺

In the same manner as above except that the solid obtained above (99 mg, 27 µmοl) and 5-Me-dC(Bz)-CE phosphoramidite instead of dA-CE phosphoramidite were used, a pentamer, oligonucleotide HO-^{Me}C(O)A(O)G(O)T(S)T-SUC-TOB, was obtained (93 mg, yield 84%).
m/z: Calcd. 2957.41, Found 1479.71 [M+2H]²⁺

### Example 20: Synthesis of HO-^{Me}C(O)A(O)G(O)T(S)T-SUC-TOB using compound (Ib-1) as oxidant (with addition of aromatic amine)

In the same manner as in Example 19 except that a step of adding 2,6-xylidine (3 mol per 1 mol of compound (Ib-1)) as aromatic amine to a solution and stirring the solution at room temperature for 30 min was added after the oxidation step (i.e., step of adding compound (Ib-1) to a solution and stirring the solution at room temperature for 30 min), HO-^{Me}C(O)A(O)G(O)T(S)T-SUC-TOB was obtained (121 mg).

### Comparison of Example 19 and Example 20

HO-^{Me}C(O)A(O)G(O)T(S)T-SUC-TOB obtained in Example 19 and Example 20 was dissolved in tetrahydrofuran, protecting groups (2-cyanoethyl group) bound to phosphate moiety and thiophosphate moiety were removed with DBU, oligonucleotide from which protecting groups have been removed (hereinafter referred to as " desired oligonucleotide"), and a tetramer oligonucleotide lacking any of ^{Me}C, A, and G from the desired oligonucleotide (hereinafter referred to as "defective byproduct") were measured by MS analysis. In the MS analysis, the peak area of each compound was calculated using extraction ion chromatogram (EIC) of each observed compound (desired oligonucleotide and defective byproduct), and the proportion of the defective byproduct was calculated by the following formula: $proportion of defective byproduct \left(%\right) = \left(\begin{matrix}total peak area of \\ respective defective byproducts / peak area of desired \\ oligonucleotide\end{matrix}\right) \times 100 .$

**[Table 6]**

| | oxidant used | addition of aromatic amine | proportion of defective byproduct |
|---|---|---|---|
| Example 19 | 2,2'-dibenzothiazolyl disulfide (compound (Ib-1)) | not added | 0.95% |
| Example 20 | 2,2'-dibenzothiazolyl disulfide (compound (Ib-1)) | added | <0.50% |

As is clear from the results of Table 6, production of defective byproducts can be further suppressed by adding aromatic amine (2,6-xylidine) after the oxidation step using compound (Ib-1).

### Example 21: Solid phase synthesis of 20-mer, oligonucleotide

(DMTrO-
Um(O)Cm(O)Am(O)Am(O)Gm(S)Gm(S)Am(S)Am(S)Gm(S)Am(S)Um(S)Gm(S)Gm(S)C
m(S)Am(S)Um(S)Um(S)Um(S)Cm(S)Um-OH) (SEQ ID NO: 1)

A 20-mer oligonucleotide with the aforementioned sequence was solid phase synthesized by a phosphoramidite method (in the aforementioned sequence, Am shows a 2'-O-methyladenosine residue, Cm shows a 2'-O-methylcytidine the residue, Gm shows a 2'-O-methylguanosine residue, Um shows a 2'-O-methyluridine residue, (O) shows a phosphate ester bond represented by the aforementioned formula (P3-1), and (S) shows a thiophosphate ester bond represented by the aforementioned formula (P4-1)).

Specifically 5'-O-(4,4'-dimethoxytrityl)-2'-O-methyluridine was carried at 41 pmollg on a porous glass (CPG) solid support (manufactured by ChemGenes) (amount of solid support: 24 mg, amount of 5'-O-(4,4'-dimethoxytrityl)-2'-O-methyluridine: 1 µmοl) and placed in a column equipped with a glass filter, and synthesis was performed from the 3' side to the 5' side using an oligonucleotide synthesizer (trade name "nS-8", manufactured by GeneDesign, Inc.). Respective acetonitrile solutions of N⁶-benzoyl-5'-O-(4,4'-dimethoxytrityl)-2'-O-methyladenosine phosphoramidite and N⁴-acetyl-5'-O-(4,4'-dimethoxytrityl)-2'-O-methylcytidine phosphoramidite, N²-isobutyryl-5'-O-(4,4'-dimethoxytrityl)-2'-O-methylguanosine phosphoramidite, and 5'-O-(4,4'-dimethoxytrityl)-2'-O-methyluridine phosphoramidite were used as phosphoramidite, an acetonitrile solution of 5-ethylthio-1H-tetrazole was used as the activator, a pyridine solution of DDTT was used as the sulfurizing agent, a mixed solution of a THF solution of 10 wt% acetic anhydride and a THF solution of 1-methylimidazole and pyridine was used as the capping reagent, and a dichloromethane solution of trichloroacetic acid (TCA) was used as the detritylation reagent. As the oxidant, (1) a pyridine solution of 0.01 M compound (Ib-1), (2) a pyridine solution of 0.1 M compound (Ic-1), or (3) an acetonitrile solution of 0.02 M compound (Ic-1) was used.

For cleaving the synthesized oligonucleotide from the solid support and deprotecting the base portion thereof, the synthesized oligonucleotide was treated with a mixed solution of 28 wt% aqueous ammonia and ethanol (ammonia water:ethanol volume ratio=3:1) at 55°C for 15 hr. Then the solid support was collected by filtration, and the filtrate was concentrated under reduced pressure using a centrifugal concentrator. The synthesized oligonucleotide was analyzed by LC-MS and the molecular weight was measured. As a result, it was confirmed that the desired oligonucleotide could be synthesized by solid phase synthesis using compound (Ib-1) or compound (Ic-1) as an oxidant.
m/z: Calcd. 7215.88, Found 2404.43 [M-3H]³-

### Synthetic Example 9: Synthesis of 2-phenylisothiazolo[5,4-b]quinolin-3(2H)-one (compound (Ic-11))

Under an argon atmosphere, 2-chloroquinoline-3-carboxylic acid (2.5 g, 12 mmol) and thionyl chloride (40 mL) were added to a 100 mL flask, and the mixture was stirred at 80°C for 17 hr. Thionyl chloride was evaporated under reduced pressure, toluene (30 mL) was added, and the obtained solution was concentrated under reduced pressure. Toluene (60 mL) was added again, and the obtained solution was dried under reduced pressure to give a solid. THF (15 mL) was added to the obtained solid, and triethylamine (2.5 mL, 18 mmol) and aniline (1.3 mL, 14 mmol) were slowly added dropwise in an ice bath. The obtained mixture was heated to room temperature, THF (2 mL) and triethylamine (0.5 mL, 3.6 mmol) were successively added, and the mixture was stirred for 2.5 hr. The solvent was evaporated under reduced pressure, and dichloromethane (50 mL) was added. The obtained organic layer was washed twice with a saturated sodium hydrogen carbonate aqueous solution and once with saturated brine, and dried over sodium sulfate. Sodium sulfate was removed by was removed by filtration, and the filtrate was dried under reduced pressure to give a solid.

Dehydrated DMF (10 mL) was added to the aforementioned solid to prepare a diluted solution. Under an argon atmosphere, dehydrated DMF (2 mL), sodium hydroxide (0.48 g, 12 mmol), and tert-butylmercaptan (1.4 mL, 12 mmol) were added to a 100 mL flask and the mixture was stirred under ice-cooling. The aforementioned diluted solution was added dropwise to the obtained mixture, and the mixture was heated to room temperature and stirred for 4 hr. Thereafter, water (120 mL) was slowly added dropwise to the mixture in an ice bath. The precipitated solid was collected by filtration and ethanol (400 mL) was added. The mixture was stirred at 100°C and then cooled to room temperature. The solid precipitated from the mixture was collected by filtration, and the obtained solid was dried under reduced pressure (1.7 g). The filtrate was concentrated under reduced pressure, ethanol (400 mL) was added, and the mixture was stirred at 100°C. Then, water (200 mL) was added dropwise, and the mixture was cooled to room temperature. The solid precipitated from the mixture was collected by filtration, and the obtained solid was dried under reduced pressure (0.85 g). The obtained 1.7 g of solid and 0.85 g of the solid were mixed, and 2.0 g of the solid therefrom was used in the next step.

Under an argon atmosphere, the aforementioned solid (2.0 g) and dichloromethane (19 mL) were added to a 100 mL flask, and the mixture was stirred under ice-cooling for 15 min. Meta-chloroperoxybenzoic acid (1.5 g, 6.4 mmol) was added to the obtained mixture, and the mixture was stirred for 1 hr to prepare an organic layer. A saturated sodium hydrogen carbonate aqueous solution (20 mL) was added to the obtained organic layer, and the mixture was stirred at room temperature for 30 min. The precipitated solid was removed by filtration, the aqueous layer of the filtrate was removed, and the organic layer was washed successively with saturated sodium thiosulfate aqueous solution (20 mL) and saturated brine (20 mL), dried over sodium sulfate, filtered, and concentrated under reduced pressure (1.3 g). Toluene (72 mL) and pyridine (7.2 mL) were added to the obtained concentrate, and the mixture was heated to 100°C and stirred for 2 hr. Thereafter, the mixture was cooled to room temperature, and the precipitated solid was recovered by filtration. Ethanol (200 mL) was added to the obtained solid, and the mixture was stirred at 100°C. Water (67 mL) was added dropwise, and the mixture was cooled to room temperature. The precipitated solid was collected by filtration, the obtained solid silica was purified by gel column chromatography (hexane/ethyl acetate volume ratio=2/1), and concentrated under reduced pressure. Ethanol was added to the obtained concentrate, and the mixture was stirred at 100°C, water was added dropwise, and the mixture was cooled to room temperature. The precipitated solid was collected by filtration, and dried under reduced pressure to give the desired product (121 mg).
¹H NMR (400 MHz, Chloroform-d) δ 8.85 (s, 1H), 8.06 (d, J = 8.6 Hz, 1H), 7.99 (dd, J = 8.3, 1.4 Hz, 1H), 7.83 (ddd, J = 8.5, 6.8, 1.5 Hz, 1H), 7.73 to 7.65 (m, 2H), 7.57 (ddd, J = 8.1, 7.0, 1.0 Hz, 1H), 7.48 to 7.39 (m, 2H), 7.32 to 7.24 (m, 1H).
m/z: Calcd. 278.05, Found 279.06 [M+H]⁺

### Synthetic Example 10: Synthesis of 2-phenyl-5-trifluoromethylisothiazolo[5,4-b]pyridin-3(2H)-one (compound (Ic-12))

2-Chloro-5-(trifluoromethyl)pyridine-3-carboxylic acid (2.5 g, 11 mmol) and thionyl chloride (37 mL) were added to a 200 mL flask, and the mixture was stirred at 80°C for 4 hr. Thionyl chloride was evaporated under reduced pressure, toluene (30 mL) was added, and the obtained solution was concentrated under reduced pressure to give a concentrate.

To the aforementioned concentrate was added dehydrated THF (5 mL), and the mixture was stirred under ice-cooling to prepare a diluted solution. A solution of triethylamine (1.9 mL, 13 mmol), aniline (1.2 mL, 13 mmol) and dehydrated THF (10 mL) was added to the aforementioned diluted solution, and the obtained mixture was heated to room temperature and stirred for 2.5 hr. After evaporation of the solvent under reduced pressure, dichloromethane (30 mL) and saturated sodium hydrogen carbonate aqueous solution (50 mL) were added to the obtained residue, and the mixture was stirred. The aqueous layer was removed, and the organic layer was washed successively with saturated sodium hydrogen carbonate aqueous solution (50 mL) and saturated brine (50 mL), dried over sodium sulfate and dried under reduced pressure to give a solid (3.1 g).

Dehydrated DMF (5 mL) was added to the aforementioned solid to prepare a diluted solution. Under an argon atmosphere, dehydrated DMF (2 mL), sodium hydroxide (0.47 g, 12 mmol), and tert-butylmercaptan (1.3 mL, 11 mmol) were added to a 100 mL flask and the mixture was stirred under ice-cooling. The aforementioned diluted solution was added dropwise to the obtained mixture, and the mixture was heated to room temperature and stirred for 4 hr. Thereafter, water (70 mL) was slowly added dropwise to the mixture under ice-cooling. The precipitated solid was collected by filtration and ethanol (30 mL) was added. The mixture was stirred at 100°C, water (30 mL) was added dropwise, and the mixture was cooled to room temperature. The precipitated solid was collected by filtration, and the obtained solid was dried under reduced pressure. Ethanol (30 mL) was added to the obtained solid, and the mixture was stirred at 100°C, after which water (30 mL) was added dropwise and the mixture was cooled to room temperature. The precipitated solid was collected by filtration, and the obtained solid was dried under reduced pressure (3.4 g).

Under an argon atmosphere, the aforementioned solid (3.4 g) and dichloromethane (32 mL) were added to a 100 mL flask, and the mixture was stirred under ice-cooling for 15 min. Meta-chloroperoxybenzoic acid (2.5 g, 10 mmol) was added to the obtained mixture, and the mixture was stirred for 2 hr to prepare an organic layer. A saturated sodium hydrogen carbonate aqueous solution (20 mL) was added to the obtained organic layer, and the mixture was stirred at room temperature for 1 hr. The aqueous layer was removed, and the organic layer was washed successively with saturated sodium thiosulfate aqueous solution (50 mL) and saturated brine (50 mL), dried over sodium sulfate, filtered, and concentrated under reduced pressure (3.4 g). Toluene (186 mL) and pyridine (19 mL) were added to the obtained concentrate, and the mixture was heated to 100°C and stirred for 1.5 hr. Thereafter, the mixture was cooled to room temperature, and the solvent was removed under reduced pressure. Ethanol (160 mL) was added to the obtained residue, and the mixture was heated to 100°C. Water (80 mL) was slowly added dropwise and the mixture was stirred at room temperature for 1 hr, and under ice-cooling for 15 min. The precipitated solid was collected by filtration and dried under reduced pressure. Ethanol (120 mL) was added to the obtained solid, and the mixture was heated to 100°C. Water (50 mL) was slowly added dropwise. The mixture was further stirred at room temperature for 1 hr, and under ice-cooling for 15 min. The precipitated solid was collected by filtration and dried under reduced pressure to give the desired product (2.1 g).
¹H NMR (400 MHz, Chloroform-d) δ 9.08 (d, J = 2.2 Hz, 1H), 8.62 (d, J = 2.0 Hz, 1H), 7.75 to 7.67 (m, 2H), 7.59 to 7.49 (m, 2H), 7.46 to 7.37 (m, 1H).
m/z: Calcd. 296.02, Found 297.03 [M+H]⁺

### Synthetic Example 11: Synthesis of 2-phenyl-2H-1,2-benzothiazin-3(4H)-one (compound (Ic-13))

Under an argon atmosphere, 2-chloropyridine-3-acetonitrile (5.0 g, 33 mmol) and 4 mol/L sodium hydroxide aqueous solution (75 mL) were added to a 300 mL flask, and the mixture was stirred at 110°C for 1 hr. The mixture was cooled to room temperature and stirred for 1 hr. Under ice-cooling, 6 mol/L hydrochloric acid (60 mL) was slowly added dropwise, and the mixture was stirred for 1 hr. The precipitated solid was collected by filtration, washed with 2-propanol (40 mL), and dried under reduced pressure (5.6 g).

Under an argon atmosphere, the aforementioned solid and dehydrated THF (109 mL) were added to a 200 mL flask, and the mixture was stirred under ice-cooling for 15 min. Triethylamine (9.2 mL, 66 mmol), aniline (3.6 mL, 39 mmol), and 1-ethyl-3-[3-(dimethylamino)propyl]carbodiimide hydrochloride (9.4 g, 49 mmol) were successively added to the obtained mixture. The mixture was heated to room temperature and stirred for 2 hr. Ethyl acetate (100 mL) was added to the mixture, and the obtained organic layer was washed three times with saturated sodium hydrogen carbonate aqueous solution (100 mL) and once with saturated brine (100 mL), and dried over sodium sulfate. Sodium sulfate was removed by filtration, and the filtrate was concentrated under reduced pressure to give a concentrate (8.1 g).

Dehydrated DMF (20 mL) was added to the aforementioned concentrate to prepare a diluted solution. Under an argon atmosphere, dehydrated DMF (2 mL), sodium hydroxide (2.6 g, 64 mmol), and tert-butylmercaptan (4.4 mL, 39 mmol) were added to a 300 mL flask and the mixture was stirred under ice-cooling for 10 min. The aforementioned diluted solution was added dropwise to the obtained mixture, and the mixture was stirred at 110°C for 23 hr. Thereafter, water (260 mL) was slowly added dropwise to the mixture under ice-cooling. Dichloromethane was added to the mixture, and the obtained organic layer was washed twice with water and once with saturated brine, and dried over sodium sulfate. Sodium sulfate was removed by filtration, and the filtrate was concentrated under reduced pressure to give a concentrate. The obtained concentrate was purified by silica gel column chromatography (hexane/ethyl acetate volume ratio=1/1), and concentrated under reduced pressure to give a concentrate (1.4 g).

Under an argon atmosphere, dichloromethane (16 mL) was added to the aforementioned concentrate, and the mixture was stirred under ice-cooling for 15 min. Meta-chloroperoxybenzoic acid (1.2 g, 5.2 mmol) was added to the obtained mixture, and the mixture was stirred for 1 hr to prepare an organic layer. A saturated sodium hydrogen carbonate aqueous solution (16 mL) was added to the obtained organic layer, and the mixture was stirred at room temperature for 30 min. The aqueous layer was removed, the organic layer was washed with saturated sodium thiosulfate aqueous solution (20 mL) and saturated brine (20 mL), dried over sodium sulfate, filtered, and concentrated under reduced pressure (1.3 g). Toluene (85 mL) and pyridine (8.5 mL) were added to the obtained concentrate, and the mixture was heated to 100°C and stirred for 1 hr. The mixture was heated to 120°C and stirred for 4 hr. Thereafter, the mixture was cooled to room temperature, and the solvent was evaporated under reduced pressure. The obtained residue was purified by silica gel column chromatography (hexane/ethyl acetate volume ratio=1/3), and concentrated under reduced pressure to give a concentrate. Ethanol was added to the obtained concentrate, and the mixture was heated to 100°C and water was slowly added dropwise. The mixture was cooled to room temperature, and the precipitated solid was collected by filtration to give the desired product (146 mg).
¹H NMR (400 MHz, Chloroform-d) δ 8.82 to 8.71 (m, 1H), 8.28 (d, J = 4.1 Hz, 1H), 7.71 (d, J = 7.4 Hz, 1H), 7.55 (d, J = 7.9 Hz, 2H), 7.32 to 7.28 (m, 1H), 7.17 to 7.07 (m, 2H), 4.00 (s, 2H).
m/z: Calcd. 242.05, Found 243.05 [M+H]⁺

### Synthetic Example 12: Synthesis of 2-(4-methyl-2-pyridinyl)isothiazolo[5,4-b]pyridin-3(2H)-one (compound (Ic-15))

Under an argon atmosphere, 2-chloropyridine-3-carbonyl chloride (5.0 g, 28 mmol) and dehydrated THF (12 mL) were added to a 100 mL flask, and the mixture was stirred under ice-cooling. A solution of triethylamine (4.8 mL, 34 mmol), 2-amino-5-methylpyridine (3.7 g, 34 mmol), and dehydrated THF (28 mL) was added dropwise to the obtained mixture over 30 min, and the mixture was heated to room temperature and stirred for 23 hr. After evaporation of the solvent under reduced pressure, dichloromethane (50 mL) and saturated sodium hydrogen carbonate aqueous solution (50 mL) were added to the obtained residue, and the mixture was stirred. The aqueous layer was removed, and the organic layer was washed successively with saturated sodium hydrogen carbonate aqueous solution (50 mL) and saturated brine (50 mL), dried over sodium sulfate, and concentrated under reduced pressure to give a concentrate (7.1 g).

Dehydrated DMF (20 mL) was added to the aforementioned solid to prepare a diluted solution. Under an argon atmosphere, dehydrated DMF (4 mL), sodium hydroxide (1.3 g, 34 mmol), and tert-butylmercaptan (3.6 mL, 32 mmol) were added to a 300 mL flask and the mixture was stirred under ice-cooling. The aforementioned diluted solution was added dropwise to the obtained mixture, and the mixture was heated to room temperature and stirred for 5 hr. Thereafter, water (240 mL) was slowly added dropwise to the mixture over 25 min under ice-cooling. The precipitated solid was collected by filtration, ethanol (60 mL) was added, and the mixture was stirred at 100°C. Water (60 mL) was added dropwise, and the mixture was cooled under ice-cooling. The precipitated solid was collected by filtration, and the obtained solid was dried under reduced pressure (5.4 g).

Under an argon atmosphere, the aforementioned solid and dichloromethane (60 mL) were added to a 100 mL flask, and the mixture was stirred under ice-cooling for 15 min. Meta-chloroperoxybenzoic acid (4.7 g, 20 mmol) was added to the obtained mixture, and the mixture was stirred for 2 hr to prepare an organic layer. A saturated sodium hydrogen carbonate aqueous solution (60 mL) was added to the obtained organic layer, and the mixture was stirred at room temperature for 1 hr. The precipitated solid was removed by filtration. The aqueous layer of the filtrate was removed, and the organic layer was washed successively with saturated sodium thiosulfate aqueous solution (50 mL) and saturated brine (50 mL), dried over sodium sulfate, filtered, and concentrated under reduced pressure (5.7 g). Toluene (356 mL) and pyridine (36 mL) were added to the obtained concentrate, and the mixture was heated to 100°C and stirred for 1 hr. Thereafter, the mixture was cooled to room temperature, and precipitated solid was removed by filtration, and the solvent of the filtrate was removed under reduced pressure. The obtained residue was purified by silica gel column chromatography (hexane/ethyl acetate volume ratio=1/1) and concentrated under reduced pressure to give the desired product (1.3 g).
¹H NMR (400 MHz, Chloroform-d) δ 8.82 (dd, J = 4.8, 1.7 Hz, 1H), 8.60 (d, J = 8.5 Hz, 1H), 8.33 (dd, J = 7.9, 1.7 Hz, 1H), 8.26 (s, 1H), 7.66 (dd, J = 8.5, 2.3 Hz, 1H), 7.39 (dd, J = 7.9, 4.7 Hz, 1H), 2.39 (s, 3H).
m/z: Calcd. 243.05, Found 244.05 [M+H]⁺

### Synthetic Example 13: Synthesis of oligonucleotide precursor having phosphite ester bond

Under an argon atmosphere, HO-^{Me}C-SUC-TOB (15.1 g, 11.2 mmol), Piv-TOB (30.1 g, 30.2 mmol), dehydrated chloroform (562 mL), and dehydrated acetonitrile (169 mL) were added to a 500 mL flask to prepare a solution. Thereafter, molecular sieve 3A (11.2 g), dG-CE phosphoramidite (18.9 g, 22.5 mmol), and triphenylphosphine (1.5 g, 5.6 mmol) were added, and the solution was stirred for 30 min. Then, 5-ethylthio-1H-tetrazole (2.9 g, 22.5 mmol) was added, and the solution was stirred at room temperature for 60 min. Thereafter, 2,2,2-trifluoroethanol (8.2 mL, 112 mmol) was added to the solution, and the mixture was stirred at room temperature for 30 min. Then, 2,6-dimethylaniline (10 mL, 81 mmol) and POS (5.5 g, 28 mmol) were successively added, and the solution was stirred at room temperature for 1.0 hr. Thereafter, 2,3-dimethylfuran (11.8 mL, 0.11 mol), trifluoroacetic acid (27.1 mL, 0.35 mol), and 2,6-dimethylaniline (436 µL, 3.5 mmol) were successively added, and the solution was stirred at room temperature for 60 min. Trifluoroacetic acid (1.7 mL, 22 mmol) and 2,6-dimethylaniline (28 µL, 0.22 mmol) were further added, and the solution was stirred at room temperature for 30 min. Then, pyridine (91 mL, 1.1 mmol) and dehydrating methanol (7.3 mL) were successively added to the solution, and the solution was stirred at room temperature for 120 min. Molecular sieve 3A was removed by was removed by filtration, acetonitrile (4.7 L) was added, and the precipitated solid was recovered by suction filtration using a Kiriyama funnel and then dried to give Piv-TOB and a dimer, oligonucleotide HO-G(S)^{Me}C-SUC-TOB, as a mixture (50 g, yield 99%).
m/z: Calcd. 1808.12, Found 1809.13 [M+H]⁺

In the same manner as above except that the solid obtained above (50 g), dehydrated dichloromethane instead of dehydrated chloroform, and dA-CE phosphoramidite instead of dG-CE phosphoramidite were used, Piv-TOB and a trimer, oligonucleotide HO-A(S)G(S)^{Me}C-SUC-TOB, were obtained as a mixture (54.4 g, yield 99%).
m/z: Calcd. 2259.24, Found 2260.25 [M+H]⁺

Under an argon atmosphere, the solid (3.0 g) obtained above, dehydrated dichloromethane (30 mL), and dehydrated acetonitrile (9.1 mL) were added to a 20 mL two-necked flask to prepare a solution. Thereafter, molecular sieve 3A (0.6 g), dG-CE phosphoramidite (1.0 g, 1.2 mmol), and 5-ethylthio-1H-tetrazole (161 mg, 1.2 mmol) were successively added, and the solution was stirred at room temperature for 60 min. Thereafter, 2,2,2-trifluoroethanol (197 µE, 6.1 mmol) was added to the solution, and the mixture was stirred at room temperature for 30 min. Molecular sieve 3A was removed by filtration, acetonitrile (152 mL) was added, and the precipitated solid was recovered by suction filtration using a Kiriyama funnel and then dried to give Piv-TOB and a tetramer, oligonucleotide precursor DMTrO-G(III)A(S)G(S)^{Me}C-SUC-TOB, as a mixture (3.3 g, yield 98%).
m/z: Calcd. 2997.50, Found 1499.75 [M+2H]²⁺

### Examples 22 to 41 and Comparative Example 4: Oxidation using various oxidants

A mixture of DMTrO-G(III)A(S)G(S)^{Me}C-SUC-TOB and Piv-TOB (100 mg: 52.8 mg as DMTrO-G(III)A(S)G(S)^{Me}C-SUC-TOB, 0.018 mmol), and dehydrated dichloromethane (0.9 mL) were added to a flask to prepare a solution. Pyridine (0.11 mmol) and water (8.8 µL) were added to the solution, various oxidants (53 µmοl) shown in the following Tables 7 to 9 were added, and the mixture was stirred at room temperature for 3 hr to perform oxidation to synthesize a tetramer, oligonucleotide DMTrO-G(O)A(S)G(S)^{Me}C-SUC-TOB. Thereafter, triphenylphosphine (9.2 mg, 35 µmol) was added, and the solution was stirred at room temperature for 30 min. Thereafter, acetonitrile (129 mL) was added, and the precipitated solid was recovered by suction filtration using a Kiriyama funnel, and dried under reduced pressure to give Piv-TOB and a tetramer, oligonucleotide DMTrO-G(O)A(S)G(S)^{Me}C-SUC-TOB, as a mixture.
m/z: Calcd. 3013.49, Found 3014.54 [M+H]⁺

As compound (Ib-2) to compound (Ib-5) and compound (Ib-8), those obtained in the aforementioned Synthetic Examples 3 to 7 were used.

As compound (Ic-11) to compound (Ic-13) and compound (Ic-15), those obtained in the aforementioned Synthetic Examples 9 to 12 were used.

As compound (Ic-1), compound (Ic-2), compound (Ic-5), compound (Ic-10), compound (Ic-14), compound (Ic-16), and compound (Ic-17), those synthesized by the method described in the Supporting Information of Non Patent Literature 2 were used.

Other oxidants used were commercially available products.

### Comparison of Examples 22 to 41 and Comparative Example 4 (proportion of desulfurized byproduct)

As described below, an oligonucleotide having a thiophosphate ester bond, and byproducts obtained by conversion of the thiophosphate ester bond to a phosphate ester bond (hereinafter referred to as "desulfurized byproduct") were analyzed, and the proportion of the desulfurized byproduct was calculated.

The solid (5 mg) obtained in Examples 22 to 41 or Comparative Example 4 and 28 wt% aqueous ammonia (5 mL) were placed in an autoclave, heated at 65°C for 4 hr, and cooled to room temperature. Insoluble materials in the reaction mixture were removed by a syringe filter, and the mixture was concentrated under reduced pressure by a centrifugation evaporator to give an aqueous solution of DMTrO-G(O)A(S)G(S)^{Me}C-OH having a thiophosphate ester bond (hereinafter referred to as "desired oligonucleotide").
m/z: Calcd. 1547.37, Found 1546.34 [M-H]⁻

The obtained mixture containing the desired oligonucleotide and desulfurized byproduct was analyzed by liquid chromatography-mass spectrometry (LC-MS). Using the extraction ion chromatogram (EIC) of the observed each compound (desired oligonucleotide and desulfurized byproduct), the peak area of each compound was calculated, and the proportion of the desulfurized byproduct was calculated by the following formula: $proportion \left(%\right) of desulfurized byproduct = \left(\begin{matrix}peak area of \\ desulfurized byproduct / peak area of desired oligonucleotide\end{matrix}\right) \times 100 .$ The results are shown in Tables 7 and 8.

**[Table 7]**

| | oxidant used | proportion of desulfurized byproduct |
|---|---|---|
| Comparative Example 4 | iodine | 0.5% |
| Example 22 | 2-phenylisothiazolo[5,4-b]pyridin-3(2H)-one (compound (Ic-1)) | <0.3% |
| Example 23 | 2-phenylisothiazolo[4,5-c]pyridin-3(2H)-one (compound (Ic-10)) | <0.3% |
| Example 24 | 5-nitro-2-phenyl-1,2-benzothiazol-3(2H)-one (compound (Ic-5)) | <0.3% |
| Example 25 | 2-phenylisothiazolo[5,4-b]quinolin-3(2H)-one (compound (Ic-11)) | 0.4% |
| Example 26 | 2-phenyl-5-trifluoromethylisothiazolo[5,4-b]pyridin-3(2H)-one (compound (Ic-12)) | <0.3% |
| Example 27 | 2-phenyl-2H-1,2-benzothiazin-3(4H)-one (compound (Ic-13)) | <0.3% |
| Example 28 | 2-isopropylisothiazolo[5,4-b]pyridin-3(2H)-one (compound (Ic-2)) | <0.3% |
| Example 29 | 2-(2,6-dimethylphenyl)isothiazolo[5,4-b]pyridin-3(2H)-one (compound (Ic-14)) | <0.3% |
| Example 30 | 2-(4-methyl-2-pyridinyl)isothiazolo[5,4-b]pyridin-3(2H)-one (compound (Ic-15)) | <0.3% |
| Example 31 | 2-(4-methoxyphenyl)isothiazolo[5,4-b]pyridin-3(2H)-one (compound (Ic-16)) | <0.3% |
| Example 32 | 2-(4-fluorophenyl)isothiazolo[5,4-b]pyridin-3(2H)-one (compound (Ic-17)) | <0.3% |

**[Table 8]**

| | oxidant used | proportion of desulfurized byproduct |
|---|---|---|
| Example 33 | 2,2'-dibenzothiazolyl disulfide (compound (Ib-1)) | <0.3% |
| Example 34 | 2-(2-benzothiazolyldithio)propanoic acid (compound (Ib-2)) | 0.4% |
| Example 35 | 3-(2-benzothiazolyldithio)propanoic acid (compound (Ib-3)) | <0.3% |
| Example 36 | 2,2'-dibenzoimidazolyl disulfide (compound (Ib-4)) | <0.3% |
| Example 37 | 2,2'-dibenzoxazolyl disulfide (compound (Ib-5)) | <0.3% |
| Example 38 | 5,5'-dithiobis(1-phenyl-1H-tetrazole) (compound (Ib-7)) | <0.3% |
| Example 39 | 2,2'-dithiobis(pyridine-N-oxide) (compound (Ib-8)) | <0.3% |
| Example 40 | 4,4'-dipyridyl disulfide (compound (Ib-9)) | <0.3% |
| Example 41 | 3-(2-pyridyldithio)propanoic acid (compound (Ib-10)) | <0.3% |

As is clear from the results of Tables 7 and 8, production of desulfurized byproducts can be suppressed by using compound (Ic-1) etc. shown in Tables 7 and 8 as an oxidant instead of iodine.

### Comparison of Examples 22 to 32 and Comparative Example 4 (proportion of defective byproduct)

The aforementioned operations up to the oxidation in Examples 22 to 32 and Comparative Example 4 were performed to prepare a solution containing a tetramer, oligonucleotide DMTrO-G(O)A(S)G(S)^{Me}C-SUC-TOB (hereinafter referred to as " oxidized product"). This solution (10 µL) and DDTT (1 mg) were placed in a 1 mL vial, diluted with tetrahydrofuran (450 µL), DBU (20 µL) was added, and the solution was stirred at room temperature for 30 sec to sulfurize unreacted DMTrO-G(III)A(S)G(S)^{Me}C-SUC-TOB to synthesize DMTrO-G(S)A(S)G(S)^{Me}C-SUC-TOB (hereinafter referred to as "sulfurized product"), whereby a test solution containing the oxidized product and the sulfurized product was prepared. The obtained test solution was subjected to MS analysis and the proportion of the defective byproduct was calculated using the abundance of the observed each compound (desired oligonucleotide as oxidized product) and defective byproduct (HO-A(S)G(S)^{Me}C-SUC-TOB)) and by the following formula: $proportion of defective byproduct \left(%\right) = \left(\begin{matrix}abundance of \\ defective byproduct / abundance of oxidized product\end{matrix}\right) \times 100 .$ The results are shown in Table 9.

**[Table 9]**

| | oxidant used | proportion of defective byproduct |
|---|---|---|
| Comparative Example 4 | iodine | 1.9% |
| Example 22 | 2-phenylisothiazolo[5,4-b]pyridin-3(2H)-one (compound (Ic-1)) | <0.6% |
| Example 23 | 2-phenylisothiazolo[4,5-c]pyridin-3(2H)-one (compound (Ic-10)) | 1.4% |
| Example 24 | 5-nitro-2-phenyl-1,2-benzothiazol-3(2H)-one (compound (Ic-5)) | 3.2% |
| Example 25 | 2-phenylisothiazolo[5,4-b]quinolin-3(2H)-one (compound (Ic-11)) | 1.2% |
| Example 26 | 2-phenyl-5-trifluoromethylisothiazolo[5,4-b]pyridin-3(2H)-one (compound (Ic-12)) | <0.6% |
| Example 27 | 2-phenyl-2H-1,2-benzothiazin-3(4H)-one (compound (Ic-13)) | 0.8% |
| Example 28 | 2-isopropylisothiazolo[5,4-b]pyridin-3 (2H)-one (compound (Ic-2)) | 1.7% |
| Example 29 | 2-(2,6-dimethylphenyl)isothiazolo[5,4-b]pyridin-3(2H)-one (compound (Ic-14)) | <0.6% |
| Example 30 | 2-(4-methyl-2-pyridinyl)isothiazolo[5,4-b]pyridin-3(2H)-one (compound (Ic-15)) | <0.6% |
| Example 31 | 2-(4-methoxyphenyl)isothiazolo[5,4-b]pyridin-3(2H)-one (compound (Ic-16)) | <0.6% |
| Example 32 | 2-(4-fluorophenyl)isothiazolo[5,4-b]pyridin-3(2H)-one (compound (Ic-17)) | <0.6% |

As is clear from the results of Table 9, production of defective byproduct can be suppressed by using compound (Ic-1), etc. (excluding compound (Ic-5)) shown in Table 9 as an oxidant instead of iodine.

### [Industrial Applicability]

According to the production method of the present invention, oligonucleotide can be produced while suppressing the production of the aforementioned defective byproduct or desulfurized byproduct, as compared with the use of iodine. The oligonucleotide obtained by the production method of the present invention can be used for various applications such as pharmaceutical products (RNA, DNA, oligonucleic acid medicine, etc.) for human or animal, functional food, food for specified health uses, food, chemical product, polymer material for living body or industrial use, and the like.

This application is based on a patent application No. 2021-21175 filed in Japan, the contents of which are incorporated in full herein.

## Claims

1. A method for producing an oligonucleotide having a phosphate ester bond by oxidizing an oligonucleotide precursor having a phosphite ester bond or a phosphonate ester bond with an oxidant, wherein
the oxidant is a compound represented by the formula (I): 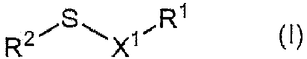 wherein
X¹ is a single bond, a sulfur atom, an oxygen atom, -S(=O)₂- or -N(-R³)-,
when X¹ is a single bond, then R¹ is a halogen atom, and R² is an optionally substituted aryl group or an optionally substituted heteroaryl group,
when X¹ is a sulfur atom or -S(=O)₂-, then R¹ is an optionally substituted alkyl group, an optionally substituted aryl group, or an optionally substituted heteroaryl group, and R² is an optionally substituted aryl group or an optionally substituted heteroaryl group,
when X¹ is an oxygen atom, then R¹ and R² form, together with a sulfur atom and an oxygen atom bonded thereto, an optionally substituted heterocycle, and
when X¹ is -N(-R³)-, then R¹ is an optionally substituted alkyl group, an optionally substituted aryl group, or an optionally substituted heteroaryl group, and R² and R³ form, together with a sulfur atom and a nitrogen atom bonded thereto, an optionally substituted heterocycle, or R¹ and R³ form, together with a nitrogen atom bonded thereto, an optionally substituted heterocycle, and R² is an optionally substituted aryl group or an optionally substituted heteroaryl group
provided that 2,2-dipyridyl disulfide is excluded.

2. The method according to claim 1, wherein the compound represented by the formula (I) comprises a compound represented by the formula (Ia): 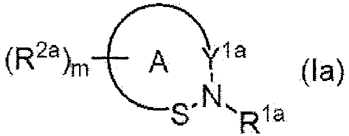 wherein
R^{1a} is an optionally substituted alkyl group, an optionally substituted aryl group, or an optionally substituted heteroaryl group,
ring A is a 5- or 6-membered unsaturated heterocycle,
Y^{1a} is -S(=O)-, -S(=O)₂-, -C(=O)-, or -C(=S)-,
m is an integer of 0 to 3,
R^{2a} in the number of m are each independently an optionally substituted alkyl group, an optionally substituted alkoxy group, or an electron-withdrawing group, and
when m is an integer of not less than 2, the adjacent two R^{2a} optionally form, together with ring A, an optionally substituted fused ring, and/or a compound represented by the formula (Ib): 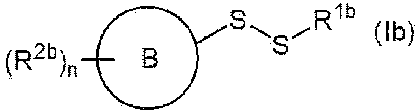 wherein
R^{1b} is an optionally substituted alkyl group, an optionally substituted aryl group, or an optionally substituted heteroaryl group,
ring B is a 6-membered aromatic hydrocarbon ring or a 5- or 6-membered aromatic heterocycle,
n is an integer of 0 to 5,
R^{2b} in the number of n are each independently an optionally substituted alkyl group, an optionally substituted alkoxy group, or an electron-withdrawing group, and
when n is an integer of not less than 2, the adjacent two R^{2b} optionally form, together with ring B, an optionally substituted bicyclic fused aromatic heterocycle
provided that 2,2-dipyridyl disulfide is excluded.

3. The method according to claim 2, wherein the compound represented by the formula (Ia) is a compound represented by the formula (Ic): 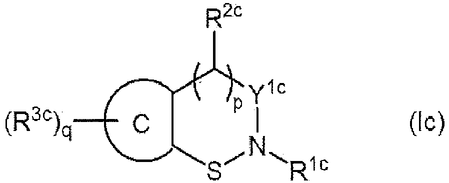 wherein
R^{1c} is an optionally substituted alkyl group, an optionally substituted aryl group, or an optionally substituted heteroaryl group,
Y^{1c} is -S(=O)-, -S(=O)₂-, -C(=O)-, or -C(=S)-,
p is 0 or 1,
R^{2c} is a hydrogen atom, an optionally substituted alkyl group, an optionally substituted alkoxy group, or an electron-withdrawing group,
ring C is a 6-membered aromatic hydrocarbon ring, a 6-membered nitrogen-containing aromatic heterocycle, or a 10-membered bicyclic fused aromatic heterocycle containing a nitrogen atom,
q is an integer of 0 to 4, and
R^{3c} in the number of q are each independently an optionally substituted alkyl group, an optionally substituted alkoxy group, or an electron-withdrawing group.

4. The method according to claim 3, wherein the compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, 2-[1-methyl-1-(2-pyridyl)ethyl]-5-nitro-1,2-benzothiazol-3(2H)-one, 2-phenylisothiazolo[4,5-c]pyridin-3(2H)-one, 2-phenylisothiazolo[5,4-b]quinolin-3(2H)-one, 2-phenyl-5-trifluoromethylisothiazolo[5,4-b]pyridin-3(2H)-one, 2-phenyl-2H-1,2-benzothiazin-3(4H)-one, 2-(2,6-dimethylphenyl)isothiazolo[5,4-b]pyridin-3(2H)-one, 2-(4-methyl-2-pyridinyl)isothiazolo[5,4-b]pyridin-3(2H)-one, 2-(4-methoxyphenyl)isothiazolo[5,4-b]pyridin-3(2H)-one, and 2-(4-fluorophenyl)isothiazolo[5,4-b]pyridin-3(2H)-one.

5. The method according to claim 3, wherein the ring C is a 6-membered aromatic hydrocarbon ring or a 6-membered nitrogen-containing aromatic heterocycle.

6. The method according to claim 5, wherein the compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, and 2-[1-methyl-1-(2-pyridyl)ethyl]-5-nitro-1,2-benzothiazol-3(2H)-one.

7. The method according to any one of claims 2 to 6, wherein, in the compound represented by the formula (Ib), a group represented by the formula (r1): 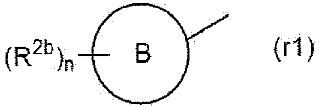 is
(1) a 2-benzothiazolyl group optionally substituted by 1 to 4 substituents selected from the group consisting of an optionally substituted alkyl group, an optionally substituted alkoxy group, and an electron-withdrawing group,
(2) a 2-benzoimidazolyl group optionally substituted by 1 to 5 substituents selected from the group consisting of an optionally substituted alkyl group, an optionally substituted alkoxy group, and an electron-withdrawing group,
(3) a 2-benzoxazolyl group optionally substituted by 1 to 4 substituents selected from the group consisting of an optionally substituted alkyl group, an optionally substituted alkoxy group, and an electron-withdrawing group,
(4) a 5-(1,2,3-triazolyl) group optionally substituted by 1 or 2 substituents selected from the group consisting of an optionally substituted alkyl group, an optionally substituted alkoxy group, and an electron-withdrawing group,
(5) a 1H-tetrazol-5-yl group optionally substituted by one substituent selected from the group consisting of an optionally substituted alkyl group and an optionally substituted aryl group,
(6) a (pyridine-N-oxide)-2-yl group optionally substituted by 1 to 4 substituents selected from the group consisting of an optionally substituted alkyl group, an optionally substituted alkoxy group, and an electron-withdrawing group,
(7) a 4-pyridyl group optionally substituted by 1 to 4 substituents selected from the group consisting of an optionally substituted alkyl group, an optionally substituted alkoxy group, and an electron-withdrawing group,
(8) a 2-pyridyl group optionally substituted by 1 to 4 substituents selected from the group consisting of an optionally substituted alkyl group, an optionally substituted alkoxy group, and an electron-withdrawing group,
(9) a phenyl group optionally substituted by 1 to 5 substituents selected from the group consisting of an optionally substituted alkyl group, an optionally substituted alkoxy group, and an electron-withdrawing group, or
(10) a 2-imidazolyl group optionally substituted by 1 to 3 substituents selected from the group consisting of an optionally substituted alkyl group, an optionally substituted alkoxy group, and an electron-withdrawing group.

8. The method according to claim 7, wherein the compound represented by the formula (Ib) is at least one selected from the group consisting of 2,2'-dibenzothiazolyl disulfide, 2-(2-benzothiazolyldithio)propanoic acid, 3-(2-benzothiazolyldithio)propanoic acid, 2,2'-dibenzoimidazolyl disulfide, 2,2'-dibenzoxazolyl disulfide, 5,5'-di(1,2,3-triazolyl) disulfide, 5,5'-dithiobis(1-phenyl-1H-tetrazole), 2,2'-dithiobis(pyridine-N-oxide), 4,4'-dipyridyl disulfide, 3-(2-pyridyldithio)propanoic acid, 2,2'-dithiobis(1H-imidazole), and 2,2'-dithiobis(1-methyl-1H-imidazole) .

9. The method according to any one of claims 1 to 8, wherein the oxidation is performed in the presence of water.

10. The method according to any one of claims 1 to 9, wherein the oligonucleotide precursor having a phosphite ester bond or a phosphonate ester bond is
(1) an oligonucleotide precursor having a phosphite ester bond which is obtained by condensation of a nucleoside, nucleotide, or oligonucleotide with a phosphoramidited nucleoside, nucleotide, or oligonucleotide, or
(2) an oligonucleotide precursor having a phosphite ester bond which is obtained by condensation of an oligonucleotide with a phosphoramidite.

11. The method according to any one of claims 1 to 10, wherein the oligonucleotide precursor having a phosphite ester bond or a phosphonate ester bond is an oligonucleotide precursor having a thiophosphate ester bond in addition to the phosphite ester bond or the phosphonate ester bond.

12. A method for producing an oligonucleotide by an one-pot synthesis, wherein the one-pot synthesis comprises
step (1) in which nucleoside, nucleotide, or oligonucleotide (a), each having a hydrophobic protecting group, and nucleoside, nucleotide, or oligonucleotide (b), each of which is phosphoramidited and in which a hydroxy group is protected by a temporary protecting group removable under acidic conditions are condensed in a solution containing a non-polar solvent to form oligonucleotide precursor (c) having a phosphite ester bond and the hydrophobic protecting group, in which the hydroxy group is protected by the temporary protecting group removable under acidic conditions,
step (2) in which quencher (i) for the phosphoramidited, nucleoside, nucleotide, or oligonucleotide (b) is added to the solution after step (1) to quench the phosphoramidited nucleoside, nucleotide, or oligonucleotide (b)
step (3) in which an oxidant is added to the solution after step (2) to oxidize the oligonucleotide precursor (c) to form oligonucleotide (d) having a phosphate ester bond and the hydrophobic protecting group, in which the hydroxy group is protected by the temporary protecting group removable under acidic conditions,
step (4) in which quencher (ii) for the oxidant is added to the solution after step (3) to quench the oxidant,
step (5) in which an acid is added to the solution after step (4) to remove the temporary protecting group removable under acidic conditions from the oligonucleotide (d) to form oligonucleotide (e) having an unprotected hydroxy group and the hydrophobic protecting group, and
step (7) in which a polar solvent is added to the solution containing the oligonucleotide (e) to precipitate the oligonucleotide (e), and
the oxidant is a compound represented by the formula (I): 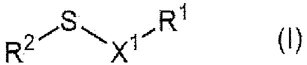 wherein
X¹ is a single bond, a sulfur atom, an oxygen atom, -S(=O)₂-, or -N(-R³)-,
when X¹ is a single bond, then R¹ is a halogen atom, and R² is an optionally substituted aryl group or an optionally substituted heteroaryl group,
when X¹ is a sulfur atom or -S(=O)₂-, then R¹ is an optionally substituted alkyl group, an optionally substituted aryl group, or an optionally substituted heteroaryl group, and R² is an optionally substituted aryl group or an optionally substituted heteroaryl group,
when X¹ is an oxygen atom, then R¹ and R² form, together with a sulfur atom and an oxygen atom bonded thereto, an optionally substituted heterocycle, and
when X¹ is -N(-R³)-, then R¹ is an optionally substituted alkyl group, an optionally substituted aryl group, or an optionally substituted heteroaryl group, and R² and R³ form, together with a sulfur atom and a nitrogen atom bonded thereto, an optionally substituted heterocycle, or R¹ and R³ form, together with a nitrogen atom bonded thereto, an optionally substituted heterocycle, and R² is an optionally substituted aryl group or an optionally substituted heteroaryl group
provided that 2,2-dipyridyl disulfide is excluded.

13. The method according to claim 12, wherein the compound represented by the formula (I) comprises a compound represented by the formula (Ia): 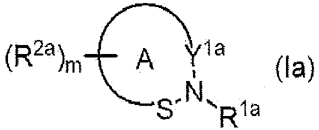 wherein
R^{1a} is an optionally substituted alkyl group, an optionally substituted aryl group, or an optionally substituted heteroaryl group,
ring A is a 5- or 6-membered unsaturated heterocycle,
Y^{1a} is -S(=O)-, -S(=O)₂-, -C(=O)-, or -C(=S)-,
m is an integer of 0 to 3,
R^{2a} in the number of m are each independently an optionally substituted alkyl group, an optionally substituted alkoxy group, or an electron-withdrawing group, and
when in is an integer of not less than 2, the adjacent two R^{2a} optionally form, together with ring A, an optionally substituted fused ring, and/or
a compound represented by the formula (Ib): 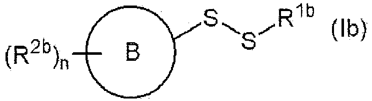 wherein
R^{1b} is an optionally substituted alkyl group, an optionally substituted aryl group, or an optionally substituted heteroaryl group,
ring B is a 6-membered aromatic hydrocarbon ring or a 5- or 6-membered aromatic heterocycle,
n is an integer of 0 to 5,
R^{2b} in the number of n are each independently an optionally substituted alkyl group, an optionally substituted alkoxy group, or an electron-withdrawing group, and
when n is an integer of not less than 2, the adjacent two R^{2b} optionally form, together with ring B, an optionally substituted bicyclic fused aromatic heterocycle
provided that 2,2-dipyridyl disulfide is excluded.

14. The method according to claim 13, wherein the compound represented by the formula (Ia) is a compound represented by the formula (Ic): 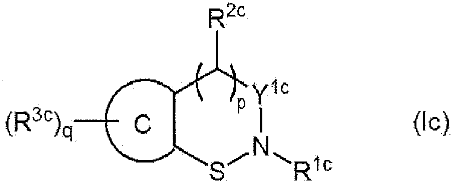 wherein
R^{1c} is an optionally substituted alkyl group, an optionally substituted aryl group, or an optionally substituted heteroaryl group,
Y^{1c} is -S(=O)-, -S(=O)₂-, -C(=O)- or -C(=S)-,
p is 0 or 1,
R^{2c} is a hydrogen atom, an optionally substituted alkyl group, an optionally substituted alkoxy group, or an electron-withdrawing group,
ring C is a 6-membered aromatic hydrocarbon ring, a 6-membered nitrogen-containing aromatic heterocycle, or a 10-membered bicyclic fused aromatic heterocycle containing a nitrogen atom,
q is an integer of 0 to 4, and
R^{3c} in the number of q are each independently an optionally substituted alkyl group, an optionally substituted alkoxy group, or an electron-withdrawing group.

15. The method according to claim 14, wherein the compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, 2-[1-methyl-1-(2-pyridyl)ethyl]-5-nitro-1,2-benzothiazol-3(2H)-one, 2-phenylisothiazolo[4,5-c]pyridin-3(2H)-one, 2-phenylisothiazolo[5,4-b]quinolin-3(2H)-one, 2-phenyl-5-trifluoromethylisothiazolo[5,4-b]pyridin-3(2H)-one, 2-phenyl-2H-1,2-benzothiazin-3(4H)-one, 2-(2,6-dimethylphenyl)isothiazolo[5,4-b]pyridin-3(2H)-one, 2-(4-methyl-2-pyridinyl)isothiazolo[5,4-b]pyridin-3(2H)-one, 2-(4-methoxyphenyl)isothiazolo[5,4-b]pyridin-3(2H)-one, and 2-(4-fluorophenyl)isothiazolo[5,4-b]pyridin-3(2H)-one.

16. The method according to claim 14, wherein the ring C is a 6-membered aromatic hydrocarbon ring or a 6-membered nitrogen-containing aromatic heterocycle.

17. The method according to claim 16, wherein the compound represented by the formula (Ic) is at least one selected from the group consisting of 2-phenylisothiazolo[5,4-b]pyridin-3(2H)-one, 2-isopropylisothiazolo[5,4-b]pyridin-3(2H)-one, 2-isopropylisothiazolo[4,5-c]pyridin-3(2H)-one, 2-(2-pyridyl)-1,2-benzothiazol-3(2H)-one, 5-nitro-2-phenyl-1,2-benzothiazol-3(2H)-one, 2-(2,6-dimethylphenyl)-5-nitro-1,2-benzothiazol-3(2H)-one, 5-nitro-2-(2-pyridyl)-1,2-benzothiazol-3(2H)-one, 5-nitro-2-(4-pyridyl)-1,2-benzothiazol-3(2H)-one, and 2-[1-methyl-1-(2-pyridyl)ethyl]-5-nitro-1,2-benzothiazol-3(2H)-one.

18. The method according to any one of claims 13 to 17, wherein, in the compound represented by the formula (Ib), a group represented by the formula (r1): 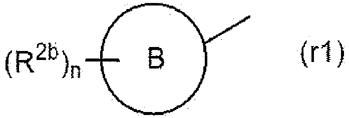 is
(1) a 2-benzothiazolyl group optionally substituted by 1 to 4 substituents selected from the group consisting of an optionally substituted alkyl group, an optionally substituted alkoxy group, and an electron-withdrawing group,
(2) a 2-benzoimidazolyl group optionally substituted by 1 to 5 substituents selected from the group consisting of an optionally substituted alkyl group, an optionally substituted alkoxy group, and an electron-withdrawing group,
(3) a 2-benzoxazolyl group optionally substituted by 1 to 4 substituents selected from the group consisting of an optionally substituted alkyl group, an optionally substituted alkoxy group, and an electron-withdrawing group,
(4) a 5-(1,2,3-triazolyl) group optionally substituted by 1 or 2 substituents selected from the group consisting of an optionally substituted alkyl group, an optionally substituted alkoxy group, and an electron-withdrawing group,
(5) a 1H-tetrazol-5-yl group optionally substituted by one substituent selected from the group consisting of an optionally substituted alkyl group and an optionally substituted aryl group,
(6) a (pyridine-N-oxide)-2-yl group optionally substituted by 1 to 4 substituents selected from the group consisting of an optionally substituted alkyl group, an optionally substituted alkoxy group, and an electron-withdrawing group,
(7) a 4-pyridyl group optionally substituted by 1 to 4 substituents selected from the group consisting of an optionally substituted alkyl group, an optionally substituted alkoxy group, and an electron-withdrawing group,
(8) a 2-pyridyl group optionally substituted by 1 to 4 substituents selected from the group consisting of an optionally substituted alkyl group, an optionally substituted alkoxy group, and an electron-withdrawing group,
(9) a phenyl group optionally substituted by 1 to 5 substituents selected from the group consisting of an optionally substituted alkyl group, an optionally substituted alkoxy group, and an electron-withdrawing group, or
(10) a 2-imidazolyl group optionally substituted by 1 to 3 substituents selected from the group consisting of an optionally substituted alkyl group, an optionally substituted alkoxy group, and an electron-withdrawing group.

19. The method according to claim 18, wherein the compound represented by the formula (Ib) is at least one selected from the group consisting of 2,2'-dibenzothiazolyl disulfide, 2-(2-benzothiazolyldithio)propanoic acid, 3-(2-benzothiazolyldithio)propanoic acid, 2,2'-dibenzoimidazolyl disulfide, 2,2'-dibenzoxazolyl disulfide, 5,5'-di(1,2,3-triazolyl) disulfide, 5,5'-dithiobis(1-phenyl-1H-tetrazole), 2,2'-dithiobis(pyridine-N-oxide), 4,4'-dipyridyl disulfide, 3-(2-pyridyldithio)propanoic acid, 2,2'-dithiobis(1H-imidazole), and 2,2'-dithiobis(1-methyl-1H-imidazole) .

20. The method according to any one of claims 12 to 19, wherein the quencher (i) is water.

21. The method according to any one of claims 12 to 20, wherein the quencher (ii) is an organic phosphorus compound.

22. The method according to claim 21, wherein the organic phosphorus compound is at least one selected from the group consisting of a phosphine, a phosphorous acid triester, a phosphinite, a phosphonous acid diester, and a phosphinate ester.

23. The method according to claim 21, wherein the organic phosphorus compound is a phosphine.

24. The method according to any one of claims 12 to 23, wherein the one-pot synthesis further comprises adding an aromatic amine to the solution after step (3).

25. The method according to any one of claims 12 to 24, wherein the one-pot synthesis further comprises step (6) of adding, after step (5) and before step (7), a base to the solution after step (5) .

26. The method according to any one of claims 12 to 25, wherein the oligonucleotide precursor (c) is an oligonucleotide precursor having a thiophosphate ester bond in addition to the phosphite ester bond.

27. A compound represented by the formula (In): 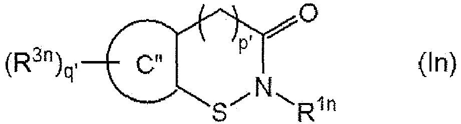 wherein
(1) R¹ⁿ is an optionally substituted phenyl group, p' is 0, ring C" is a 10-membered bicyclic fused aromatic heterocycle containing a nitrogen atom, and q' is 0, or
(2) R¹ⁿ is an optionally substituted phenyl group, p' is 0, ring C" is a 6-membered nitrogen-containing aromatic heterocycle, q' is 1, and R³ⁿ is a C₁₋₆ perfluoroalkyl group, or
(3) R¹ⁿ is an optionally substituted phenyl group, p' is 1, ring C" is a 6-membered nitrogen-containing aromatic heterocycle, and q' is 0, or
(4) R¹ⁿ is a pyridyl group substituted by one C₁₋₆ alkyl group, p' is 0, ring C" is a 6-membered nitrogen-containing aromatic heterocycle, and q' is 0.

28. The compound according to claim 27 wherein the compound represented by the formula (In) is 2-phenylisothiazolo[5,4-b]quinolin-3(2H)-one, 2-phenyl-5-trifluoromethylisothiazolo[5,4-b]pyridin-3(2H)-one, 2-phenyl-2H-1,2-benzothiazin-3(4H)-one, or 2-(4-methyl-2-pyridinyl)isothiazolo[5,4-b]pyridin-3(2H)-one.
